(19) Europäisches Patentamt
European Patent Office
Office européen des brevets

(11) Veröffentlichungsnummer : **0 579 071 A2**

# (12) EUROPÄISCHE PATENTANMELDUNG

(21) Anmeldenummer : **93110679.3**

(22) Anmeldetag : **05.07.93**

(51) Int. Cl.[5] : **C07D 231/00,** C07D 261/08, C07D 231/12, C07D 263/32, C07D 333/24, C07D 207/337, C07D 233/64, C07D 239/26, C07D 413/12, C07C 251/38, A01N 43/78, // A01N43/80, A01N43/56, A01N43/76, A01N43/10

(30) Priorität : **16.07.92 DE 4223357**

(43) Veröffentlichungstag der Anmeldung : **19.01.94 Patentblatt 94/03**

(84) Benannte Vertragsstaaten : **AT BE CH DE DK ES FR GB GR IE IT LI NL PT SE**

(71) Anmelder : **BASF Aktiengesellschaft**
**Carl-Bosch-Strasse 38**
**D-67063 Ludwigshafen (DE)**

(72) Erfinder : **Mueller, Bernd, Dr.**
**Jean-Gans-Strasse 21**
**D-6710 Frankenthal (DE)**
Erfinder : **Sauter, Hubert, Dr.**
**Neckarpromenade 20**
**D-6800 Mannheim 1 (DE)**
Erfinder : **Wingert, Horst, Dr.**
**D 3.1**
**D-6800 Mannheim 1 (DE)**
Erfinder : **Koenig, Hartmann, Dr.**
**Albert-Einstein-Allee 16**
**W-6703 Limburgerhof (DE)**
Erfinder : **Roehl, Franz, Dr.**
**Sebastian-Kneip-Strasse 17**
**W-6707 Schifferstadt (DE)**
Erfinder : **Ammermann, Eberhard, Dr.**
**Von Gagern-Strasse 2**
**W-6148 Heppenheim (DE)**
Erfinder : **Lorenz, Gisela, Dr.**
**Erlenweg 13**
**D-6730 Neustadt (DE)**

(54) **Heteroaromatische Verbindungen und diese enthaltende Pflanzenschutzmittel.**

(57) Heteroaromatishe Verbindungen der Formel IA und IB

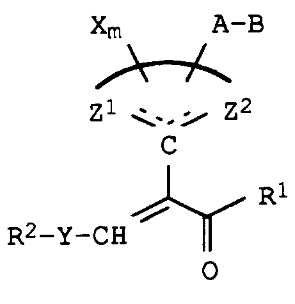

IA

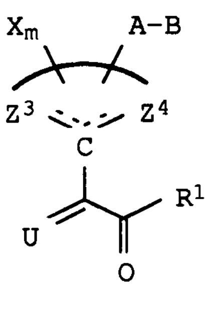

IB

in denen die gestrichelte Linie für eine Doppelbindung zwischen dem C-Atom und einem Z-Atom steht, und der Index und die Substituenten die folgende Bedeutung haben :

$R^1$
Alkyl, Alkoxy, Alkylamino
$R^2$
Alkyl ;
A
eine direkte Bindung ; Alkylen, Alkenylen, Alkinylen :

EP 0 579 071 A2

O, S, S-Oxide, N und ihre Alkylenderivate oder Oximreste
B
Wasserstoff, Halogen,
Alkyl, Alkenyl, Alkinyl ;
Cycloalkyl, Cycloalkenyl, Cycloalkinyl, Heterocyclyl, Aryl, Heteroaryl
U
$CH_2$, CHCl, $CHR^2$ oder $NOR^2$ ;
X
Wasserstoff, Cyano, Nitro, Halogen,
Alkyl, Alkenyl, Alkinyl, Cycloalkyl, Cycloalkenyl, Heterocyclyl, Aryl, Heteroaryl ; ihre Oxi- und Thioderivate, ihre Carbonylderivate, Carbonyloxyderivate, ihre Aminoderivate, ihre Oximderivate,
Amino, welches ein oder zwei der oben genannten Gruppen tragen kann ;
m
1 oder 2,
Y
Sauerstoff oder Schwefel ;
$Z^1$-$Z^2$, $Z_3$-$Z_4$ bedeuten
zusammen mit dem C-Atom an das sie gebunden sind, den Rest eines Heteroaromaten und Fungizide, die diese Verbindungen enthalten.

Die vorliegende Erfindung betrifft heteroaromatische Verbindungen und ihre Verwendung als Pflanzenschutzmittel, insbesondere zur Bekämpfung von Pilzen, Insekten, Nematoden und Spinnmilben.

Es ist bekannt, Phenylacrylsäureester, z.B. den 2-(2-Methylphenyl)-3-methoxy-acrylsäuremethylester (EP 178826), als Fungizid zu verwenden. Ihre fungizide Wirkung ist jedoch unbefriedigend.

Es wurde überraschend gefunden, daß heteroaromatische Verbindungen der Formel I

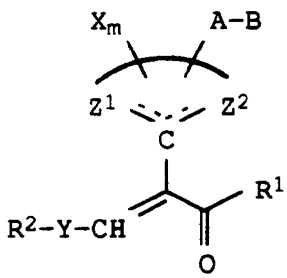

IA

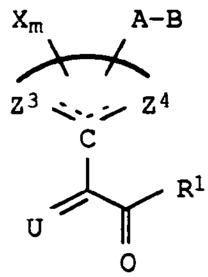

IB

in denen die gestrichelte Linie für eine Doppelbindung zwischen dem C-Atom und $Z^1$ oder $Z^3$ oder zwischen dem C-Atom und $Z^2$ oder $Z^4$ steht, und der Index und die Substituenten die folgende Bedeutung haben:

$R^1$

$C_1$-$C_6$-Alkyl, $C_1$-$C_6$-Alkoxy, $C_1$-$C_6$-Alkylamino oder Di-($C_1$-$C_6$-alkyl)-amino;

$R^2$

$C_1$-$C_6$-Alkyl;

A

eine direkte Bindung; ggf. subst. Alkylen, Alkenylen, Alkinylen;
$-(CHR^4)_n-O-$, $-(CHR^4)_n-S-$, $-(CHR^4)_n-NH-$, $-(CHR^4)_n-NR^5-$, $-(CHR^4)_n-S(=O)-$, $-(CHR^4)_n-S(=O)_2-$, $-(CHR^4)_n-O-S(=O)-$, $-(CHR^4)_n-S(=O)-O-$, $-(CHR^4)_n-O-S(=O)_2-$, $-(CHR^4)_n-S-(=O)_2-O-$, $-(CHR^4(_n-C(=O)-$, $-(CHR^4)_n-C(=O)O-$, $-(CHR^4)_n-O-C(=O)-$, $-(CHR^4)_n-CR^5=N-O-$, $-(CHR^4)_n-CR^5=N-N=CR^6$, $-(CHR^4)_n-O-N=CR^7-$,

n — 0, 1, 2, 3 oder 4, wobei die Reste $R^4$ verschieden sein können, wenn der Wert von n größer als 1 ist;

$R^4$, $R^5$ und $R^6$ — unabhängig voneinander Wasserstoff; ggf. subst. $C_1$-$C_6$-Alkyl, $C_2$-$C_6$-Alkenyl, $C_2$-$C_6$-Alkinyl oder Aryl;

$R^7$ — Wasserstoff, Cyano;
ggf. subst. Alkyl, Alkenyl, Alkinyl, Cycloalkyl, Cycloalkenyl, Cycloalkinyl, Heterocyclyl, Aryl, Heteroaryl;
ggf. subst. Alkoxy, Alkenyloxy, Alkinyloxy, Cycloalkoxy, Cycloalkenyloxy, Cycloalkinyloxy, Heterocyclyloxy, Aryloxy, Heteroaryloxy;
oder $R^7$ und B gemeinsam mit dem C-Atom, an das sie gebunden sind, ein ggf. subst., gesättigtes oder teilweise ungesättigtes, alicyclisches oder heterocyclisches System;

B

Wasserstoff, Halogen,
ggf. subst. Alkyl, Alkenyl, Alkinyl;
Cycloalkyl, Cycloalkenyl, Cycloalkinyl, Heterocyclyl, Aryl, Heteroaryl, $-(CHR^4)_p$-Cycloalkyl, $-(CHR^4)_p$-Cycloalkenyl, $-(CHR^4)_p$-Cycloalkinyl, - $(CHR^4)_p$-Heterocyclyl, $-(CHR^4)_p$-Aryl, - $(CHR^4)_p$-Heteroaryl $-(CHR^4)_p$-O-Cycloalkyl, $-(CHR^4)_p$-O-Cycloalkenyl, $-(CHR^4)_p$-O-Cycloalkinyl, $-(CHR^4)_p$-O-Heterocyclyl, $-(CHR^4)_p$-O-Aryl, $-(CHR^4)_p$-O-Heteroaryl, $-(CHR^4)_p$-S-Cycloalkyl, $-(CHR^4)_p$-S-Cycloalkenyl, $-(CHR^4)_p$-S-Cycloalkinyl, $-(CHR^4)_p$-S-Heterocyclyl, $-(CHR^4)_p$-S-Aryl, $-(CHR^4)_p$-S-Heteroaryl, $-(CHR^4)_p$-NH-Cycloalkyl, $-(CHR^4)_p$-NH-Cycloalkenyl, $-(CHR^4)_p$-NH-Cycloalkinyl, $-(CHR^4)_p$-NH-Heterocyclyl, $-(CHR^4)_p$-NH-Aryl, $-(CHR^4)_p$-NH-Heteroaryl, $-(CHR^4)_p$-$NR^5$-Cycloalkyl, $-(CHR^4)_p$-$NR^5$-Cycloalkenyl, $-(CHR^4)_p$-$NR^5$-Cycloalkinyl, $-(CHR^4)_p$-$NR^5$-Heterocyclyl, $-(CHR^4)_p$-$NR^5$-Aryl $-(CHR^4)_p$-$NR^5$-Heteroaryl, wobei die vorstehend genannten cyclischen Reste ihrerseits Substituenten tragen können;

p — 1, 2, 3 oder 4, wobei die Reste $R^4$ verschieden sein können, wenn der Wert von n größer als 1 ist;

U

eine Gruppe $CH_2$, CHCl, $CHR^2$ oder $NOR^2$;

X

Wasserstoff, Cyano, Nitro, Halogen, Haloalkyl, Haloalkoxy,
ggf. subst. Alkyl, Alkenyl, Alkinyl, Cycloalkyl, Cycloalkenyl, Heterocyclyl, Aryl, Heteroaryl;
ggf. subst. Alkoxy, Alkenyloxy, Alkinyloxy, Cycloalkoxy, Cycloalkenyloxy, Heterocyclyloxy, Aryloxy, Heteroaryloxy;
ggf. subst. Alkylthio, Alkenylthio, Alkinylthio, Cycloalkylthio, Cycloalkenylthio, Heterocyclylthio, Arylthio, Heteroarylthio;
Amino, welches ein oder zwei der folgenden Gruppen tragen kann: ggf. subst. Alkyl, Alkenyl, Alkinyl, Cycloalkyl, Cycloalkenyl, Heterocyclyl, Aryl und/oder Heteroaryl;
ggf. subst. Alkylcarbonyl, Alkenylcarbonyl, Alkinylcarbonyl, Cycloalkylcarbonyl, Cycloalkenylcarbonyl, Heterocyclylcarbonyl, Arylcarbonyl, Heteroarylcarbonyl;
ggf. subst. Alkylcarbonyloxy, Alkenylcarbonyloxy, Alkinylcarbonyloxy, Cycloalkylcarbonyloxy, Cycloalkenylcarbonyloxy, Heterocyclylcarbonyloxy, Arylcarbonyloxy, Heteroarylcarbonyloxy;
ggf. subst. Alkoxycarbonyl, Alkenyloxycarbonyl, Alkinyloxycarbonyl, Cycloalkoxycarbonyl, Cycloalkenyloxycarbonyl, Heterocyclyloxycarbonyl, Aryloxycarbonyl, Heteroaryloxycarbonyl;
ggf. subst. Alkylcarbonylamino, Alkenylcarbonylamino, Alkinylcarbonylamino, Cycloalkylcarbonylamino, Cycloalkenylcarbonylamino, Heterocyclylcarbonylamino, Arylcarbonylamino, Heteroarylcarbonylamino, wobei diese Reste an der Aminogruppe zusätzlich eine der folgenden Gruppen tragen können: ggf. subst. Alkyl, Alkenyl, Alkinyl, Cycloalkyl, Cycloalkenyl, Heterocyclyl, Aryl und/oder Heteroaryl;
Aminocarbonyl, welches an der Aminogruppe ein oder zwei der folgenden Gruppen tragen kann: ggf. subst. Alkyl, Alkenyl, Alkinyl, Cycloalkyl, Cycloalkenyl, Heterocyclyl, Aryl, und/ oder Heteroaryl;
ggf. subst. Alkyl-S(=O)-, Alkenyl-S(=O)-, Alkinyl-S(=O)-, Cycloalkyl-S(=O)-, Cycloalkenyl-S(=O)-, Heterocyclyl-S(=O)-, Aryl-S(=O)-, Heteroaryl-S(=O)-;
ggf. subst. Alkyl-S(=O)-O-, Alkenyl-S(=O)-O-, Alkinyl-S(=O)-O-, Cycloalkyl-S(=O)-O-, Cycloalkenyl-S(=O)-O-, Heterocyclyl-S(=O)-O-, Aryl-S(=O)-O-, Heteroaryl-S(=O)-O-,;
ggf. subst. Alkyl-O-S(=O)-, Alkenyl-O-S(=O)-, Alkinyl-O-S(=O)-, Cycloalkyl-O-S(=O)-, Cycloalkenyl-O-S(=O)-, Heterocyclyl-O-S(=O)-, Aryl-O-S(=O)-, Heteroaryl-O-S(=O)-;
ggf. subst. Alkyl-$S(=O)_2$-, Alkenyl-$S(=O)_2$-, Alkinyl-$S(=O)_2$-, Cycloalkyl-$S(=O)_2$-, Cycloalkenyl-$S(=O)_2$-, Heterocyclyl-$S(=O)_2$-, Aryl-$S(=O)_2$-, Heteroaryl-$S(=O)_2$-;
ggf. subst. Alkyl-$S(=O)_2$-O-, Alkenyl-$S(=O)_2$-O-, Alkinyl-$S(=O)_2$-O-, Cycloalkyl-$S(=O)_2$-O-, Cycloalkenyl-$S(=O)_2$-O-, Heteroxyclyl-$S(=O)_2$-O-, Aryl-$S(=O)_2$-O-, Heteroaryl-$S(=O)_2$-O-;
ggf. subst. Alkyl-O-$S(=O)_2$-, Alkenyl-O-$S(=O)_2$-, Alkinyl-O-$S(=O)_2$-, Cycloalkyl-O-$S(=O)_2$-, Cycloalkenyl-O-$S(=O)_2$-, Heterocyclyl-O-$S(=O)_2$-, Aryl-O-$S(=O)_2$-, Heteroaryl-O-$S(=O)_2$-;
ggf. subst. Alkyl-ON=$CR^8$-, Alkenyl-ON=$CR^8$-, Alkinyl-ON=$CR^8$-, Cycloalkyl-ON=$CR^8$-, Cycloalkenyl-ON=$CR^8$-, Heterocyclyl-ON=$CR^8$-, Aryl-ON=$CR^8$-, Heteroaryl-ON=$CR^8$-;

$R^8$ Wasserstoff; Cyano;
ggf. subst. Alkyl, Alkenyl, Alkinyl, Cycloalkyl, Cycloalkenyl, Cycloalkinyl, Heterocyclyl, Aryl, Heteroaryl;
ggf. subst. Alkoxy, Alkenyloxy, Alkinyloxy, Cycloalkoxy, Cycloalkenyloxy, Cycloalkinyloxy, Heterocyclyloxy, Aryloxy, Heteroaryloxy;

m
1 oder 2, wobei die Reste X verschieden sein können, wenn m den Wert 2 hat;

Y
Sauerstoff oder Schwefel;

$Z^1$-$Z^2$
zusammen mit dem C-Atom an das sie gebunden sind, entweder ein 6-gliedriger Heteroaromat enthaltend neben Kohlenstoffringgliedern zwei oder drei Stickstoffatome oder ein 5-gliedriger Heteroaromat enthaltend neben Kohlenstoffringgliedern zwei oder drei Stickstoffatome oder ein oder zwei Stickstoffatome und ein Sauerstoff- oder ein Schwefelatom;

$Z^3$-$Z^4$
zusammen mit dem C-Atom an das sie gebunden sind entweder ein 6-gliedriger Heteroaromat enthaltend neben Kohlenstoffringgliedern ein, zwei oder drei Stickstoffatome oder ein 5-gliedriger Heteroaromat enthaltend neben Kohlenstoffringgliedern ein, zwei oder drei Stickstoffatome oder ein Sauerstoff- oder ein Schwefelatom oder ein oder zwei Stickstoffatome und ein Sauerstoff- oder ein Schwefelatom ;

und ihre pflanzenverträglichen Säureadditionsprodukte und Basenadditionsprodukte,
ausgenommen solche Verbindungen, in denen
der Ring C-$Z^1$-$Z^2$ ein Pyrazolring ist, der Rest -Y-$R^2$ die Bedeutung -O-$CH_3$ hat und -A-B in 4-Stellung am Pyrazolring steht, -A- der Rest -O-$CH_2$- ist, B gegebenenfalls substituiertes Phenyl oder gegebenenfalls substituiertes Thiazolyl bedeutet oder
der Ring C-$Z^1$-$Z^2$ ein Pyrazolring ist, der Rest -Y-$R^2$ die Bedeutung -O-$CH_3$ hat und -A-B in 4-Stellung am Py-

razolring steht, -A- der Rest O-CO- ist und B die in Anspruch 1 genannte Bedeutung besitzt oder der Ring C-$Z^1$-$Z^2$ ein Thiazolring ist, Y Sauerstoff bedeutet und $R^1$ die Bedeutung $OCH_3$ hat, oder der Ring C-$Z^3$-$Z^4$ ein Thiazolring ist, X an ein N-Atom des Thiazolrings gebunden ist und U den Rest $NOR^2$ bedeutet, neben einer hohen fungitoxischen, insektiziden, nematiziden und akariziden Wirkung auch eine sehr gute Pflanzenverträglichkeit besitzen.

Säuren für Säureadditionsprodukte sind z.B. Mineralsäuren, wie beispielsweise Chlorwasserstoffsäure, Bromwasserstoffsäure, Phosphorsäure, Schwefelsäure, Salpetersäure, oder aber Carbonsäuren, wie Ameisensäure, Essigsäure, Oxalsäure, Malonsäure, Milchsäure, Äpfelsäure, Bernsteinsäure, Weinsäure, Zitronensäure, Salicylsäure, p-Toluolsulfonsäure, Dodecylbenzolsulfonsäure, oder aber auch allgemein Protonenacide Verbindungen, z.B. Saccharin.

Basen für Basenadditionsprodukte sind z.B. Kalium-, Natrium-, -hydroxid, -carbonat, Ammoniumhydroxid.

Die neuen Verbindungen der Formel I können bei der Herstellung als Gemische von Stereoisomeren (E/Z-Isomere, Diastereomere, Enantiomere) anfallen, die in üblicher Weise, z.B. durch Kristallisation oder Chromatographie, in die Einzelkomponenten getrennt werden können. Sowohl die einzelnen Isomeren als auch ihre Gemische können als Fungizide, Akarizide, Nematizide oder Insektizide verwendet werden und werden von der Erfindung erfaßt.

Die oben genannten Alkyle können beliebig substituiert sein, beispielsweise mit 1 - 4 gleichen oder verschiedenen Substituenten $R^{11}$, die Alkyle besitzen bevorzugt 1 - 8 Kohlenstoffatome und bedeuten beispielsweise Methyl, Ethyl, Propyl, n-Propyl, i-Propyl, Butyl, n-Butyl, i-Butyl, t-Butyl, s-Butyl, Pentyl, Pentyl-1, Pentyl-2, Pentyl-3, 2-Methylbutyl-1, 2-Methylbutyl-2, 2-Methylbutyl-3, 3-Methylbutyl-1, 2,2-Dimethylpropyl-1, Hexyl, Hexyl-1, Hexyl-2, Hexyl-3, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Heptyl, Heptyl-1, Heptyl-2, Heptyl-3, Heptyl-4, 1-Methylhexyl, 2-Methylhexyl, 3-Methylhexyl, 4-Methylhexyl, 5-Methylhexyl, 1-Ethylpentyl, 2-Ethylpentyl, 3-Ethylpentyl, 1-Propylbutyl, 1-Isopropylbutyl, Octyl, Octyl-1, Octyl-2, Octyl-3, Octyl-4, 1-Methylheptyl, 2-Methylheptyl, 3-Methylheptyl, 4-Methylheptyl 5-Methylheptyl, 6-Methylheptyl, 1-Ethylhexyl, 2-Ethylhexyl, 3-Ethylhexyl, 4-Ethylhexyl, 1-Propylpentyl oder 2-Propylpentyl

Die genannten Alkenyle können beliebig substituiert sein, beispielsweise mit 1 - 4 gleichen oder verschiedenen Substituenten $R^{11}$, die Alkenyle besitzen bevorzugt 2 - 8 Kohlenstoffatome und bedeuten beispielsweise Ethenyl, 1-Propenyl, 2-Propenyl, Butenyl, 1-Butenyl, 2-Butenyl, 3-Butenyl, 1-Methyl-2-propenyl, 2-Methyl-2-propenyl, Pentenyl, 1-Pentenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, Hexenyl, 1-Hexenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,3-Dimethyl-2-butenyl, 2,2-Dimethyl-3-butenyl, 2,3-dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenol oder 1-Ethyl-1-methyl-2-propenyl.

Die genannten Alkinyle können beliebig substituiert sein, beispielsweise mit 1 - 4 gleichen oder verschiedenen Substituenten R11, sie besitzen bevorzugt 2 - 8 Kohlenstoffatome und bedeuten beispielsweise Ethinyl, 1-Propinyl, 2-Propinyl, Butinyl, 1-Butinyl, 2-Butinyl, 3-Butinyl, 1-Methyl-2-propinyl, Pentinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-methyl-2-pentinyl, Hexinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl oder 1-Ethyl-2-methyl-2-propinyl.

Die genannten Halogene bedeuten Fluor, Chlor, Brom oder Jod.

Die genannten Cycloalkyle können beliebig substituiert sein, beispielsweise mit 1 - 4 gleichen oder verschiedenen Substituenten $R^{11}$, sie besitzen bevorzugt 3 - 10 Kohlenstoffatome und bedeuten beispielsweise Cyclopropyl, Cyclobutyl, Cyclopentyl, Cyclohexyl, Cycloheptyl, Cyclooctyl, Cyclononyl, Cyclodecyl, Bornanyl, Norbornanyl, Dicyclohexyl, Bicyclo[3,3,0]octyl, Bicyclo[3,2,1]octyl, Bicyclo[2,2,2]octyl oder Bicyclo[3,3,1]nonenyl.

Die genannten Cycloalkinyle können beliebig substituiert sein, beispielsweise mit 1 - 4 gleichen oder verschiedenen Substituenten $R^{11}$, sie besitzen bevorzugt 6 - 12 Kohlenstoffatome und bedeuten beispielsweise Cyclohexin, Cycloheptin, Cyclooctin, Cyclononin, Cyclodecin, Cycloundecin oder Cyclododecin.

Die genannten Haloalkyle können beliebig substituiert sein, beispielsweise mit 1 - 4 gleichen oder verschiedenen Substituenten $R^{11}$, sie besitzen bevorzugt 1 - 8 Kohlenstoffatome und bedeuten beispielsweise Brommethyl, Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-Difluorethyl, 2,2-Dichlor-2-fluorethyl, 2,2,2-Trichlorethyl oder Pentafluorethyl.

Die genannten Haloalkoxyreste können beliebig substituiert sein, beispielsweise mit 1 - 4 gleichen oder verschiedenen Substituenten $R^{11}$, sie besitzen bevorzugt 1 - 8 Kohlenstoffatome und bedeuten beispielsweise Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy oder Pentafluorethyloxy.

Die genannten Alkoxy- oder Alkylthioreste können beliebig substituiert sein, beispielsweise mit 1 bis 4 gleichen oder verschiedenen Resten $R^{11}$, sie besitzen bevorzugt 1 bis 8 Kohlenstoffatome und die Alkylgruppen der Alkoxy- oder Alkylthioreste besitzen beispielsweise die Bedeutung der vorne genannten Alkylgruppen.

Die genannten Aryle können beliebig substituiert sein, beispielsweise mit 1 - 4 gleichen oder verschiedenen Substituenten $R^{11}$, sie besitzen bevorzugt 6, 10 oder 14 Kohlenstoffatome und bedeuten beispielsweise Phenyl, 1-Naphthyl, 2-Naphthyl, 1-Anthracenyl, 2-Anthracenyl oder 9-Anthracenyl.

Die genannten Hetaryle können beliebig substituiert sein, beispielsweise mit 1 - 4 gleichen oder verschiedenen Substituenten $R^{11}$, und bedeuten beispielsweise Furyl, 2-Furyl, 3-Furyl, Thienyl, 2-Thienyl, 3-Thienyl, Pyrrolyl, 1-Pyrrolyl, 2-Pyrrolyl, 3-Pyrrolyl, Isoxazolyl, 3-Isoxazolyl, 4-Isoxazolyl, 5-Isoxazolyl, Isothiazolyl, 3-Isothiazolyl, 4-Isothiazolyl, 5-Isothiazolyl, Pyrazolyl, 1-Pyrazolyl, 3-Pyrazolyl, 4-Pyrazolyl, 5-Pyrazolyl, Oxazolyl, 2-Oxazolyl, 4-Oxazolyl, 5-Oxazolyl, Thiazolyl, 2-Thiazolyl, 4-Thiazolyl, 5-Thiazolyl, Imidazolyl, 1-Imidazolyl, 2-Imidazolyl, 4-Imidazolyl, 5-Imidazolyl, 1,2,3-Thiadiazolyl, 1,2,4-Thiadiazolyl, 1,2,5-Thiazolyl, 1,3,4-Thiadiazolyl, Tetrazolyl, 1,2,3,4-Thiatriazolyl, 1,2,3,4-Oxatriazolyl, Pyridyl, 2-Pyridyl, 3-Pyridyl, 4-Pyridyl, Pyridazinyl, 3-Pyridazinyl, 4-Pyridazinyl, Pyrimidinyl, 2-Pyrimidinyl, 4-Pyrimidinyl, 5-Pyrimidinyl, Pyrazinyl, 2-Pyrazinyl, 3-Pyrazinyl, 1,2,4-Triazinyl, 1,3,5-Triazinyl oder 1,2,4,5-Tetrazinyl.

Dabei können benachbarte Substituenten des Heteroaromaten kondensiert sein zu einem aromatischen oder heteroaromatischen, gesättigten oder teilweise ungesättigten carbocyclischen oder heterocyclischen Ring der seinerseits mit 1 - 4 gleichen oder verschiedenen Substituenten $R^{11}$ substituiert sein kann, so daß Hetaryl auch kondensierte Ringsysteme umfaßt wie z.B. Benzofuranyl, Isobenzofuranyl, 1-Benzothienyl, 2-Benzothienyl, Indolyl, Isoindolyl, Benzisoxazolyl, Benzoxazolyl, Benzisothiazolyl, Benzthiazolyl, 2-Benzthiazolyl, 4-Benzthiazolyl, 5-Benzthiazolyl, 6-Benzthiazolyl, 7-Benzthiazolyl, Indazolyl, Benzimidazolyl, Benzthiazolyl, Benzofurazanyl, Dibenzofuranyl, Dibenzothienyl, Acridinyl, Phenathridinyl, Carbazolyl, Chinolinyl, Isochinolinyl, Phthalazinyl, Chinazolinyl, Chinoxalinyl, Cinnolinyl, 1,5-Naphthyridinyl, 1,6-Naphthyridinyl, 1,7-Napthyridinyl, 1,8-Napthyridinyl, Pteridinyl, Pyrrolopyridinyl, Pyrrolopyridazinyl, Pyrrolopyrimidinyl, Pyrrolopyrazinyl, Pyrrolotriazinyl, Furopyridinyl, Furopyridazinyl, Furopyrimidinyl, Furopyrazinyl, Furotriazinyl, Thienopyridinyl, Thienopyridazinyl, Thienopyrimidinyl, Thienopyrazinyl, Thienotriazinyl, Imidazopyridinyl, Imidazopyridazinyl, Imidazopyrimidinyl, Imidazopyrazinyl, Pyrazolopyridinyl, Pyrazolopyridazinyl, Pyrazolopyrimidinyl, Pyrazolopyrazinyl, Isoxazolopyridinyl, Isoxazolopyridazinyl, Isoxazolopyrimidinyl, Isoxazolopyrazinyl, Oxazolopyridinyl, Oxazolopyridazinyl, Oxazolopyrimidinyl, Oxazolopyrazinyl, Thiazolopyridinyl, Thiazolopyridazinyl, Thiazolopyrimidinyl, Thiazolopyrazinyl, Isothiazolopyridinyl, Isothiazolopyridazinyl, Isothiazolopyrimidinyl, Isothiazolopyrazinyl, Triazolopyridinyl, Triazolopyridazinyl, Triazolopyrimidinyl oder Triazolopyrazinyl.

Der heteroaromatische Ring

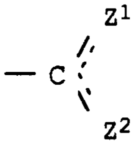

oder

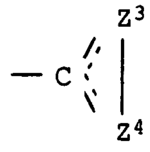

bedeutet beispielsweise Furyl, Thienyl, Pyrrolyl, Isoxazolyl, Isothiazolyl, Pyrazolyl, Oxazolyl,Thiazolyl, Imidazolyl, 1,2,3-Thiadiazolyl, 1,2,4-Thiadiazolyl, 1,2,5-Thiazolyl, 1,3,4-Thiadiazolyl, 1,3,4-Oxadiazolyl, Pyridyl, Pyridazinyl, Pyrimidinyl, Pyrazinyl, 1,2,4-Triazinyl oder 1,3,5-Triazinyl, wobei die Reste mit der Gruppe

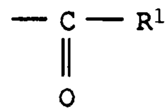

an jedem Ringkohlenstoffatom des heteroaromatischen Ringes D stehen können.

Dabei können 2 benachbarte Substituenten X oder $R^{11}$ kondensiert sein zu einem aromatischen oder heteroaromatischen, gesättigten oder teilweise ungesättigten carbocyclischen oder heterocyclischen Ring, der seinerseits mit 1 - 4 gleichen oder verschiedenen Substituenten $R^{11}$ substituiert sein kann, so daß der oben genannte heteroaromatisch Ring auch kondensierte Ringsysteme umfaßt wie zum Beispiel Benzofuranyl, Isobenzofuranyl, Benzothienyl, Indolyl, Isoindolyl, Benzisoxazolyl, Benzoxazolyl, Benzisothiazolyl, Indazolyl, Benzimidazolyl, Benzthiazolyl, Benzofurazanyl, Dibenzofuranyl, Dibenzothienyl, Acridinyl, Phenanthridinyl, Carbazolyl, Chinolinyl, Isochinolinyl, Phthalazinyl, Chinazolinyl, Chinoxalinyl, Cinnolinyl, Naphthyridinyl, Pteridinyl, Pyrrolopyridinyl, Pyrrolopyridazinyl, Pyrrolopyrimidinyl, Pyrrolopyrazinyl, Pyrrolotriazinyl, Furopyridinyl, -Furopyridazinyl, Furopyrimidinyl, Furopyrazinyl, Furotriazinyl, Thienopyridinyl, Thienopyridazinyl, Thienopyrimidinyl, Thienopyrazinyl, Thienotriazinyl, Imidazopyridinyl, Imidazopyridazinyl, Imidazopyrimidinyl, Imidazopyrazinyl, Pyrazolopyridinyl, Pyrazolopyridazinyl, Pyrazolopyrimidinyl, Pyrazolopyrazinyl, Isoxazolopyridinyl, Isoxazolopyridazinyl, Isoxazolopyrimidinyl, Isoxazolopyrazinyl, Oxazolopyridinyl, Oxazolopyridazinyl, Oxazolopyrimidinyl, Oxazolopyrazinyl, Thiazolopyridinyl, Thiazolopyridazinyl, Thiazolopyrimidinyl, Thiazolopyrazinyl, Isothiazolopyridinyl, Isothiazolopyridazinyl, Isothiazolopyrimidinyl Isothiazolopyrazinyl, Triazolopyridinyl, Triazolopyridazinyl, Triazolopyrimidinyl oder Triazolopyrazinyl.

Die genannten Heterocyclylreste sind gesättigt oder teilweise ungesättigt, können beliebig substituiert sein, beispielsweise mit 1 - 4 gleichen oder verschiedenen Substituenten $R^{11}$, und bedeuten beispielsweise 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,2,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,5-Dihydrofur-2-yl, 2,5-Dihydrofur-3 yl, 2,3-Dihydrothien-2-yl, 2,3-Dihydrothien-3-yl, 2,5-Dihydrothien-3-yl, 2,5-Di-hydrothien-2-yl, 2,4-Pyrrolin-2-yl, 2,3-Pyrrolin-3-yl, 2,5-Pyrrolin-2-yl, 2,5 Pyrrolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothia-zolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-3-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Diyhdropyrazol-1-yl, 3,4-Dihydropyrazol-2-yl, 3,4-Dihydropyrazol-4-yl, 3,4-Dihydropyrazol-5-yl, 4,5-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-4-yl, 3,4-Dihydrooxazol-5-yl 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, Oxazol-2-in-2-yl, 2-Tetrahydropyranyl, 1,3-Dioxolan-2-yl, Thiazol-2-in-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl, N-Morpholinyl oder Dihydrochinazolinyl.

$R^{11}$ ist beliebig substituiert, beispielsweise mit 1 - 4 gleichen oder verschiedenen Substituenten $R^{12}$, und bedeutet beispielsweise Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Alkoxy, Alkenyloxy, Alkinyloxy, Cycloalkyloxy, Aryloxy, Hetaryloxy, Heterocyclyloxy, Cycloalkenyloxy, Alkoximino, Alkenyloximino, Alkinyloximino, Cycloalkyloximino, Cycloalkenyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Alkylaminocarbonyl, Dialkylaminocarbonyl, Alkenylaminocarbonyl, Dialkenylaminocarbonyl, Alkylthio, Alkenylthio, Alkinylthio, Cycloalkylthio, Arylthio, Hetarylthio, Heterocyclylthio, Cycloalkenylthio, Alkylamino, Alkenylamino, Alkinylamino, Cycloalkylamino, Arylamino, Hetarylamino, Heterocyclylamino, Cycloalkenylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloylkenylsulfonyl, Alkylsulfinyl, Alkenylsulfinyl, Alkinylsulfinyl, Cycloalkylsulfinyl, Arylsulfinyl, Hetarylsufinyl, Heterocyclylsulfinyl, Cyclolkenylsulfinyl, Alkylcarbonylamino, Alkenylcarbonylamino, Alkinylcarbonylamino, Amino, Cycloalkylcarbonylamino, Arylamino, Hetarylamino oder Heterocyclylamino.

$R^{12}$ kann beliebig substituiert sein und bedeutet beispielsweise Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Alkoxy, Alkenyloxy, Alkinyloxy, Cycloalkyloxy, Aryloxy, Hetaryloxy, Heterocyclyloxy, Cycloalkenyloxy, Alkoximino, Alkenyloximino, Alkinyloximino, Cycloalkyloximino, Cycloalkenyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Alkylaminocarbonyl, Dialkylaminocarbonyl, Alkenylaminocarbonyl, Dialkenylaminocarbonyl, Alkylthio, Alkenylthio, Alkinylthio, Cycloalkylthio, Arylthio, Hetarylthio, Heterocyclylthio, Cycloalkenylthio, Alkylamino, Alkenylamino, Alkinylamino, Cycloalkylamino, Arylamino, Hetarylamino, Heterocyclylamino, Cycloalkenylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cyclolkenylsulfinyl, Alkylcarbonylamino, Alkenylcarbonylamino, Alkinylcarbonylamino, Amino, Cycloalkylcarbonylamino, Arylamino, Hetarylamino oder Heterocyclylamino.

Die neuen Verbindungen können beispielsweise nach folgenden Verfahren hergestellt werden:

Die Ketoester 1 können durch Wittig-Reaktion mit $(C_6H_5)_3P^+\text{-}CH_2\text{-}O\text{-}CH_3Cl^-$, $(C_6H_5)_3P^+\text{-}CH_3Hal^-$, $(C_6H_5)_3P^+\text{-}CH_2\text{-}R_5Hal^-$(Hal = Halogen (Cl, Br, J)) oder $(C_6H_5)_3P^+\text{-}CH_2\text{-}ClCl^-$ bzw. durch Reaktion mit Methoxyamin in die Wirkstoffe 2 überführt werden (Schema 1).

$T^1$ ist der substituierte heteroaromatische Ring

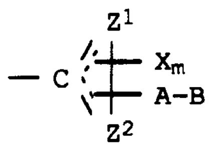

oder

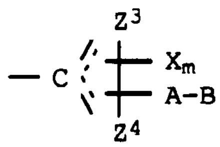

Schema 1

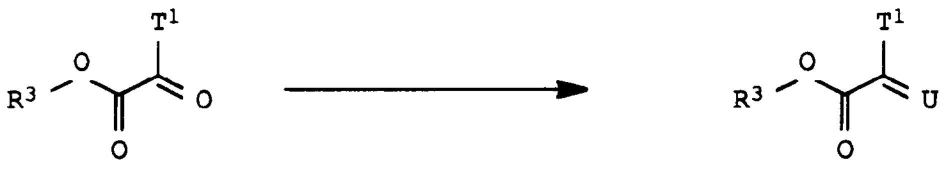

1 2

U = CH-$OR^2$, $CH_2$, CH-$R^2$, CH-Cl, $NOR^2$, $R^3$ = Alkyl

Durch Reaktion der Chlorvinylderivate 3 mit Thiolen R2-SH unter alkalischen Bedingungen sind die Thioenolether 4 erhältlich (Schema 2).

Schema 2

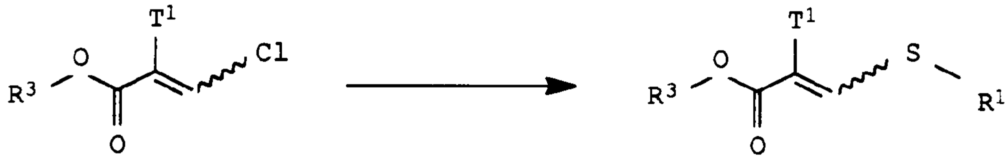

3 4

Aus den Estern 5 sind durch Reaktion mit Aminen $HNR_3R_4$ die Amide 6 erhältlich. Außerdem können die Ester 5 auch mit Carbonsäureesterenolaten (siehe z.B. E. Tao et al., Org. Prep. Proc. Int. Briefs 17 (1985), 235) zu den β-Ketoestern 7 umgesetzt werden, die nach Decarboxylierung mit Lithiumchlorid in Dimethylsulfoxid (siehe z.B. S. Takai et al., Tetrahedron Letters 49 (1975), 4389) die Ketone 8 liefern (Schema 3).

Schema 3

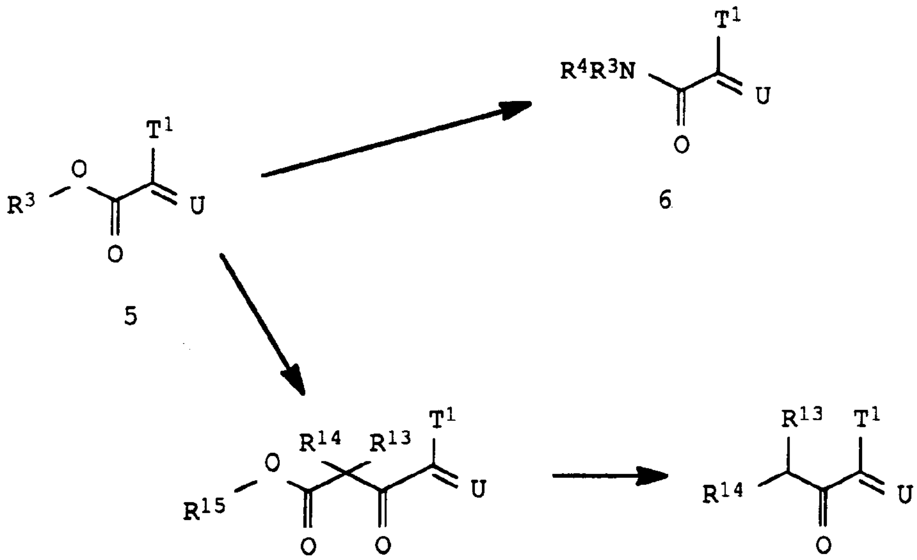

6

5

7 8

$R^{13}$, $R^{14}$ = H, $C_1$-$C_5$-Alkyl
$R^{15}$ = $C_1$-$C_5$-Alkyl

Die α-Ketoester 2 können z.B. nach den in Schema 4 gezeigten oder weiteren Literaturmethoden (siehe z.B. L. Weinstock et al., Synthetic Communications 11, 943 - 946 (1981); J. March, Advanced Organic Chemistry, 3. Auflage 1985, J. Wiley & Sons. R. Larock, Comprehensive Organic Transformations, 1. Auflage 1989, VCH Publishers; M. Fieser, Reagents for Organic Synthesis, J. Wiler & Sons; S. Patai, The Chemistry of Acid Derivatives, J. Wiley & Sons) hergestellt werden.

Schema 4

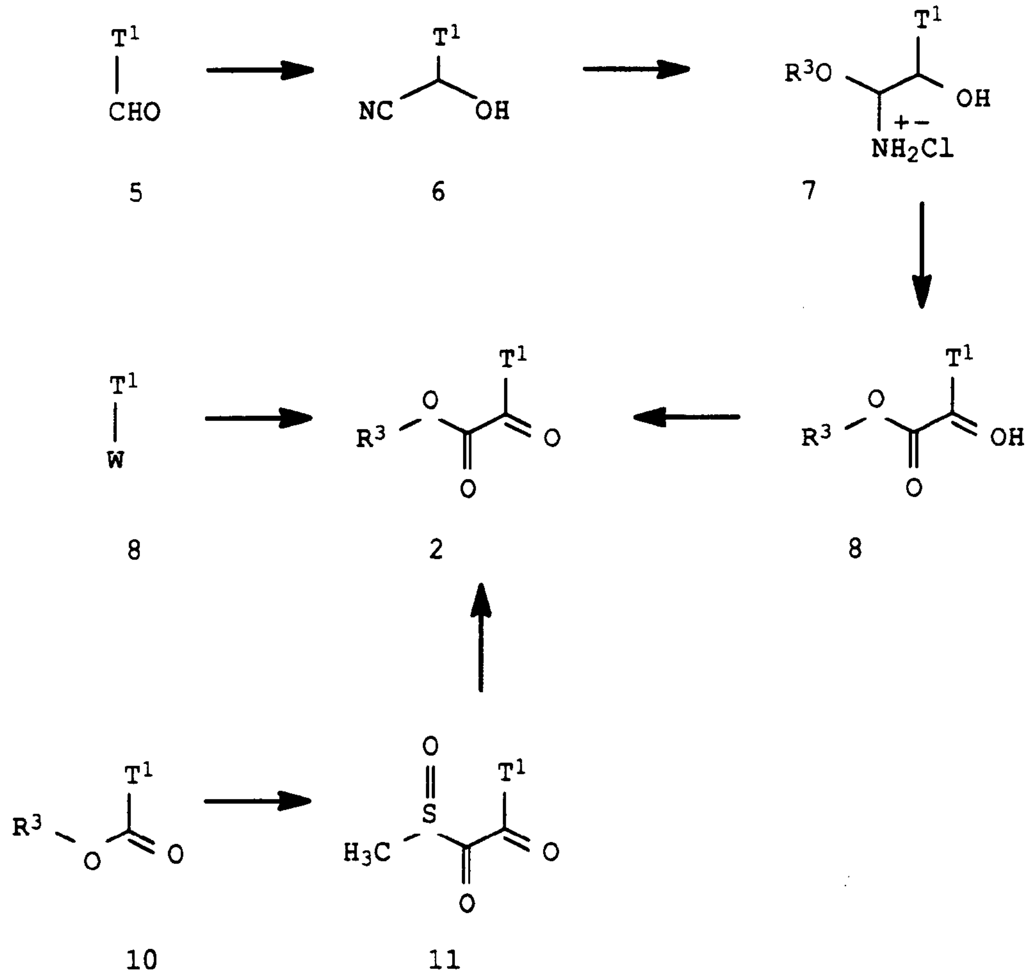

5 6 7

8 2 8

10 11

So können z.B. die α-Ketoester 2 durch Reaktion der entsprechenden Heteroaromaten 9 (W = Wasserstoff) mit Oxalsäureestern oder Oxalsäureesterhalogeniden bzw. -imidazolid in Gegenwart von sauren (z.B. $AlCl_3$, $TiCl_4$, $SnCl_4$, $ZnCl_2$ oder $FeCl_3$ analog EP 385357) oder basischen (z.B. Pyridin, Triethylamin, Ethyldiisopropylamin, Lithiumdiisopropylamid oder Butyllithium) Katalysatoren hergestellt werden. Außerdem können die Heteroaromaten 9 (W = Wasserstoff oder Halogen) durch Reaktion mit Mg, Li, Butyllithium, Lithiumdiisopropylamid oder Lithium-tetramethylpiperidid (analog EP 253213) zu den entsprechenden metallierten Heteroaromaten umgesetzt werden, die dann mit Oxalsäureestern, Oxalsäureesterhalogeniden oder Oxalsäureesterimidazolid zu den α-Ketoestern 2 umgesetzt werden können. Außerdem können durch Addition von HCN an die Aldehyde 5 die Cyanhydrine 6 erhalten werden. Diese können dann mit HCl und Methanol zu den Imidoester-hydrochloriden 7 umgesetzt werden, die anschließend zu den α-Hydroxyestern 8 hydrolysiert werden können. Durch Oxidation der α-Hydroxyester 8 werden dann die a-Ketoester 2 erhalten (analog EP 422597).

Außerdem können die Ester 10 mit Dimethylsulfoxid unter alkalischen Bedingungen zu den β-Ketosulfoxiden 11 umgesetzt werden. Die β-Ketosulfoxide 11 liefern dann durch Umsetzung mit Brom und anschließend mit Salzsäure in Gegenwart eines Alkohols $R^3$-OH in einer Pummerer-Reaktion (siehe z.B. JACS 1966, 5498; Synthesis 1982, 41) die α-Ketoester 2.

Die Enolether 12 können z.B. hergestellt werden durch Reaktion der entsprechenden heteroaromatisch substituierten Essigesterderivate 13 unter alkalischen Bedingungen mit Ameisensäureestern (analog Organikum, 15. Auflage 1976, S. 548, VEB) und anschließender Alkylierung des gebildeten Enolats. Außerdem können die Enolether 12 hergestellt werden durch Eliminierung von $R^2$-OH (analog T. Yamada et al., Journal Chem. Soc. Chem. Commun. 1980, 838 und dort zitierte Literatur) aus den Acetalen 14 (analog T. Mukaiyama et al., Chem Lett. 1976, 769).

Alternativ können auch die Acrylester 15 durch aufeinanderfolgende Reaktion mit Brom, einem Alkoholat $R^2$-O-M (M = Na, K, Li, Mg) und einer Protonensäure wie z.B. $NaHSO_4$ (analog G. Shaw et al., J. Chem. Soc. 1958, 153 und dort zitierte Literatur) zu den Acrylestern 12 umgesetzt werden (Schema 5).

Schema 5

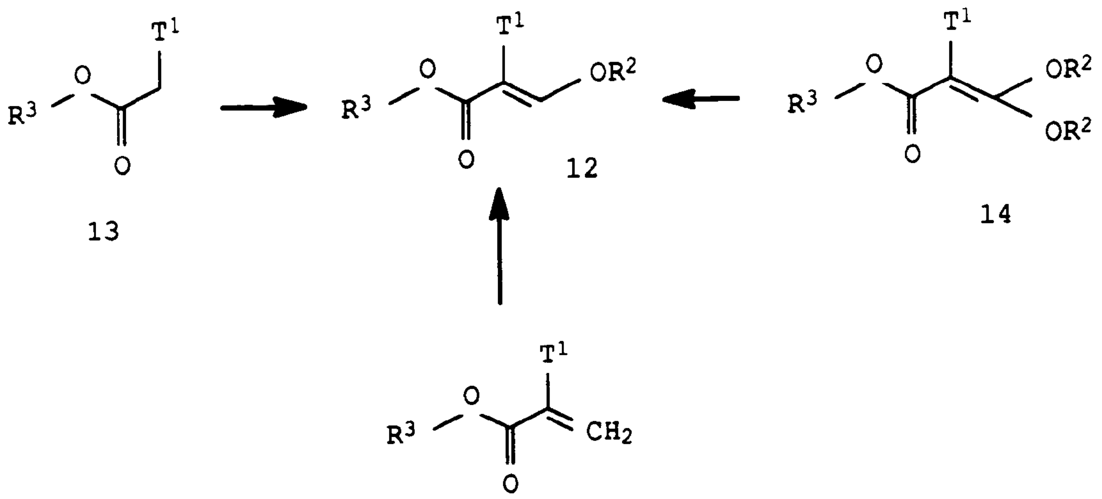

13 12 14

15

Wichtige Zwischenprodukte zur Synthese der heteroaromatischen Verbindungen gemäß Anspruch 1 sind die Oximether 19 und 20, die sich durch Nitrosierung/Alkylierung der β-Ketoester 16 zum Oximether 18 und anschließender Umsetzung von 18 mit Halogenen (Hal, $Cl_2$ oder $Br_2$) bzw. Dimethylformamid-dimethylacetal (DMF - DMA) herstellen lassen (Schema 6).

Schema 6

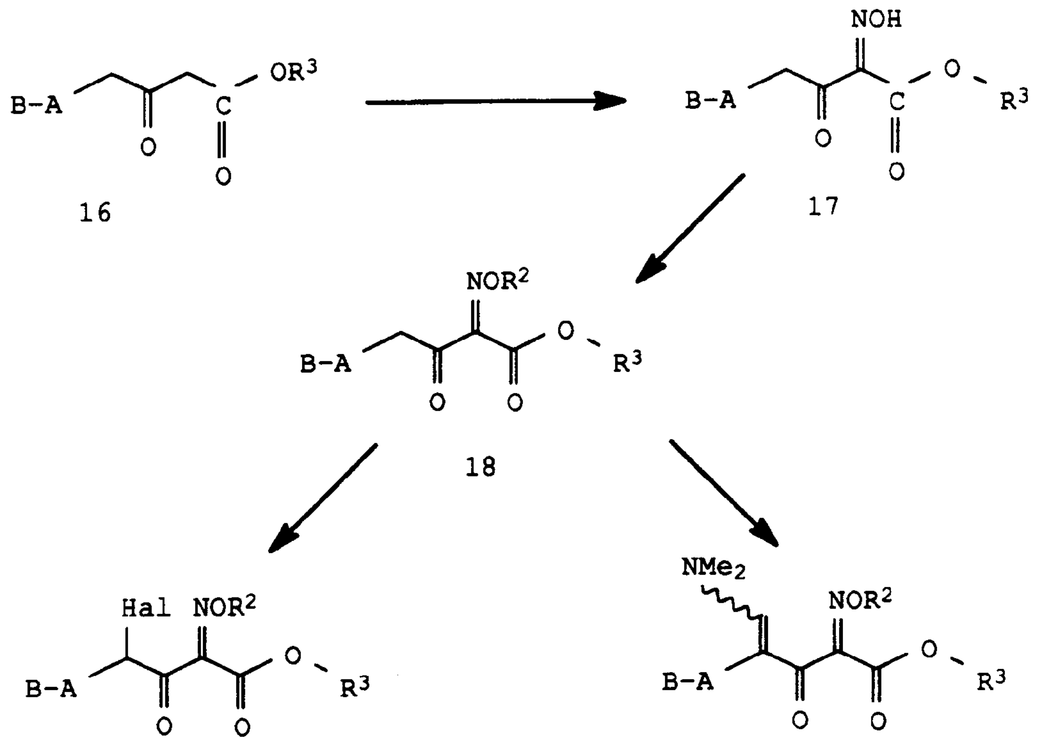

16

17

18

19

20

Die α-Halogen-carbonylverbindungen 19 können dann zu Thiazolen 21 (analog M. Tomlinsen, J. Chem. Soc. 1935, 1030), Imidazolen 22 (analog H. Brederck et al., Chem Ber. 86 (1953), 88 bzw. Oxazolen 23 (analog Z. Itov et al., Khim.-farm. Zh. 23, 452 (1989); Schema 7) cyclisiert werden.

Schema 7

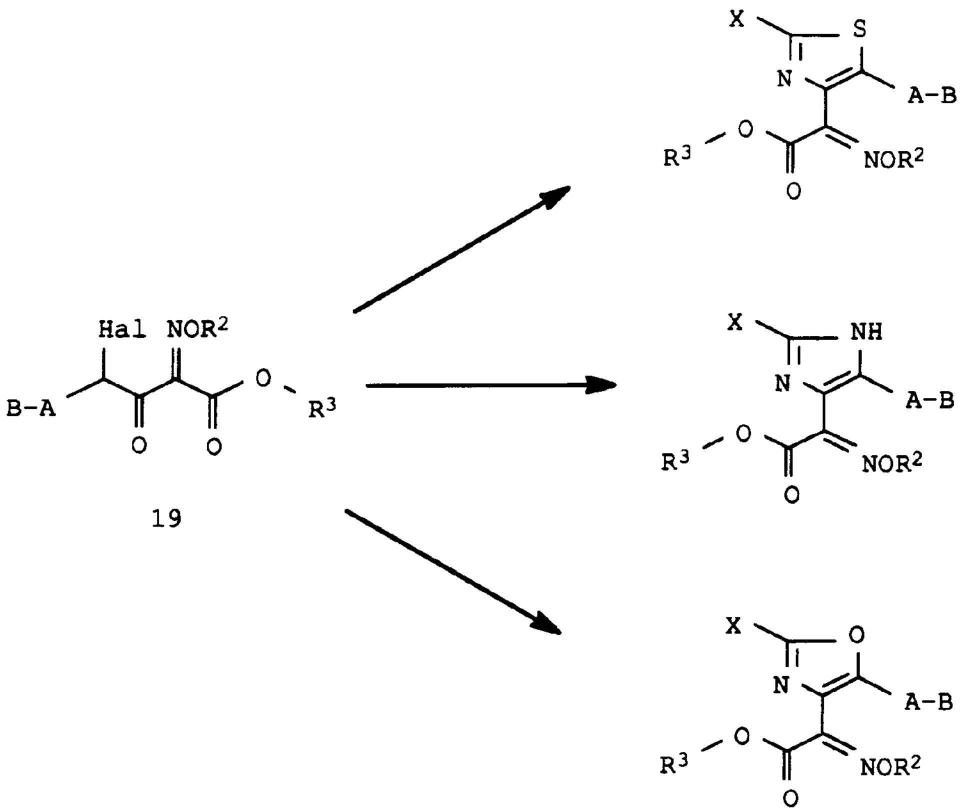

19

21

22

23

Die Enamine 20 können zu Isoxazolen 24 (analog P. Schenone, J. Heterocycl. Chem. 20 (1983), 645), Pyrazolen 25 (J. Heterocycl. Chem. 14 (1977), 345) oder Pyrimidinen 26 umgesetzt werden (Schema 8).

Schema 8

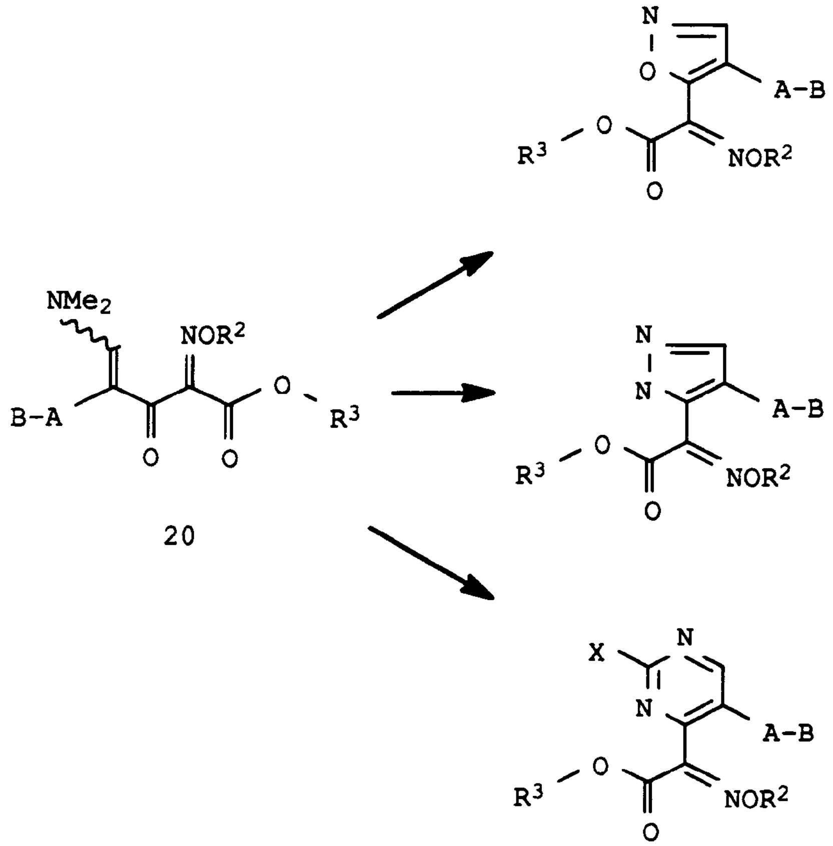

20

24

25

26

Weitere wichtige Zwischenprodukte zur Synthese der heteroaromatischen Verbindungen gemäß Anspruch 1 sind die Oximether 29 und 30, die sich durch selektive Oximierung der α,γ-Diketoester 27 (hergestellt analog Organikum, 15. Auflage 1976, S. 584) zu den Oximethern 28 und anschließender Halogenierung (Hal = Cl, Br) bzw. durch Umsetzung der Verbindungen 28 mit Dimehylformamid-dimethylacetal (DMF = DMA) herstellen lassen (Schema 9).

Schema 9

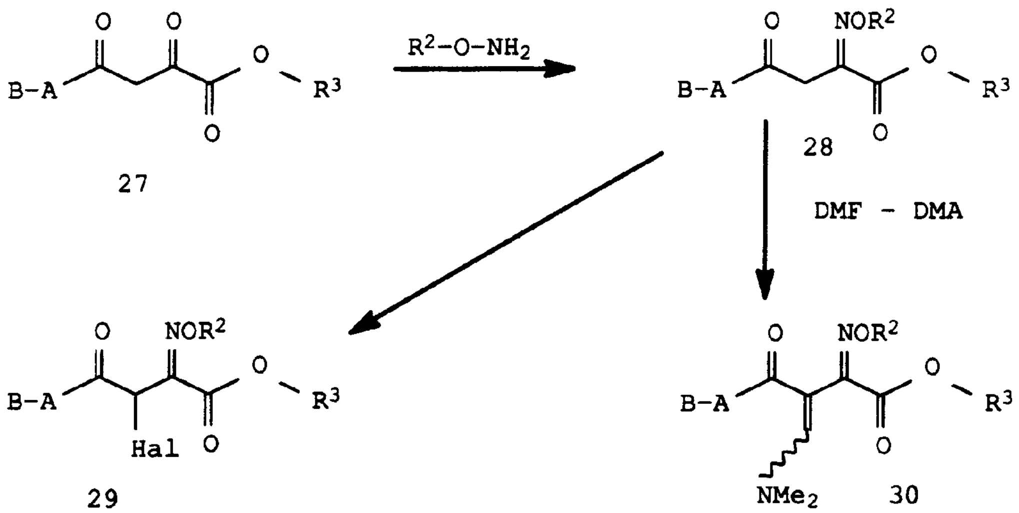


27 28 29 30

Die α-Halogen-carbonylverbindung 29 kann dann zu Thiazolen 31 Imidazolen 32, Oxazolen 33 oder Furanen 34 (analog Winberg et al., J. Amer. Chem. Soc. 82 (1960), 1428) umgesetzt werden (Schema 10, vgl. Schema 7).

Schema 10

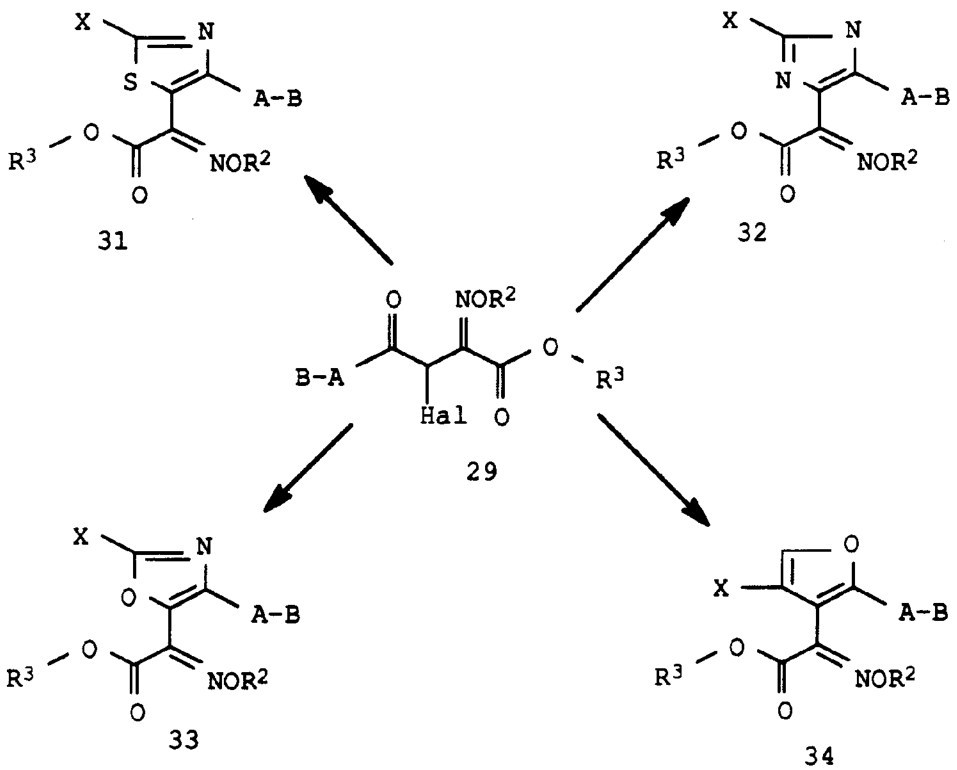

21

31 32 29 33 34

Das Enamin 30 kann dann zu Isoxazolen 35, Pyrazolen 36 oder Pyrimidinen 37 umgesetzt werden (Schema 11, vgl. Schema 8).

Schema 11

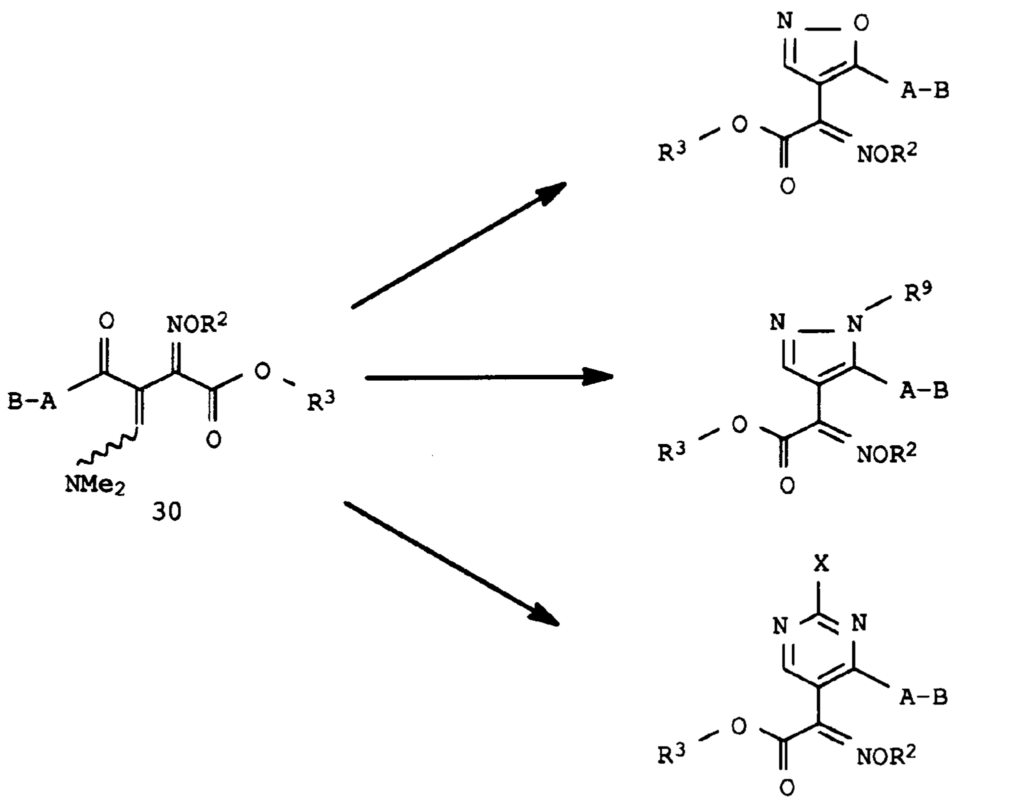

30

35

36

37

Außerdem sind Isoxazole wie 41 aus dem Brenztraubensäurederivat 38 zugänglich. 38 wird mit N-Methylmorpholin-N-oxid zum Aldehyd 39 oxidiert, der mit Hydroxylamin zum Oxim 40 umgesetzt wird. Das Oxim 40 reagiert mit Acetylenen in Gegenwart von NaOCl zum Isoxazol 41 (Schema 12).

Schema 12

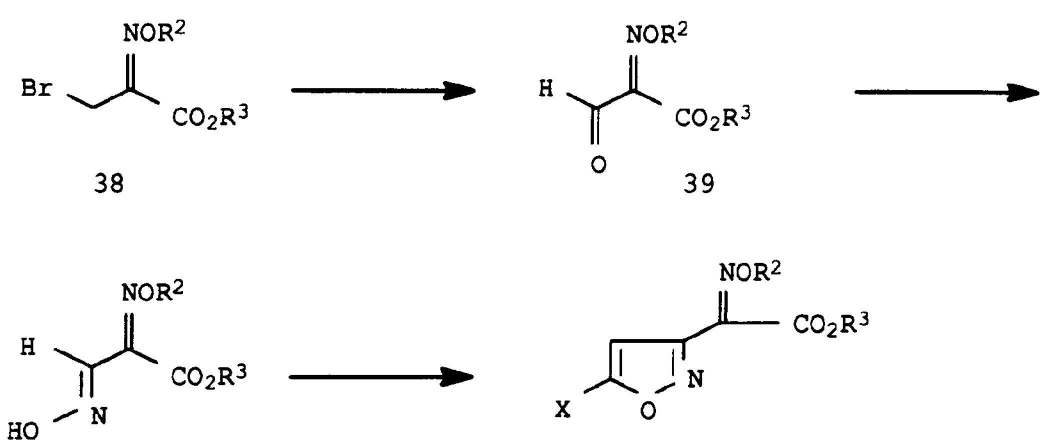

38

39

40

41

Die heteroaromatischen Verbindungen 45 können durch Umsetzung der Halogenide 44 (Hal = Cl, Br) mit den entsprechenden Nucleophilen hergestellt werden. Die Bromide 44 werden durch radikalische Halogenierung der heteroaromatischen Verbindungen 42 oder durch Etherspaltung der Methylether 43 erhalten (Schema 13).

Schema 13

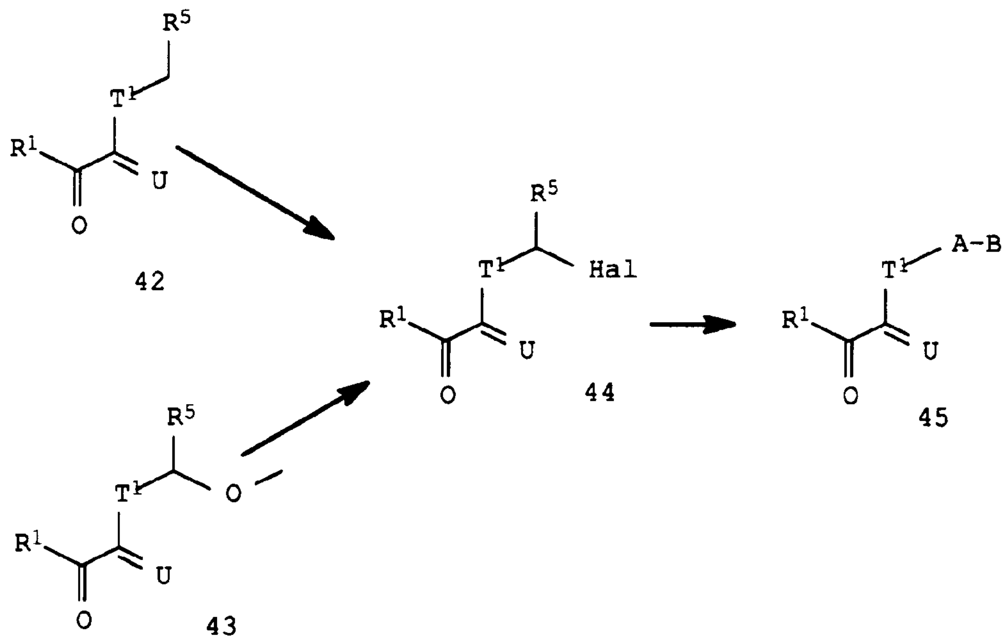

42 43 44 45

A: $-CH(R^5)-O-N=C(R^6)-$, $-CH(R^5)-O-N=$, $-CH(R^5)-S-$, $-CH(R^5)-O-$, $-CH(R^5)-NR^7-$, $-CH(R^5)-O-C(=O)-$

Die heteroaromatischen Verbindungen 48 können durch Umsetzung der Carbonylverbindungen 47 mit Oximethern $H_2N-O-B$, Azinen $H_2N-N=B$ bzw. durch Wittig-Reaktion mit den entsprechenden Phosphoniumsalzen, Phosphonaten oder Phosphinoxiden hergestellt werden. Die Carbonylverbindungen 47 werden durch Oxidation der Halogenide 42 mit N-Methyl-morpholin-N-oxid oder durch Schwermetallsalz-katalysierte Hydrolyse der Dihalogenide 46 erhalten (Schema 14). Die Dihalogenide 46 sind durch radikalische Halogenierung der Monohalogenide 44 oder durch radikalische Dihalogenierung der heteroaromatischen Verbindungen 42 zugänglich.

Schema 14

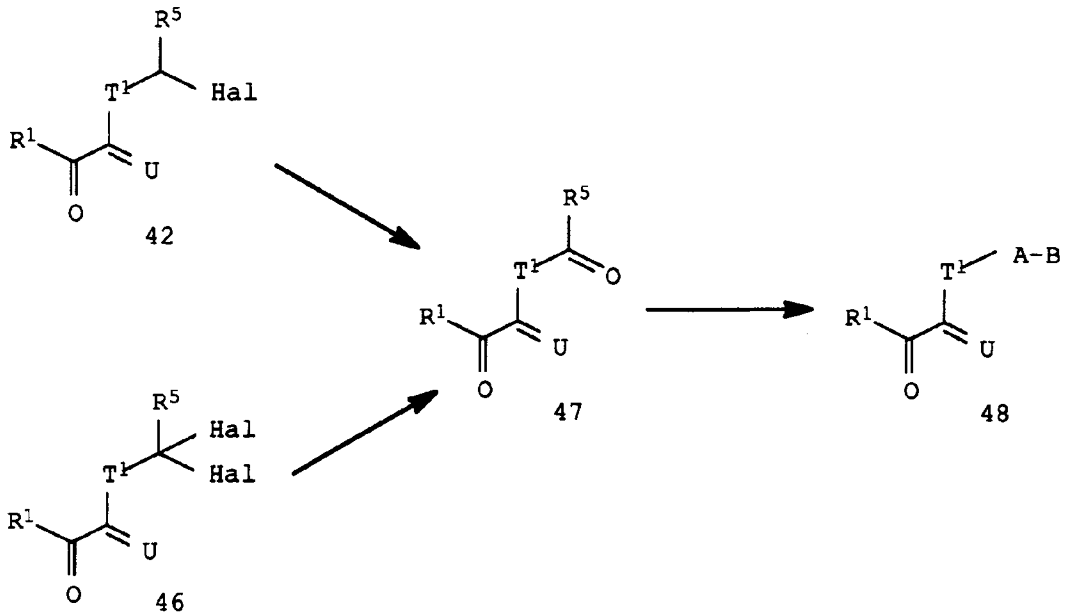

42 47 48 46

A: $-C(R^5)=N-O-$, $-C(R^5)=N-N=$, $-C(R^5)-C(R^7-$

Alternativ können die Halogenide 42 zu den entsprechenden Phosphorverbindungen 49 (Phosphoniumsalze, Phosphonate oder Phosphinoxide) und dann mit Carbonylverbindungen in einer Wittig-Reaktion zu den entsprechenden Olefinen 50 umgesetzt werden (Schema 15).

Schema 15

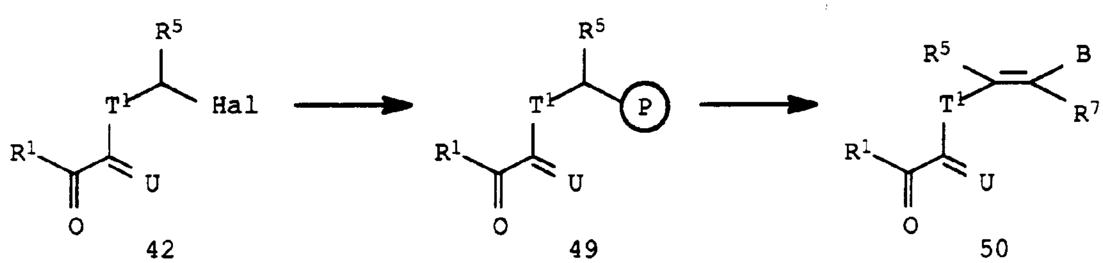

42 49 50

Außerdem können die Ketoester 51 in der Seitenkette halogeniert (Hal= Cl, Br) und dann mit den entsprechenden Nukleophilen zu den heteroaromatischen Verbindungen 53 umgesetzt werden. Aus den Ketoestern 53 sind dann analog Schema 1 die erfindungsgemäßen Verbindungen 54 erhältlich (Schema 16).

Schema 16

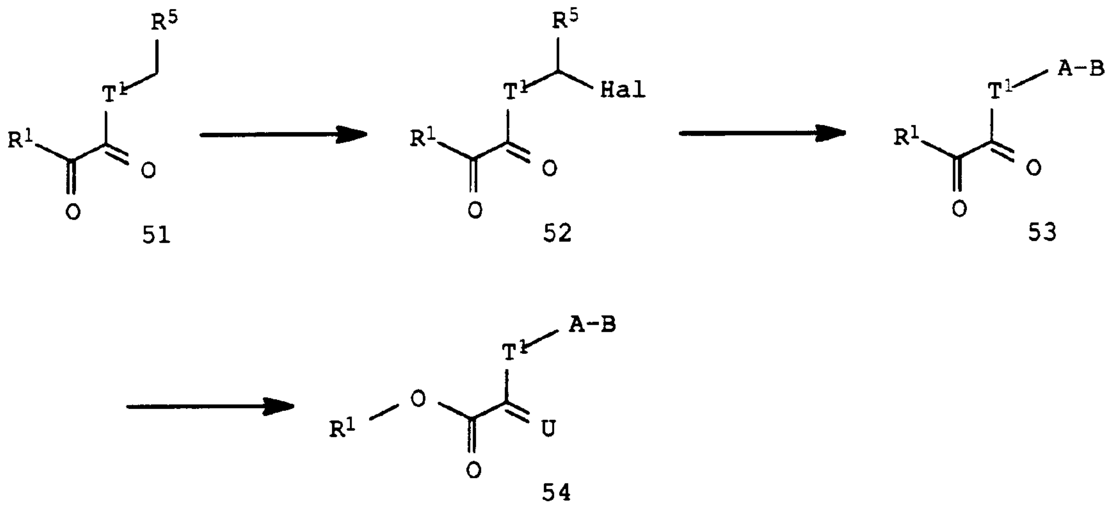

51 52 53 54

Die folgenden Beispiele erläutern die Herstellung der neuen Verbindungen.

Beispiel 1

β-Methoxy-α-(N-phenylpyrazolyl-4)-acrylsäuremethylester (Tabelle 3, Nr. 7 und 8)

a) α-Hydroxy-α-(N-phenylpyrazolyl-4)-essigsäuremethylester

Eine Mischung von 63 g (0,37 mol) N-Phenylpyrazol-4-carbaldehyd in 300 ml Diethylether und 58 g (0,9 mol) KCN und 48 g (0,9 mol) $NH_4Cl$ in 300 ml Wasser wird über Nacht bei Raumtemperatur (20°C) gerührt. Man trennt die Phasen und extrahiert die wäßrige Phase mit Diethylether. Die vereinigten organischen Phasen werden über $MgSO_4$ getrocknet und eingeengt. Der Rückstand wird mit Hexan/Essigester 4:1 säulenchromatographisch gereinigt. Man erhält 50 g (Reinheit ca. 70 %) des Cyanhydrins, das direkt weiter umgesetzt wird.

Man löst das Cyanhydrin in 200 ml Methanol, gibt 150 ml 2n HCl in Ether hinzu und rührt über Nacht bei Raumtemperatur. Dann wird die Reaktionsmischung eingeengt, mit 200 ml Wasser versetzt und 1 h bei 80°C gerührt. Man kühlt auf Raumtemperatur und extrahiert dreimal mit Methylenchlorid. Die vereinigten organischen Phasen werden über $MgSO_4$ getrocknet und eingeengt. Der Rückstand wird säulenchromatographisch mit Hexan/Essigester-Gemischen gereinigt. Man erhält 16 g (66 mmol, 18 %) der Titelverbindung als gelbes Öl.

$^1$H-NMR ($CDCl_3$; δ in ppm):
7,95 (s, 1 H, Pyrazolyl); 7,75 (s, 1 H, Pyrazolyl); 7,65 (d, 2H, J = 8 Hz, Phenyl; 7,4 (t, 2 H, J = 8 Hz, Phenyl); 7,3 (t, breit, 1 H, Phenyl); 5,3 (s, breit, 1 H, CH-O); 3,85 (s, 3 H, $OCH_3$); 3,7 (s, breit, 1 H, OH)

b) α-Keto-α-(N-phenylpyrazolyl-4)-essigsäuremethylester

Zu einer Mischung von 22 g (87 mmol) des α-Hydroxyesters aus Beispiel 1 a) in 50 ml $CH_2Cl_2$ und 0,6 g KBr, 1,2 g $NaH_2PO_4$, 1,7 g $Na_2HPO_4$ und 50 mg Tetramethylpiperidin-N-oxyl in 50 ml Wasser wird bei 20 - 30°C 160 g (0,57 mol) 26 %ige NaOCl-Lösung getropft. Man rührt 30 Minuten bei Raumtemperatur, trennt die Phasen und extrahiert die wäßrige Phase noch dreimal mit $CH_2Cl_2$. Die vereinigten organischen Phasen werden über $MgSO_4$ getrocknet und eingeengt. Der Rückstand wird säulenchromatographisch mit Hexan/Essigester-Gemischen gereinigt. Man erhält 15,5 g (62 mmol, 71 %) der Titelverbindung als farblosen Festkörper.

$^1$H-NMR ($CDCl_3$; δ in ppm):
8,8 (s, 1 H, Pyrazolyl); 8,4 (s, 1 H, Pyrazolyl); 7,75 (d, 1 H, J = 8 Hz, Phenyl); 7,5 (t, 2 H, J = 8 Hz, Phenyl); 7,4 (t, breit, 2 H, Phenyl); 4,0 (s, 3 H, O-$CH_3$)

c) α-Keto-α-(N-phenylpyrazolyl-4)-acrylsäuremethylester (Tabelle 3,Nr. 7 und 8)

8,5 g (25 mmol) Methoxymethyl-triphenylphosphoniumchlorid in 50 ml Tetrahydrofuran wird bei 0°C portionsweise mit 2,1 g (19 mmol) Kalium-t-butanolat versetzt. Man rührt die rote Reaktionsmischung 15 min bei 0°C, gibt 4 g (16 mmol) des Ketoestes aus Beispiel 1 b), gelöst in 10 ml Tetrahydrofuran, hinzu und rührt ca. 3 Stunden bei Raumtemperatur. Nach beendeter Reaktion verdünnt man die Reaktionsmischung mit Wasser und extrahiert die wäßrige Phase dreimal mit Diethylether. Die vereinigten etherischen Phasen werden über $MgSO_4$ getrocknet und eingeengt. Der Rückstand wird säulenchromatographisch mit Hexan/Essigester-Gemischen gereinigt. Man erhält 1,3 g (4,7 mmol, 29 %) des trans-Isomeren und 2,0 g (7,2 mmol, 45 %) des cis-Isomeren der Titelverbindung als hellgelbe Öle.
$^1$H-NMR ($CDCl_3$; δ in ppm):
trans-Isomeres: 8,4 (s, 1 H, Pyrazolyl); 8,2 (s, 1 H, Pyrazolyl); 7,7 (d, breit, 2 H, Phenyl); 7,5 (s, 1 H, Vinyl); 7,4 (t, 1 H, J = 8 Hz, Phenyl); 7,25 (t, breit, 1 H, Phenyl); 4,0 (s, 3 H, O-$CH_3$); 3,8 (s, 3 H, O-$CH_3$)
cis-Isomeres: 8,05 (s, 1 H, Pyrazolyl); 7,65 (m, 3 H, Pyrazolyl, 2 x Phenyl); 7,45 (t, 2 H, J = 8 Hz, Phenyl); 7,25 (t, breit, 1 H, Phenyl); 6,9 (s, 1 H, Vinyl); 3,95 (s, 3 H, O-$CH_3$); 3,8 (s, 3 H, O-$CH_3$)

Beispiel 2

α-(N-Phenylpyrazolyl-4)-crotonsäuremethylester (Tabelle 3, Nr. 9)

12,5 g (30 mmol) Ethyl-triphenylphosphoniumjodid in 50 ml Tetrahydrofuran wird bei Raumtemperatur portionsweise mit 2 g (18 mmol) Kalium-t-butanolat versetzt. Man rüht die orange Reaktionsmischung 30 min. und gibt sodann 3,7 g (15 mmol) des Ketoesters aus Beispiel 1 b) und 0,5 ml Methanol hinzu. Nach 1 Stunde gibt man $NH_4Cl$-Lösung hinzu und extrahiert die wäßrige Phase dreimal mit Diethylether. Die vereinigten organischen Phasen werden über $MgSO_4$ getrocknet und eingeengt. Der Rückstand wird säulenchromatographisch mit Hexan/Essigester-Gemischen gereinigt. Man erhält 1,3 g (5 mmol, 33 %) der Titelverbindung (cis/trans = 1:1) als hellgelbes Öl.
$^1$H-NMR ($CDCl_3$; δ in ppm):
8,05, 8,02 (2 s, 1 H, Pyrazolyl); 7,6 - 7,8 (m, 3 H, Pyrazolyl, 2 x Phenyl); 7,45 (m, 2 H, Phenyl); 7,3 (m, 1 H, Phenyl); 7,15 (q, 1 H des trans-Isomeren, J = 9 Hz; Vinyl); 6,5 (q, 1 H des cis-Isomeren, J = 9 Hz, Vinyl); 3,85, 3,8 (2 s, 3 H, O-$CH_3$); 2,1 2,0(2 d, 3 H, J = 9 Hz, $CH_3$)

Beispiel 3

α-Keto-α-(5-methylthiazol-4)-essigsäuremethylester-O-methyloxim (Tabelle 3, Nr. 146 und 147)

a) β-Keto-α-methoximino-valeriansäuremethylester

500 g (3,84 mol) β-Ketovaleriansäuremethylester und 310 g (4,5 mol) $NaNO_2$ in 2 l Wasser werden bei 10°C innerhalb 1 Stunde mit 300 g (5 mol) Essigsäure versetzt. Dann entfernt man das Kühlbad, worauf die Reaktionstemperatur auf ca. 40°C steigt. Man läßt auf Raumtemperatur abkühlen und extrahiert die Reaktionsmischung dreimal mit Methylenchlorid. Die vereinigten organischen Phasen werden mit $NaHCO_3$-Lösung extrahiert, über $MgSO_4$ getrocknet und eingeengt. Der so erhaltene Rückstand wird in 2 l Aceton gelöst, portionsweise mit 600 g (4,3 mol) $K_2CO_3$ und anschließend tropfenweise mit 600 g (4,6 mol) Methyljodid versetzt. Man rührt über Nacht bei Raumtemperatur, saugt den unlöslichen Festkörper ab und engt das Filtrat ein. Der Rückstand wird im Vakuum destilliert, man erhält 390 g (2,25 mol, 59 %) der Titelverbindung als hellgelbe Flüssigkeit (Kp (25 mbar) = 100 - 104°C).
$^1$H-NMR ($CDCl_3$; δ in ppm):
4,1 (s, 3 H, O-$CH_3$); 3,9 (s, 3 H, O-$CH_3$); 2,8 (q, 2 H, J = 8 Hz, $CH_2$); 1,1 (t, 3 H, $CH_3$)

b) γ-Brom-β-keto-α-methoximino-valeriansäuremethylester

400 g (2,3 mol) des Ketons aus Beispiel 3 a) in 1 l Essigsäure wird bei ca. 25°C tropfenweise mit 380 g (2,38 mol) Brom versetzt. Man rührt 30 min. nach, verdünnt die Reaktionsmischung mit Wasser und extrahiert die wäßrige Phase dreimal mit Methylenchlorid. Die vereinigten organischen Phasen werden mit $NaHCO_3$-Lösung neutralgewaschen, über $MgSO_4$ getrockenet und eingeengt. Man erhält als Rückstand 560 g (2,22 mol, 97 %) der Titelverbindung als gelbes Öl.
$^1$H-NMR ($CDCl_3$; δ in ppm):
5,25 (q, 1 H, J = 7 Hz, CHBr); 4,15 (s, 1 H, O-$CH_3$); 3,9 (s, 3 H, O-$CH_3$); 1,8 (d, 3 H, $CH_3$)

c) α-Keto-α-(5-methylthiazolyl-4)-essigsäuremethylester-O-methyloxim (Tabelle 3, Nr. 146 und 147)

560 g (2,2 mol) des α-Bromketons aus Beispiel 3 b) in 2 l Methanol werden portionsweise mit 200 g Thioformamid (Reinheit ca. 70 %ig, ca. 2,3 mol) versetzt, wobei man die Reaktionstemperatur mit einem Kühlbad bei ca. 30°C hält. Man rührt über Nacht bei Raumtemperatur, gibt weitere 30 g Thioformamid hinzu und rührt 5 Stunden bei 40°C. Anschließend wird die Reaktionsmischung eingeengt und der Rückstand wird in Methylenchlorid aufgenommen. Die organische Phase wird mit $NaHCO_3$-Lösung neutralgewaschen, über $MgSO_4$ getrocknet und eingeengt. Der Rückstand wird säulenchromatographisch mit Hexan/Essigester-Gemischen gereinigt. Man erhält 233 g des cis-Isomeren und 190 g des trans-Isomeren (polares Isomeres) der Titelverbindung, jeweils stark verunreinigt. Durch Destillation im Hochvakuum erhält man 106 g (0,49 mol, 23 %) des cis-Isomeren als hellgelbes Öl. Durch Kristallisation mit Methyl-t-butylether und Hexan erhält man 71 g (0,33 mol, 15 % des trans-Isomeren (farblose Kristalle; Fp = 65°) der Titelverbindung.
$^1$H-NMR ($CDCl_3$; δ in ppm):
cis-Isomeres: 8,6 (s, 1 H, Thiazolyl); 4,05 (s, 3 H, O-$CH_3$); 3,95 (s, 3 H, O-$CH_3$); 2,7 (s, 3 H, $CH_3$) trans-Isomeres: 8,65 (s, 1 H, Thiazolyl); 4,1 (s, 3 H, O-$CH_3$); 3,9 (s, 3 H, O-$CH_3$); 2,4 (s, 3 H, $CH_3$)

Beispiel 4

α-Keto-α-(5-brommethylthiazolyl-4)-essigsäuremethylester-O-methyloxim (Tabelle 3, Nr. 148 und 149)

Eine Mischung von 106 g (0,5 mol) des Methylthiazols (cis-Isomeres aus Beispiel 3 c), 100 g (0,56 mol) N-Bromsuccinimid und 1 g Azoisobutyrodinitril in 1 l Tetrachlorkohlenstoff wird 3 Stunden zum Rückfluß erhitzt. Dann gibt man zusätzlich 20 g (0,11 mol) N-Bromsuccinimid hinzu und erhitzt weitere 2 Stunden. Man kühlt die Reaktionsmischung auf Raumtemperatur und saugt das ausgefallene Succinimid ab. Das Filtrat wird mit Wasser extrahiert, über $MgSO_4$ getrocknet und eingeengt. Der Rückstand wird säulenchromatographisch mit Hexan/Essigester-Gemischen gereinigt. Im Vorlauf erhält man kernbromierte und mehrfach bromierte Produkte, und anschließend 58 g des cis-Isomeren (ca. 90 %ige Reinheit; 0,18 mol, 36 %) und 11 g des trans-Isomeren (ca. 90 %ige Reinheit; 34 mmol, 7 %) der Titelverbindung als gelbe Öle.
$^1$H-NMR ($CDCl_3$; δ in ppm):
cis-Isomeres: 7,75 (s, 1 H, Thiazolyl); 5,0 (s, 2 H, $CH_2Br$); 4,1 (s, 3 H, O-$CH_3$) trans-Isomeres: 8,85 (s, 1 H, Thiazolyl); 4,55 (s, 2 H, $CH_2Br$); 4,1 (s, 3 H, O-$CH_3$); 3,9 (s 3 H, O-$CH_3$)

Beispiel 5

α-Keto-α-(5-(4'-(acetyl-O-isopropyloxim)-2'-methyl-phenoxymethyl)-thiazolyl-4)-essigsäuremethylester-O-methyloxim (Tabelle 3, Nr. 150 und 151)

7 g (30 mmol) 4-Acetyl-2-methylphenol-O-isopropyloxim in 25 ml Dimethylformamid wird portionsweise mit 0,9 g (40 mmol) Natriumhydrid versetzt. Nach Abklingen der Gasentwicklung gibt man portionsweise 10 g (30 mmol) des Brommethylthiazols aus Beispiel 4 hinzu und rührt 2 Stunden bei Raumtemperatur. Anschließend verdünnt man die Reaktionsmischung mit Wasser und extrahiert die wäßrige Phase dreimal mit Methyl-t-butylether. Die vereinigten organischen Phasen werden über $MgSO_4$ getrocknet und eingeengt. Als Rückstand erhält man 10,6 g des cis-Methyloximisomeren der Titelverbindung. Der Rückstand wird in 120 ml Methylenchlorid gelöst und mit 20 ml 2 n HCl in Ether versetzt. Man rührt 2 Tage bei Raumtemperatur und wäscht mit $NaHCO_3$-Lösung neutral. Die organische Phase wird über $MgSO_4$ getrocknet und eingeengt. Der Rückstand wird säulenchromatographisch mit Hexan/Essigester-Gemischen gereinigt. Man erhält 2,2 g (5,2 mmol, 18 %) des cis-Isomeren (Fp = 107°C) und 5,8 g (14 mmol, 46 %) des trans-Isomeren (Fp = 87°C) der Titelverbindung als farblose Festkörper.
$^1$H-NMR ($CDCl_3$; δ in ppm):
cis-Isomeres: 8,75 (s, 1 H, Thiazolyl); 7,55 (s, breit, 1 H, Phenyl); 7,45 (d, breit, 1 H, Phenyl); 6,8 (d, 1 H, J = 8 Hz, Phenyl); 5,5 (s, 2 H, O-$CH_2$); 4,45 (m, 1 H, O-CH($Me_2$)); 4,05 (s, 3 H, O-$CH_3$); 4,0 (s, 3 H, $OCH_3$); 2,3 (s, 3 H, $CH_3$); 2,2 (s, 3H, $CH_3$); 1,3 (d, 6 H, J = 7 Hz, $CH(CH_3)_2$) trans-Isomeres: 8,8 (s, 1 H, Thiozolyl); 7,5 (s, breit, 1 H, Phenyl; 7,4 (d, breit, 1 H, Phenyl); 6,25 (d, 1 H, J = 8 Hz, Phenyl); 5,15 (s, 2 H, O-$CH_2$); 4,45 (m, 1 H, O-CH($Me_2$)); 4,1 (s, 3 H, O-$CH_3$); 3,9 (s, 3 H, O-$CH_3$); 2,25 (s, 3 H, $CH_3$); 2,2 (s, 3 H, $CH_3$); 1,3 (d, 6 H, J = 7 Hz, $CH(CH_3)_2$)

Beispiel 6

α-Keto-α-(5-(4'-(acetyl-O-isopropyloxim)-2'-methyl-phenoxymethyl)-thiazolyl-4)-essigsäuremethylamid-trans-O-methyloxim (Tabelle 3, Nr.152)

Eine Suspension von 2 g (4,8 mmol) des trans-Methyloxims aus Beispiel 5 in 10 ml 40 %iger Methylamin-Lösung wird 2 Stunden bei 50°C gerürt. Man saugt den unlöslichen Festkörper ab und wäscht den Rückstand mit Aceton. Umkristallisation des Festkörpers aus Essigester/Hexan liefert 1 g (2,4 mmol, 50 %) der Titelverbindung als farblosen Festkörper (Fp = 147°C).
$^1$H-NMR ($CDCl_3$; δ in ppm):
8,85 (s, 1 H, Thiazolyl); 7,5 (s, breit, 1 H, Phenyl); 7,4 (d, breit, 1 H, Phenyl); 7,95 (s, breit, 1 H, NH); 6,75 (d, 1 H, J = 8 Hz, Phenyl); 5,2 (s, 2 H, O-$CH_2$); 4,45 (m, 1 H, O-CH($Me_2$)); 4,05 (s, 3 H, O-$CH_3$); 2,95 (d, 3 H, J = 5 Hz, N-$CH_3$); 2,25 (s, 3 H, $CH_3$); 2,2 (s, 3 H, $CH_3$); 1,3 (d, 6 H, J = 6 Hz, O-C($CH_3$)$_2$)

Beispiel 7

α-Keto-α-(4-methylisoxazolyl-5)-essigsäuremethylester-O-methyloxim (Tabelle 3, Nr. 153 und 154)

a) δ-Dimethylamino-β-keto-α-methoximino-γ-methylpenten-4-säuremethyl-ester

Zu einer Mischung von 100 g (0,578 mol) des Ketons aus Beispiel 3a und 75 g (0,91 mol) Natriumacetat in 200 ml Dimethylformamid-dimethylacetal gibt man tropfenweise 70 g (0,69 mol) Acetanhydrid. Dabei läßt man die Temperatur der Reaktionsmischung auf ca. 65°C ansteigen. Nach kurzer Zeit fällt die Reaktionstemperatur ab, worauf man den Kolbeninhalt auf ca. 75°C erhitzt und ca. 2 Stunden rührt. Anschließend zieht man die flüchtigen Bestandteile zuerst bei 25 mbar und dann im Hochvakuum ab. Man nimmt den Rückstand in Methylenchlorid auf, wäscht die organische Phase viermal mit Wasser, trocknet über $MgSO_4$ und engt ein. Als Rückstand erhält man 146 g (enthält ca. 10 % Dimethylformamid; Ausbeute quantitativ) der Titelverbindung als braunes Öl.
$^1$H-NMR ($CDCl_3$; δ in ppm):
7,5 (s, 1 H, Vinyl); 4,0 (s, 3 H, O-$CH_3$); 3,9 (s, 3 H, O-$CH_3$); 3,2 (s, 6 H, N($CH_3$)$_2$); 2,05 (s, 3 H, =C-$CH_3$)

b) α-Keto-α-(4-methylisoxazolyl-5)-essigsäuremethylester-O-methyloxim (Tabelle 3, Nr. 153 und 154)

146 g (0,57 mol) des Enaminoketons aus Beispiel 7 a und 60 g (0,86 mol) Hydroxylamin-hydrochlorid in 300 ml Essigsäure werden 5 Stunden bei 60°C und anschließend über Nacht bei Raumtemperatur gerührt. Man engt die Reaktionsmischung ein, nimmt den Rückstand in Methylenchlorid auf und saugt den ungelösten Festkörper ab. Das Filtrat wird mit $Na_2CO_3$-Lösung und Wasser gewaschen, über $MgSO_4$ getrocknet und eingeengt. Der Rückstand wird mit einer Methyl-t-butylether/Hexan-Mischung ausgerührt und abgesaugt. Als Rückstand erhält man 20 g (0,1 mol, 18 %) des cis-Isomeren der Titelverbindung. Das Filtrat wird eingeengt und säulenchromatographisch mit Hexan/Essigester-Gemischen gereinigt. Man erhält 32 g (0,16 mol, 28 %) der Titelverbindung als cis/trans-Gemisch (ca. 2:1).
$^1$H-NMR ($CDCl_3$; δ in ppm):
cis-Isomeres: 8,18 (s, 1 H, Isoxazolyl); 4,1 (s, 3 H, O-$CH_3$); 3,95 (s, 3 H, O-$CH_3$); 2,2 (s, 3 H, $CH_3$) trans-Isomeres: 8,19 (s, 1 H, Isoxazolyl); 4,2 (s, 3 H, O-$CH_3$); 3,9 (s, 3 H, O-$CH_3$); 2,0 (s, 3 H, $CH_3$)

Beispiel 8

α-Keto-α-(4-brommethylisoxazolyl-5)-essigsäuremethylester-O-methyloxim (Tabelle 3, Nr. 155 und 156) und α-Keto-α-(4-dibrommethylisoxazolyl-5)-essigsäuremethylester-O-methyloxim (Tabelle 3, Nr. 157 und 158)

Eine Mischung von 25 g (0,12 mol) des cis-Methoximinomethylisoxazols aus Beispiel 7 b, 25 g (0,14 mol) N-Bromsuccinimid und 0,5 g Azoisobutyrodiniril in 150 ml Methylenchlorid wird 5 h mit einer 300 W UV-Lampe bestrahlt, wobei die Reaktionsmischung zum Rückfluß erhitzt wird. Anschließend kühlt man die Reaktionsmischung auf Raumtemperatur und extrahiert sie dreimal mit Wasser. Dann wird die organische Phase über $MgSO_4$ getrocknet und eingeengt. Der Rückstand wird säulenchromatographisch mit Hexan/Essigester-Gemischen gereinigt. Man erhält 3,8 g (10,7 mmol, 9 %) des cis-Dibromids, 10 g (29 mmol, 24 %) des cis-Bromids (Reinheit ca. 80 %), 4 g (11 mmol, 9 %) des trans-Dibromids und 14 g (51 mmol, 42 %) des trans-Bromids (Fp = 45°C) der entsprechenden Titelverbindung.
$^1$H-NMR ($CDCl_3$; δ in ppm):
cis-Dibromid: 8,7 (s, 1 H, Isoxazolyl); 6,95 (s, 1 H, $CHBr_2$); 4,2 (s, 3 H, O-$CH_3$); 3,95 (s, 3 H, O-$CH_3$) cis-Bromid: 8,4 (s, 1 H, Isoxazolyl); 4,55 (s, 2 H, $CH_2$Br); 4,15 (s, 3 H, O-$CH_3$); 4,0 (s, 3 H, O-$CH_3$) trans-Dibromid: 8,65 (s, 1 H, Isoxazolyl); 6,45 (s, 1 H, $CHBr_2$); 4,25 (s, 3 H, O-$CH_3$); 3,95 (s, 3 H, O-$CH_3$) trans-Bromid: 8,4 (s, 1 H, Isoxazolyl; 4,25 (s, 2 H, $CH_2$Br); 4,23 (s, 3 H, O-$CH_3$); 3,95 (s, 3 H, O-$CH_3$)

Beispiel 9

β-Keto-β-(4-formylisoxazolyl-5)-essigsäuremethylester-trans-O-methyloxim (Tabelle 3, Nr. 159)

35,5 g (0,1 mol) des trans-Dibromids aus Beispiel 8 in 300 ml Methanol wird mit einer Lösung von 35 g (0,21 mol) $AgNO_3$ in 200 ml Wasser versetzt. Man rührt 2 Stunden bei Raumtemperatur, saugt den ausgefallenen Festkörper ab und engt die Reaktionsmischung ein. Der Rückstand wird in Methyl-t-butylether aufgenommen und mit Wasser, $NaHCO_3$-Lösung und Wasser extrahiert. Die organische Phase wird über $MgSO_4$ getrocknet und eingeengt. Der kristalline Rückstand wird mit Methyl-t-butylether/Hexan aufgerührt und abgesaugt. Man erhält 15,8 g (75 mmol, 75 %) der Titelverbindung als farblosen Festkörper.
$^1H$-NMR ($CDCl_3$; δ in ppm):
9,85 (s, 1 H, CHO); 8,7 (s, 1 H, Isoxazolyl); 4,25 (s, 3 H, O-$CH_3$); 3,95 (s, 3 H, O-$CH_3$)

Beispiel 10

β-Keto-β-(4-(2'-o-methylphenyl-trans-ethenyl-1')-isoxazolyl-5)-essigsäuremthylester-trans-O-methyloxim (Tabelle 3, Nr. 124)

20 g (50 mmol) o-Methylbenzyl-triphenylphosphoniumchlorid in 200 ml Tetrahydrofuran wird bei Raumtemperatur portionsweise mit 4,5 g (40 mmol) Kalium-t-butanolat versetzt. Man rührt 30 min., kühlt auf -30°C und gibt 6,3 g (30 mmol) des trans-O-Methyloxim-aldehyds aus Beispiel 9, gelöst in Tetrahydrofuran,hinzu. Anschließend läßt man langsam auf Raumtemperatur erwärmen und rührt weitere 4 Stunden bei dieser Temperatur. Dann verdünnt man die Reaktionsmischung mit Wasser und extrahiert die wäßrige Phase dreimal mit Methylenchlorid. Die organische Phase wird über $MgSO_4$ getrocknet und eingeengt, dabei kristallisiert Triphenylphosphinoxid aus. Der Rückstand wird mit Methyl-t-butylether ausgerührt und der unlösliche Festkörper wird abgesaugt. Das Filtrat wird eingeengt, der Rückstand kristallisiert und wird mit Methyl-t-butylether/Hexan ausgerührt und abgesaugt. Man erhält 3,1 g (10 mmol, 33 %) der Titelverbindung als farblosen Festkörper (Fp = 93°C).
$^1H$-NMR ($CDCl_3$; δ in ppm):
8,6 (s, 1 H, Isoxazolyl); 7,45 (m, 1 H, Phenyl); 7,2 (m, 3 H, Phenyl); 7,15 (d, 1 H, J = 18 Hz, =CH); 6,6 (d, 1 H, J = 18 Hz, =CH); 4,2 (s, 3 H, O-$CH_3$); 3,95 (s, 3 H, O-$CH_3$); 2,4 (s, 3 H, $CH_3$)

Beispiel 11

β-Keto-β-(5-methoxymethylpyrimidinyl-4)-essigsäuremethylester-O-methyloxim (Tabelle 3, Nr. 145)

a) β-Keto-α-methoximino-valeriansäuremethylester

30 g (185 mmol) β-Keto-δ-methoxy-valeriansäuremethylester (Synthesis 1979, 622; Zh. Org. Khim. 23 (1987), 288) und 14 g (200 mmol) $NaNO_2$ in 60 ml Wasser werden unter leichter Kühlung mit 14g (233 mmol) Essigsäure versetzt. Man rührt 1 Stunde bei Raumtemperatur, verdünnt die Reaktionsmischung mit Wasser und extrahiert die wäßrige Phase dreimal mit Methylenchlorid. Die vereinigten organischen Phasen werden über $MgSO_4$ getrocknet und eingeengt. Der Rückstand wird in 300 ml Aceton gelöst und mit 30 g (220 mmol) $K_2CO_3$ und 40 g (280 mmol) Methyljodid versetzt. Man rührt 2 Stunden bei Raumtemperatur, saugt den unlöslichen Festkörper ab und engt die Reaktionsmischung ein.

Der Rückstand wird in Methyl-t-butylether aufgenommen, die organische Phase wird mit $NH_4Cl$-Lösung gewaschen, über $MgSO_4$ getrocknet und eingeengt. Als Rückstand erhält man 35 g (172 mmol, 93 %) der Titelverbindung als gelbe Flüssigkeit.
$^1H$-NMR ($CDCl_3$; δ in ppm):
4,1 (s, 3 H, O-$CH_3$); 3,9 (s, 3 H, O-$CH_3$); 3,7 (t, 2 H, J = 7 Hz, $CH_2$); 3,35 (s,3 H, O-$CH_3$); 3,1 (t, 2 H, J = 7 Hz, $CH_2$)

b) δ-Dimethylamino-β-keto-α-methoximino-γ-methoxymethylpenten-4-säuremethylester

Eine Mischung von 10 g (50 mmol) des Ketons aus Beispiel 11a, 7 g (85 mmol) Natriumacetat und 7 g (69 mmol) Acetanhydrid in 20 ml Dimethylformamid-dimethylacetal wird 2 Stunden bei 70°C gerührt. Man verdünnt die Reaktionsmischung mit Methylenchlorid und wäscht die organische Phase einmal mit Wasser. Dann wird die organische Phase über $MgSO_4$ getrocknet und eingeengt. Der Rückstand wird säulenchromatographisch mit Hexan/Essigester-Gemischen und schließlich mit Essigester gereinigt. Man erhält 11,4 g (44 mmol, 88 %) der Titelverbindung als gelbes Öl.

$^1H$-NMR ($CDCl_3$; δ in ppm):
7,7 (s, 1 H, Vinyl); 4,3 (s, 2 H, $CH_2$); 4,0 (s, 3 H, O-$CH_3$); 3,85 (s, 3 H, O-$CH_3$); 3,3 (s, 3 H, O-$CH_3$); 3,25 (s, 6 H, $N(CH_3)_2$)

c) α-Keto-α-(5-methoxymethylpyrimidinyl-4)-essigsäuremethylester-O-methyloxim (Tabelle 3, Nr. 145)

2,6 (10 mmol) des Enaminoketons aus Beispiel 11 b, 2 g (14,4 mmol) $K_2CO_3$ und 1,4 g (13,5 mmol) Formamidinium-acetat in 20 ml Methanol werden 3 Stunden bei Raumtemperatur gerührt. Dann verdünnt man die Reaktionsmischung mit Wasser und extrahiert die wäßrige Phase dreimal mit Methyl-t-butylether. Die vereinigten organischen Phasen werden über $MgSO_4$ getrocknet und eingeengt. Der Rückstand wird säulenchromatographisch mit Hexan/Essigester-Gemischen gereinigt. Man erhält 0,65 g (2,7 mmol, 27 %) der Titelverbindung als farblosen Festkörper (Fp = 82°C).
$^1H$-NMR($CDCl_3$, δ in ppm):
9,15 (s, 1 H, Pyrimidinyl); 9,1 (s, 1 H, Pyrimidinyl); 4,75 (s, 2 H, O-$CH_2$); 4,15 (s, 3 H, O-$CH_3$); 3,95 (s, 3 H, O-$CH_3$); 3,55 (s, 3 H, O-$CH_3$)

Beispiel 12

α-Keto-α-(N-phenyl-5-methylpyrazolyl-4)-essigsäuremethylester-O-methyloxim (Tabelle 3, Nr. 160)

a) γ-Keto-α-methoximimo-valeriansäuremethylester

117,3 g (ca. 90 %ige Reinheit; 0,73 mol; hergestellt analog Organikum, 15. Auflage 1976, S. 584) α, γ-Diketovaleriansäuremethylester und 61,8 g (0,74 mol) O-Methylhydroxylamin-hydrochlorid in 1 l Methanol werden 24 Stunden bei Raumtemperatur gerührt. Dann wird die Reaktionsmischung eingeengt, der Rückstand wird in Waser aufgenommen und mit $Na_2CO_3$-Lösung neutralisiert. Die wäßrige Phase wird anschließend dreimal mit Methylenchlorid extrahiert. Die vereinigten organischen Phasen werden über $MgSO_4$ getrocknet und eingeengt. Der Rückstand wird säulenchromatophisch mit Hexan/Essigester-Gemischen gereinigt. Als Rückstand erhält man 69,5 g (0,40 mol, 55 %) der Titelverbindung als hellgelbe Flüssigkeit.
$^1H$-NMR (CDCl3; d in ppm):
4,1 (s, 3 H, O-$CH_3$); 3,9 (s, 3 H, O-$CH_3$); 3,75 (s, 2 H, $CH_2$); 2,25 (s, 3 H, $CH_3$)

b) β-Acetyl-γ-dimethylamino-β-methoximino-buten-3-säuremethylester

Eine Mischung von 40 g (0,23 mol) des Ketons aus Beispiel 12a und 33 g (0,28 mol) Dimethylformamiddimethylacetal wird 5 Stunden bei 70°C gerührt. Dann werden die flüchtigen Komponenten zuerst bei 25 mbar und dann im Hochvakuum abgezogen. Der Rückstand wird säulenchromatographisch mit Hexan/ Essigester-Gemischen bzw. Essigester gereinigt. Man erhält 40 g (0,175 mol, 76 %) der Titelverbindung als gelben Festkörper (Fp = 122°C).
$^1H$-NMR ($CDCl_3$; δ in ppm):
7,55 (s, 1 H, Vinyl); 4,05 (s, 3 H, O-$CH_3$); 3,9 (s, 3 H, O-$CH_3$); 2,95 (s, breit, 6 H, $N(CH_3)_2$); 2,1 (s, 3 H, $CH_3$)

c) α-Keto-α-(N-phenyl-5-methylpyrazolyl-4)-essigsäuremethylester-O-methyloxim (Tabelle 3, Nr. 160)

10 g (44 mmol) des Enaminoketons aus Beispiel 12 b in 20 ml Essigsäure wird bei 0°C portionsweise mit 7,1 g (66 mmol) Phenylhydrazin versetzt. Man rührt über Nacht bei Raumtemperatur und verdünnt dann die Reaktionsmischung mit Wasser. Die wäßrige Phase wird dreimal mit Methyl-t-butylether, extrahiert die vereinigten organischen Phasen werden mit $Na_2CO_3$-Lösung und Wasser gewaschen, über $MgSO_4$ getrocknet und eingeengt. Der Rückstand wird säulenchromatographisch mit Hexan/Essigester-Gemischen gereinigt. Als Rückstand erhält man 10,6 g (39 mmol, 88 %) der Titelverbindung als gelbes Öl.
$^1H$-NMR ($CDCl_3$; δ in ppm):
7,9 (s, 1 H, Pyrazolyl); 7,3 - 7,6 (m, 5 H, Phenyl); 4,15 (s, 3 H, O-$CH_3$); 3,9 (s, 3 H, O-$CH_3$); 2,2 (s, 3 H, $CH_3$)

Beispiel 13

α-Keto-γ-(N-phenyl-5-brommethylpyrazolyl-4)-essigsäuremethylester-O-methyloxim (Tabelle 3, Nr. 161 und 162)

Eine Mischung von 9,4 g (34,4 mmol) des Methylpyrazols aus Beispiel 12 c, 7,5 g (42 mmol) N-Bromsucinimid und 0,1 g Azoisobutyrodinitril in 100 ml Tetrachlorkohlenstoff wird 3 Stunden mit einer 300 W UV-Lampe bestrahlt, wobei sich die Reaktionsmischung auf 40 -50°C erwärmt. Dann wird der unlösliche Festkörper abgesaugt und das Filtrat wird mit Wasser gewaschen, über $MgSO_4$ getrocknet und eingeengt. Der Rückstand wird säulenchromatographisch mit Hexan/Essigester-Gemischen gereinigt. Man erhält 3 g (8,5 g mmol, 25 %) des cis-Oximetherisomeren und 4,8 g (13,6 mmol, 40 %) des trans-Oximetherisomeren der Titelverbindung.
$^1$H-NMR (CDCl3, d in ppm);
cis-Isomeres: 7,7 (s, 1 H, Pyrazolyl); 7,4 - 7,65 (m, 5 H, Phenyl); 4,65 (s, 2 H, $CH_2Br$); 4,05 (s, 3 H, O-$CH_3$); 3,95 (s, 3 H, O-$CH_3$) trans-Isomeres: 7,9 (s, 1 H, Pyrazol); 7,4 - 7,6 (m, 5 H, Phenyl); 4,5 (s, 2 , $CH_2Br$); 4,2 (s, 3 H, O-$CH_3$); 3,95 (s, 3 H, O-$CH_3$)

Beispiel 14

α-Keto-α-(N-phenyl-5-(p-bromacetophenoniminoximethyl)-pyrazolyl-4)-essigsäuremethylester-trans-O-methyloxim (Tabelle 3, Nr. 143)

Eine Mischung von 1,6 g (4,5 mmol) des trans-Methyloximether-methylbromids aus Beispiel 13, 1,6 g (4,5mmol) p-Bromacetophenonoxim und 0,6 g (4,5 mmol) $K_2CO_3$ in 5 ml Dimethylformamid wird über Nacht bei Raumtemperautr gerührt und anschließend ca. 5 Stunden auf 50°C erhitzt. Dann wird die Reaktionsmischung mit Wasser verdünnt und dreimal mit Methyl-t-butylether extrahiert. Die vereinigten organischen Phasen werden mit Wasser extrahiert, über $MgSO_4$ getrocknet und eingeengt. Der Rückstand wird säulenchromatographisch mit Hexan/Essigester-Gemischen gereinigt. Man erhält 1,1 g (2,3 mmol, 50 %) der Titelverbindung als gelbes Öl.
$^1$H-NMR ($CDCl_3$; δ in ppm):
8,0 (s, 1 H, Pyrazolyl); 7,3 - 7,6 (m, 9 H, Phenyl); 5,25 (s, 1 H, $CH_2$-O); 4,1 (s, 3 H, O-$CH_3$); 3,85 (s, 3 H, O-$CH_3$); 2,1 (s, 3 H, $CH_3$)

Beispiel 15

α-Keto-α-(N-benzylimidazolyl-2)-essigsäuremethylester-O-methyloxim (Tabelle 3, Nr. 30)

a) α-Keto-α-(N-benzylimidazolyl-2)-essigsäuremethylester

Eine Mischung von 10 g (60 mmol) N-Benzylimidazol (Chem. Pharm. Bull., 31 (4), 1213 (1983)) und 9 g (90 mmol) Triethylamin in 100 ml Methylenchlorid wird bei 20 - 30°C tropfenweise mit 9,3 g (76 mmol) Oxalsäuremethylesterchlorid versetzt. Man rührt 3 Stunden bei Raumtemperatur und extrahiert anschließend die Reaktionsmischung mit $Na_2CO_3$-Lösung und Wasser. Die organische Phase wird über $MgSO_4$ getrocknet und eingeengt. Der Rückstand wird säulenchromatographisch mit Methylenchorid/Methanol 100:1 gereinigt. Ausbeute: 10,2 g (44 mmol, 74 %)
$^1$H-NMR ($CDCl^3$, δ in ppm):
7,1 - 7,5 (m, 7 H, Phenyl, Imidazolyl); 5,6 (s, 2 H, N-$CH_2$); 3,95 (s, 3 H; O-$CH_3$)

b) α-Keto-α-(N-benzylimidazolyl-2)-essigsäuremethylester-O-methyloxim (Tabelle 3, Nr. 30)

Eine Mischung von 4,7 g (30 mmol) des Ketoesters aus Beispiel 15a und 5 g (60 mmol) Methoxyaminhydrochlorid in 60 ml Methanol wird über Nacht bei Raumtemperatur gerührt. Anschließend verdünnt man die Reaktionsmischung mit Wasser und extrahiert die wäßrige Phase dreimal mit Methylenchlorid. Die vereingten organischen Phasen werden mit Wasser extrahiert, über $MgSO_4$ getrocknet und eingeengt. Der Rückstand wird mit Methylenchlorid und Methylenchlorid/Methanol 100:1 säulenchromatographisch gereinigt. Man erhält 0,8 (4,3 mmol, 14 %) des unpolaren und 0,4 g (2,1 mmol, 7 %) des polaren Isomeren (Fp = 55 - 58°C) der Titelverbindung.
$^1$H-NMR ($CHCl_3$, δ in ppm):
unpolares Isomeres; 7,1 - 7,5 (m, 6 H, Imidazolyl, Phenyl); 6,95 (s, breit, 1 H, Imidazolyl); 5,5 (s, 2 H, N-$CH_2$); 3,95 (s, 3 H, O-$CH_3$); 3,93 (s, 3 H, O-$CH_3$)
polares Isomeres: 7,05 - 7,4 (m, 6 H, Imidazolyl, Phenyl); 6,95 (s, 1 H, Imidazolyl); 4,95 (s, 2 H, N-$CH_2$); 4,05 (s, 3 H, O-$CH_3$); 3,8 (s, 3 H, O-$CH_3$)

Beispiel 16

3-(2-Chlorphenoxymethyl)-thienyl-2-glyoxylsäuremethylester-trans-O-methyloxim (Tabelle 3, Nr. 15)

a) 3-Methylthienyl-2-glyoxylsäuremethylester

200 g (2 mol) 3-Methylthiophen und 368 g (3 mol) Oxalsäurechlorid-monomethylester werden bei 5°C vorgelegt. Zu dieser Mischung tropft man 400 g (3 mol) $AlCl_3$ in 600 ml Nitromethan zu, rührt 1 h bei 10°C und 1 h bei Raumtemperatur. Es wird auf 2 l Eiswasser gegossen, mit Ether extrahiert. Die organische Phase wird mit gesättigter Natriumcarbonat-Lösung gewaschen, getrocknet und eingeengt. Der Rückstand wird im Vakuum destilliert. Man erhält 213,7 g (58 %) der Verbinding als gelbe Kristalle (b.p.: 105 -110°C/0,5 mbar).

b) 3-Methylthienyl-2-glyoxylsäuremethylester-O-methyloxim (Tabelle 3, Nr. 163)

Eine Lösung von 56,4 g (0,306 mol) 3-Methyl-thienyl-2-glyoxylsäuremethylester und 25,5 g (0,306 mol) O-Methylhydroxylamin-hydrochlorid in 500 ml Methanol wird 15 Stunden unter Rückfluß erhitzt. Man engt ein, nimmt in Essigester auf, wäscht mit Wasser, trocknet und entfernt das Lösungsmittel im Vakuum. Man erhält 51,7 g (79 %) der oben genannten Verbindung als Isomerengemisch (60 : 40).
$^1$H-NMR ($CDCl_3$; δ in ppm):
Isomer A (unpolar): 7,4 (d, 1 H, J = 4 Hz Thienyl); 6,9 (d, 1 H, J = 4 Hz, Thienyl); 4,1 (s, 3 H, $O-CH_3$); 3,9 (s, 3 H, $O-CH_3$); 2,15 (s, 1 H, $CH_3$)
Isomer B (polar); 7,25 (d, 1 H, J = 4 Hz, Thienyl); 6,8 (d, 1 H, J = 4 Hz, Thienyl); 4,0 (s, 3 H, $O-CH_3$); 3,9 (s, 3 H, $O-CH_3$); 2,3 (s, 1 H, $CH_3$)

c) 3-Brommethylthienyl-2-glyoxylsäuremethylester-O-methyloxim (Tabelle 3, Nr. 164)

Eine Lösung von 46 g (0,216 mol) 3-Methyl-thienyl-2-glyoxylsäuremethylester-O-methyloxim und 42,1 g (0,237 mol) N-Bromsuccinimid in 400 ml Tetrachlorkohlenstoff wird 3 Stunden mit einer UV-Lampe bestrahlt. Anschließend wird filtriert und eingeengt. Man erhält 63,7 g eines schwarzen Öls das die obengenannte Verbindung zu 60 % enthält.
$^1$H-NMR ($CDCl_3$; δ in ppm):
7,1 - 7,6 (Thienyl-Protonen);
Isomer A: 4,3 (s, 2 H, $CH_2$-Br); 4,15 (s, 3 H, $O-CH_3$); 3,9 (s, 3 H, $O-CH_3$)
Isomer B: 4,65 (s, 2 H, $CH_2$-Br); 4,05 (s, 3 H, $O-CH_3$); 3,95 (s, 3 H, $O-CH_3$)

d) 3-(2'-Chlorphenoxymethyl)-thienyl-2-glyoxylsäuremthylester-trans-O-methyloxim (Tabelle 3, Nr. 15)

Eine Lösung von 11,7 g der unter Beispiel 16c beschriebenen Verbindung in 100 ml Aceton wird mit 5,14 g (0,04 mol) 3-Chlorphenol, 6 g Kaliumcarbonat und 500 mg Kaliumjodid versetzt. Man erhitzt 24 Stunden unter Rückfluß, filtriert und engt ein. Der Rückstand wird in Methylenchlorid aufgenommen, mit Wasser gewaschen, getrocknet und eingeengt. Der Rückstand wird an Kieselgel mit Essigester/Hexan (1:5) chromatographiert. Man erhält 5,5 g der oben genannten Verbindung in Form ihres trans-Isomers.
$^1$H-NMR ($CDCl_3$; δ in ppm):
6,7 - 7,6 (m, 6 H, aromat. Protonen); 4,95 (s, 2 H, $O-CH_2$); 4,1 (s, 3 H, $O-CH_3$); 3,8 (s, 3 H, $O-CH_3$)

Beispiel 17

3-(2'-Chlorthiophenoxymethyl)-thienyl-2-glyoxylsäuremethylester-O-methyloxim (Tabelle 3, Nr. 22 und 23)

a) 3-Brommethylthienyl-2-glyoxylsäuremethylester

Eine Lösung von 135,7 g (0,73 mol) 3-Methyl-thienyl-2-glyoxylsäuremethylester, 131,2 g (0,73 mol) N-Bromsuccinimid und 1,35 g Azoisobutyronitril in 1000 ml Tetrachlorkohlenstoff wird 3 h unter Rückfluß erhitzt. Anschließend wird abfiltriert und eingeengt. Der Rückstand wird in Diethylether aufgenommen, mit Wasser gewaschen, getrocknet und eingeengt. Man erhält 166,4 g Rohprodukt, das aus Methanol umkristallisiert wird. Man erhält 100 g der oben genannten Verbindung als gelben Feststoff.
$^1$H-NMR ($CDCl_3$; δ in ppm):
7,75 (d, 1 H, J = 4 Hz, Thienyl); 7,3 (d, 1 H, J = 4 Hz, Thienyl); 4,95 (s, 2 H, $CH_2$-Br); 4,0 (s, 3 H, $O-CH_3$)

b) 3-(2-Chlorthiophenoxymethyl)-thienyl-2-glyoxylsäuremethylester

Zu einer Lösung von 5 g (19 mmol) 3-Brommethyl-thienyl-2-glyoxylsäure-methylester in 50 ml Aceton gibt man 2,62 g (19 mmol) Kaliumcarbonat und 2,74 g (19 mmol) 2-Chlorthiophenol und rührt 72-Stunden bei Raumtemperatur. Es wird abfiltriert und eingeengt. Der Rückstand wird in Methylenchlorid aufgenommen, mit $Na_2CO_3$-Lösung gewaschen, getrocknet und eingeengt. Das Rohprodukt wird aus Methanol umkristallisiert. Man erhält 4,24g (68 %) der oben genannten Verbindung.
$^1$H-NMR ($CDCl_3$; δ in ppm):
7,1 - 7,7 (m, 6 H, Aromat); 4,6 (s, 2 H, S-$CH_2$); 3,95 (s, 3 H, $OCH_3$)

c) 3-(2-Chlorthiophenoxymethyl)-thienyl-2-glyoxylsäuremethylester-O-methyloxim (Tabelle 3, Nr. 22 und 23)

4,24 g der unter Beispiel 17b beschriebenen Verbindung wird mit 1,07 g O-Methylhydroxylaminhydrochlorid in 100 ml Methanol 3 Stunden auf 50°C erhitzt. Man engt ein, nimmt den Rückstand in Essigester auf, wäscht mit Wasser, trocknet und engt ein. Das Rohprodukt wird an Kieselgel mit Hexan/Essigester (10 : 1) chromatographiert. Man erhält nacheinander 810 mg (cis-Isomer) und 1,87 g (trans-isomer) der oben genannten Verbindung.
$^1$H-NMR ($CDCl_3$; δ in ppm):
cis-Isomeres: 7 - 7,4 (m, 6 H, Aromat); 4,3 (s, 2 H, S-$CH_2$); 4,0 (s, 3 H, O-$CH_3$); 3,9 (s, 3 H, O-$CH_3$)
trans-Isomeres: 7,1 - 7,5 (m, 6 H, Aromat); 4,12 (s, 3 H, O-$CH_3$); 4,0 (s, 2 H, S-$CH_2$); 3,91 (s, 3 H, O-$CH_3$)

Beispiel 18

Pyrrolyl-2-glyoxylsäuremethylester-O-methyloxim (Tabelle 3, Nr. 165)

Zu einer Lösung von 148,8 g (0,97 mol) Pyrrolyl-2-glyoxylsäuremethylester (hergestellt analog: B. Lindström et al, Acta Chem, Scand. 27 (1973), 7) in 1 l Methanol gibt man 97,5g (1,2 Eq) Methoxyaminhydrochlorid und erhitzt die Lösung 2,5 Stunden unter Rückfluß. Nach dem Einrotieren wird der Rückstand in Essigester aufgenommen und mit Wasser gewaschen. Man trocknet und engt ein. Es verbleiben 81 g (46 %) des Pyrrols als Öl.
$^1$H-NMR ($CDCl_3$,δ in ppm):
10,35 (s, breit, 1 H, N-H); 7,15 (m, 1 H, Pyrrolyl); 6,95 (m, 1 H, Pyrrolyl); 6,3 (m, 1 H, Pyrrolyl); 4,15 (s, 3 H, O-$CH_3$); 3,9 (s, 3 H, O-$CH_3$)

Beispiel 19

N-(o-Methylbenzyl)-pyrrolyl-2-glyoxylsäuremethylester-O-methyloxim (Tabelle 3, Nr. 31)

Zu einer Lösung von 730 mg (2,7 mmol) 18-Krone-6 in 50 ml Ether gibt man 4 g (35,7 mmol) Kalium-tert.-butylat und tropft anschließend eine Lösung von 5 g (27,5 mmol) des Pyrrols aus Beispiel 18 in 50 ml Ether zu und rührt 30 min. bei Raumtemperatur zu. Man gibt dann eine Lösung von 6,6 g (35,7 mol) o-Methylbenzylbromid in 25 ml Ether zu und rührt über Nacht bei Raumtemperatur. Man gießt auf Wasser, extrahiert mit Ether, trocknet und engt ein. Chromatographie an Kieselgel mit Hexan/Essigester liefert 4,1 g (52 %) des Produktes als gelbes Öl.
IR (ν in $cm^{-1}$):
1733, 1461, 1438, 1319, 1286, 1221, 1072, 1035, 776, 742

Beispiel 20

N-Benzyl-pyrrolyl-2-glyoxylsäuremethylester-o-methyloxim (Tabelle 3, Nr. 30)

a) N-Benzyl-pyrrolyl-2-glyoxylsäuremethylester

Zu einer Lösung von 1,7 g (6,5 mmol) 18-Krone-6 mit 7,3 g (65 mmol) Kalium-tert.-butylat in 100 ml Ether gibt man eine Suspension von 10 g (65 mmol) Pyrrolyl-2-glyoxylsäuremethylester in 100 ml Ether, rührt 30 min. nach und tropft dann 11,1 g (65 mmol) Benzylbromid in 50 ml Ether zu. Man rührt über Nacht bei Raumtemperatur, gießt auf Wasser und extrahiert mit Ether, trocknet und engt ein. Chromatographie an Kieselgel mit Hexan/Essigester liefert 6,7 g (42 %) der Titelverbindung als rotbraunes Öl.
$^1$H-NMR ($CDCl_3$; δ in ppm):

7 - 7,4 (m, 7 H, Phenyl, Pyrrolyl), 6,25 (m, 1 H, Pyrrolyl); 5,6 (s, 2 H, N-$CH_2$); 3,9 (s, 3 H, O-$CH_3$)

b) N-Benzyl-pyrrolyl-2-glyoxylsäuremethylester-O-methyloxim (Tabelle 3, Nr. 30)

Zu einer Lösung von 3 g (12,3 mmol) des Ketoesters aus Beispiel 20a in 100 ml MeOH gibt man 6,6 g (80 mmol) Methoxyaminhydrochlorid und erhitzt 12 Stunden unter Rückfluß. Der Rückstand wird einrotiert, in Essigester aufgenommen, mit Wasser gewaschen, getrocknet und eingeengt. Chromatographie an Kieselgel mit Hexan/Essigester liefert 2 g (60 %) Isomer A und 0,6 g (18 %) Isomer B.

$^1$H-NMR ($CDCl_3$; δ in ppm):

Isomer A (unpolar): 7 - 7,3 (m, 5 H, Phenyl); 6,75 (m, 1 H, Pyrrolyl); 6,4 (m, 1 H, Pyrrolyl); 6,25 (m, 1 H, Pyrrolyl); 4,95 (s,2 H, N-$CH_2$); 4,0 (s, 3 H, O-$CH_3$); 3,75 (s, 3 H, O-$CH_3$)

Beispiel 21

α-Keto-α-(5-Phenylisoxazolyl-3)-essigsäuremethylester-O-methyloxim (Tabelle 3, Nr. 47)

a) β-Hydroximino-α-methoximino-propionsäuremethylester

Zu einer Lösung von 10 g (48 mmol) β-Brombrenztraubensäuremethylester-O-Methyloxim in 40 ml Dimethylsulfoxid gibt man bei 25 - 35°C 25,9 g (220 mol) N-Methylmorpholin-N-oxid. Nach 30-minütigem Rühren bei Raumtemperatur gibt man 6,95 g (100 mmol) Hydroxylamin-hydrochlorid zu und rührt weitere 5 Stunden bei Raumtemperatur. Anschließend gibt man auf ges. NaCl-Lösung und extrahiert viermal mit Ether. Man trocknet und engt ein. Es verbleiben 4 g (25 mmol, 52 %) der Titelverbindung).

$^1$H-NMR ($CDCl_3$; δ in ppm):

10,15 (s, 1 H, N-OH); 8,25 (s, 1 H, N=CH); 4,15 (s, 3 H, O-$CH_3$); 3,9 (s, 3 H, O-$CH_3$)

b) α-Keto-α-(5-Phenylisoxazolyl-3)-essigsäuremethylester-o-methyl-oxim (Tabelle 3, Nr. 47)

Zu einer Lösung von 2 g (12,5 mmol) Aldoxim aus Beispiel 21a und 2,55 g (25 mmol) Phenylacetylen 2 in 50 ml Methylenchlorid tropft man unter Kühlung 50 ml Hypochloridlösung. Nach 30 min. bei Raumtemperatur trennt man die organische Phase ab und extrahiert die Wasserphase mit Methylenchlorid. Die org. Phase wird getrocknet und eingeengt. Chromatographie an Kieselgel mit Hexan/Essigester liefert 1,1 g (35 %) Isoxazol 2 als gelbes Öl.

$^1$H-NMR ($CDCl_3$; δ in ppm):

7,4 - 7,9 (m, 5 H, Phenyl); 6,9 (s, 1 H, Isoxazolyl); 4,2 (s, 3 H, O-$CH_3$); 3,95 (s, 3 H, O-$CH_3$)

In entsprechener Weise können die in den folgenden Tabellen aufgeführte Verbindungen hergestellt werden. In den folgenden Tabellen sind die einzelnen Indizes I, II, III usw. bzw. a, b usw. wie folgt zu verstehen.

Der heteroaromatische Ring wird durch römische Zahlen wie I, II usw. gekennzeichnet. Kleine Buchstaben wie a, b usw. kennzeichnen die Seitenkette. $X_m$ bezeichnet die Substituenten an einem Aromaten. A und B charakterisieren die Zwischenkette bzw. die Endgruppe der Seitenkette.

Beispielsweise ergeben sich die folgenden Strukturformeln für ausgewählte Verbindungen aus den Tabellen.

In Tabelle 1 hat die Verbindung Nr. 1/I/a die folgende Formel

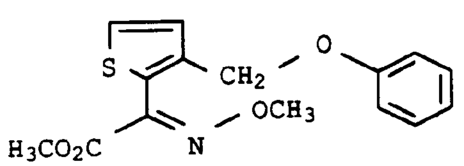

Tabelle 1, Nr. 1/I/a

In Tabelle 1 hat die Verbindung Nr. 9/II/b folgende Formel:

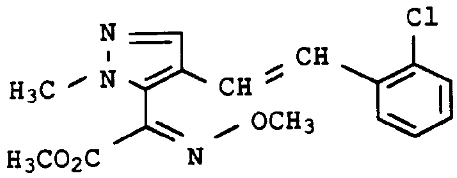

Tabelle 1, Nr. 9/II/b

In Tabelle 1 hat die Verbindung Nr. 68/III/c folgende Formel

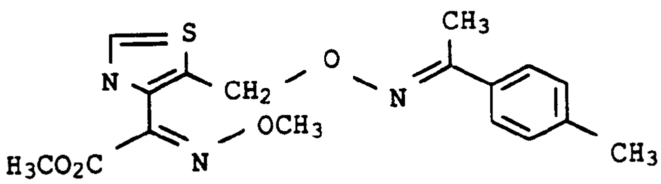

Tabelle 1, Nr. 68/III/c

In Tabelle 2 hat die Verbindung Nr. 127/I/a folgende Formel

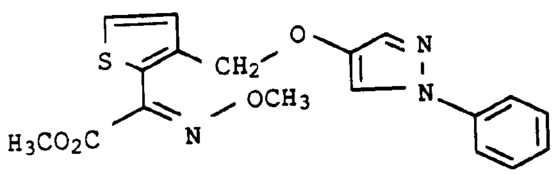

Tabelle 2 Nr. 127/I/a

In Tabelle 2 hat die Verbindung Nr. 175/II/b folgende Formel

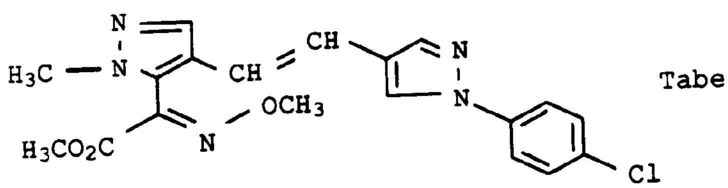

Tabelle 2, Nr. 175/II/b

In Tabelle 2 hat die Verbindung Nr. 37/III/c folgende Formel

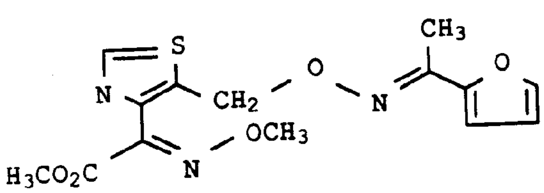

Tabelle 2, Nr. 37/III/c

Tabelle 1

I:

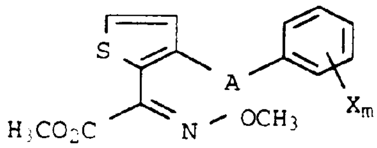

II:

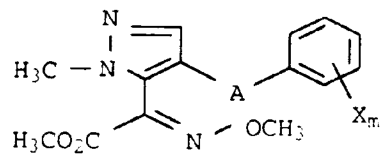

III:

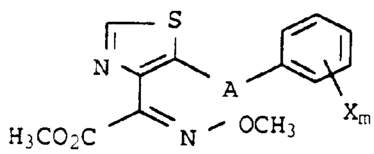

a) A = $-CH_2-O-$

b) A = 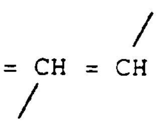

c) A = 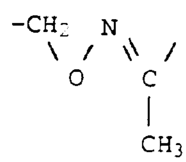

| Nummer | $X_m$ |
|---|---|
| 1 | H |
| 2 | 2-F |
| 3 | 3-F |
| 4 | 4-F |
| 5 | 2,4-$F_2$ |
| 6 | 2,4,6-$F_3$ |
| 7 | 2,3,4,5,6-$F_5$ |
| 8 | 2,3-$F_2$ |
| 9 | 2-Cl |
| 10 | 3-Cl |
| 11 | 4-Cl |

| Nummer | $X_m$ |
|---|---|
| 12 | 2,3-$Cl_2$ |
| 13 | 2,4-$Cl_2$ |
| 14 | 2,5-$Cl_2$ |
| 15 | 2,6-$Cl_2$ |
| 16 | 3,4-$Cl_2$ |
| 17 | 3,5-$Cl_2$ |
| 18 | 2,3,4-$Cl_3$ |
| 19 | 2,3,5-$Cl_3$ |
| 20 | 2,3,6-$Cl_3$ |
| 21 | 2,4,5-$Cl_3$ |
| 22 | 2,4,6-$Cl_3$ |
| 23 | 3,4,5-$Cl_3$ |
| 24 | 2,3,4,6-$Cl_4$ |
| 25 | 2,3,5,6-$Cl_4$ |
| 26 | 2,3,4,5,6-$Cl_5$ |
| 27 | 2-Br |
| 28 | 3-Br |
| 29 | 4-Br |
| 30 | 2,4-$Br_2$ |
| 31 | 2,5-$Br_2$ |
| 32 | 2,6-$Br_2$ |
| 33 | 2,4,6-$Br_3$ |
| 34 | 2,3,4,5,6-$Br_5$ |
| 35 | 2-J |
| 36 | 3-J |
| 37 | 4-J |
| 38 | 2,4-$J_2$ |
| 39 | 2-Cl, 3-F |
| 40 | 2-Cl, 4-F |
| 41 | 2-Cl, 5-F |
| 42 | 2-Cl, 6-F |
| 43 | 2-Cl, 3-Br |
| 44 | 2-Cl, 4-Br |
| 45 | 2-Cl, 5-Br |
| 46 | 2-Cl, 6-Br |
| 47 | 2-Br, 3-Cl |

| Nummer | $X_m$ |
|---|---|
| 48 | 2-Br, 4-Cl |
| 49 | 2-Br, 5-Cl |
| 50 | 2-Br, 3-F |
| 51 | 2-Br, 4-F |
| 52 | 2-Br, 5-F |
| 53 | 2-Br, 6-F |
| 54 | 2-F, 3-Cl |
| 55 | 2-F, 4-Cl |
| 56 | 2-F, 5-Cl |
| 57 | 3-Cl, 4-F |
| 58 | 3-Cl, 5-F |
| 59 | 3-Cl, 4-Br |
| 60 | 3-Cl, 5-Br |
| 61 | 3-F, 4-Cl |
| 62 | 3-F, 4-Br |
| 63 | 3-Br, 4-Cl |
| 64 | 3-Br, 4-F |
| 65 | 2,6-$Cl_2$, 4-Br |
| 66 | 2-$CH_3$ |
| 67 | 3-$CH_3$ |
| 68 | 4-$CH_3$ |
| 69 | 2,3-$(CH_3)_2$ |
| 70 | 2,4-$(CH_3)_2$ |
| 71 | 2,5-$(CH_3)_2$ |
| 72 | 2,6-$(CH_3)_2$ |
| 73 | 3,4-$(CH_3)_2$ |
| 74 | 3,5-$(CH_3)_2$ |
| 75 | 2,3,5-$(CH_3)_3$ |
| 76 | 2,3,4-$(CH_3)_3$ |
| 77 | 2,3,6-$(CH_3)_3$ |
| 78 | 2,4,5-$(CH_3)_3$ |
| 79 | 2,4,6-$(CH_3)_3$ |
| 80 | 3,4,5-$(CH_3)_3$ |
| 81 | 2,3,4,6-$(CH_3)_4$ |
| 82 | 2,3,5,6-$(CH_3)_4$ |
| 83 | 2,3,4,5,6-$(CH_3)_5$ |

| Nummer | $X_m$ |
|---|---|
| 84 | $2-C_2H_5$ |
| 85 | $3-C_2H_5$ |
| 86 | $4-C_2H_5$ |
| 87 | $2,4-(C_2H_5)_2$ |
| 88 | $2,6-(C_2H_5)_2$ |
| 89 | $3,5-(C_2H_5)_2$ |
| 90 | $2,4,6-(C_2H_5)_3$ |
| 91 | $2-n-C_3H_7$ |
| 92 | $3-n-C_3H_7$ |
| 93 | $4-n-C_3H_7$ |
| 94 | $2-i-C_3H_7$ |
| 95 | $3-i-C_3H_7$ |
| 96 | $4-i-C_3H_7$ |
| 97 | $2,4-(i-C_3H_7)_2$ |
| 98 | $2,6-(i-C_3H_7)_2$ |
| 99 | $3,5-(i-C_3H_7)_2$ |
| 100 | $2,4,6-(i-C_3H_7)_3$ |
| 101 | $2-s-C_4H_9$ |
| 102 | $3-s-C_4H_9$ |
| 103 | $4-s-C_4H_9$ |
| 104 | $2-t-C_4H_9$ |
| 105 | $3-t-C_4H_9$ |
| 106 | $4-t-C_4H_9$ |
| 107 | $2,3-(t-C_4H_9)_2$ |
| 108 | $2,4-(t-C_4H_9)_2$ |
| 109 | $2,5-(t-C_4H_9)_2$ |
| 110 | $2,6-(t-C_4H_9)_2$ |
| 111 | $3,4-(t-C_4H_9)_2$ |
| 112 | $2,4,6-(t-C_4H_9)_3$ |
| 113 | $4-n-C_9H_{19}$ |
| 114 | $4-n-C_{12}H_{25}$ |
| 115 | $4-n-C_{15}H_{31}$ |
| 116 | 4-(1,1,3,3-Tetramethylbutyl) |
| 117 | 4-(2,4,4-Trimethylpropyl) |
| 118 | $2-t-C_4H_9$, $4-CH_3$ |
| 119 | $2-t-C_4H_9$, $5-CH_3$ |

| Nummer | $X_m$ |
|---|---|
| 120 | 2,6-(t-$C_4H_9$)2, 4-$CH_3$ |
| 121 | 2-$CH_3$, 4-t-$C_4H_9$ |
| 122 | 2-$CH_3$, 4-t-$C_4H_9$ |
| 123 | 2-$CH_3$, 4-i-$C_3H_7$ |
| 124 | 2-$CH_3$, 5-i-$C_3H_7$ |
| 125 | 3-CH3, 4-i-C3H7 |
| 126 | 2-i-$C_3H_7$, 5-$CH_3$ |
| 127 | 2,4-(t-$C_4H_9$)2, 6-i-$C_3H_7$ |
| 128 | 2-Allyl |
| 129 | 3-Allyl |
| 130 | 4-Allyl |
| 131 | 1-Allyl, 6-$CH_3$ |
| 132 | 2-cyclo-$C_6H_{11}$ |
| 133 | 3-cyclo-$C_6H_{11}$ |
| 134 | 4-cyclo-$C_6H_{11}$ |
| 135 | 2,4-(cyclo-$C_6H_{11}$)2, 6-$CH_3$ |
| 136 | 2-$CH_3$, 4-cyclo-$C_6H_{11}$ |
| 137 | 2-$CH_2$-$C_6H_5$ |
| 138 | 3-$CH_2$-$C_6H_5$ |
| 139 | 4-$CH_2$-$C_6H_5$ |
| 140 | 2-$CH_2$-$C_6H_5$, 4-$CH_3$ |
| 141 | 2-$CH_3$, 4-$CH_2$-$C_6H_5$ |
| 142 | 2-$C_6H_5$ |
| 143 | 3-$C_6H_5$ |
| 144 | 4-$C_6H_5$ |
| 145 | 4-(1-i-$C_3H_7$-$C_6H_4$) |
| 146 | 4-$C_6H_5$, 2,6-$(CH_3)_2$ |
| 147 | 2-Cl, 4-$C_6H_5$ |
| 148 | 2-Br, 4-$C_6H_5$ |
| 149 | 2-$C_6H_5$, 4-Cl |
| 150 | 2-$C_6H_5$, 4-Br |
| 151 | 2-$CH_2C_6H_5$, 4-Cl |
| 152 | 2-$CH_2C_6H_5$, 4-Br |
| 153 | 2-Cl, 4-$CH_2C_6H_5$ |
| 154 | 2-Br, 4-$CH_2C_6H_5$ |
| 155 | 2-cyclo-$C_6H_{11}$, 4-Cl |

| Nummer | $X_m$ |
|---|---|
| 156 | 2-cyclo-$C_6H_{11}$, 4-Br |
| 157 | 2-Cl, 4-cyclo-$C_6H_{11}$ |
| 158 | 2-Br, 4-cyclo-$C_6H_{11}$ |
| 159 | 2-$OCH_3$ |
| 160 | 3-$OCH_3$ |
| 161 | 4-$OCH_3$ |
| 162 | 2-$OC_2H_5$ |
| 163 | 3-O-$C_2H_5$ |
| 164 | 4-O-$C_2H_5$ |
| 165 | 2-O-n-$C_3H_7$ |
| 166 | 3-O-n-$C_3H_7$ |
| 167 | 4-O-n-$C_3H_7$ |
| 168 | 2-O-i-$C_3H_7$ |
| 169 | 3-O-i-$C_3H_7$ |
| 170 | 4-O-i-$C_3H_7$ |
| 171 | 2-O-n-$C_6H_{13}$ |
| 172 | 3-O-n-$C_6H_{13}$ |
| 173 | 4-O-n-$C_6H_{13}$ |
| 174 | 2-O-n-$C_8H_{17}$ |
| 175 | 3-O-n-$C_8H_{17}$ |
| 176 | 4-O-n-$C_8H_{17}$ |
| 177 | 2-O-$CH_2C_6H_5$ |
| 178 | 3-O-$CH_2C_6H_5$ |
| 179 | 4-O-$CH_2C_6H_5$ |
| 180 | 2-O-$(CH_2)_3C_6H_5$ |
| 181 | 3-O-$(CH_2)_3C_6H_5$ |
| 182 | 4-O-$(CH_2)_3C_6H_5$ |
| 183 | 2,4-$(OCH_3)_2$ |
| 184 | 2-$CF_3$ |
| 185 | 3-$CF_3$ |
| 186 | 4-$CF_3$ |
| 187 | 2-$OCF_3$ |
| 188 | 3-$OCF_3$ |
| 189 | 4-$OCF_3$ |
| 190 | 3-$OCH_2CHF_2$ |
| 191 | 2-$NO_2$ |

| Nummer | $X_m$ |
|---|---|
| 192 | 3-$NO_2$ |
| 193 | 4-$NO_2$ |
| 194 | 2-CN |
| 195 | 3-CN |
| 196 | 4-CN |
| 197 | 2-$CH_3$, 3-Cl |
| 198 | 2-$CH_3$, 4-Cl |
| 199 | 2-$CH_3$, 5-Cl |
| 200 | 2-$CH_3$, 6-Cl |
| 201 | 2-$CH_3$, 3-F |
| 202 | 2-$CH_3$, 4-F |
| 203 | 2-$CH_3$, 5-F |
| 204 | 2-$CH_3$, 6-F |
| 205 | 2-$CH_3$, 3-Br |
| 206 | 2-$CH_3$, 4-Br |
| 207 | 2-$CH_3$, 5-Br |
| 208 | 2-$CH_3$, 6-Br |
| 209 | 2-Cl, 3-$CH_3$ |
| 210 | 2-Cl, 4-$CH_3$ |
| 211 | 2-Cl, 5-$CH_3$ |
| 212 | 2-F, 3-$CH_3$ |
| 213 | 2-F, 4-$CH_3$ |
| 214 | 2-F, 5-$CH_3$ |
| 215 | 2-Br, 3-$CH_3$ |
| 216 | 2-Br, 4-$CH_3$ |
| 217 | 2-Br, 5-$CH_3$ |
| 218 | 3-$CH_3$, 4-Cl |
| 219 | 3-$CH_3$, 5-Cl |
| 220 | 3-$CH_3$, 4-F |
| 221 | 3-$CH_3$, 5-F |
| 222 | 3-$CH_3$, 4-Br |
| 223 | 3-$CH_3$, 5-Br |
| 224 | 3-F, 4-$CH_3$ |
| 225 | 3-Cl, 4-$CH_3$ |
| 226 | 3-Br, 4-$CH_3$ |
| 227 | 2-Cl, 4,5-$(CH_3)_2$ |

| Nummer | $X_m$ |
|---|---|
| 228 | 2-Br, 4,5-$(CH_3)_2$ |
| 229 | 2-Cl, 3,5-$(CH_3)_2$ |
| 230 | 2-Br, 3,5-$(CH_3)_2$ |
| 231 | 2,6-$Cl_2$, 4-$CH_3$ |
| 232 | 2,6-$F_2$, 4-$CH_3$ |
| 233 | 2,6-$Br_2$, 4-$CH_3$ |
| 234 | 2,4-$Br_2$, 6-$CH_3$ |
| 235 | 2,4-$F_2$, 6-$CH_3$ |
| 236 | 2,4-$Br_2$, 6-$CH_3$ |
| 237 | 2,6-$(CH_3)_2$, 4-F |
| 238 | 2,6-$(CH_3)_2$, 4-Cl |
| 239 | 2,6-$(CH_3)_2$, 4-Br |
| 240 | 3,5-$(CH_3)_2$, 4-F |
| 241 | 3,5-$(CH_3)_2$, 4-Cl |
| 242 | 3,5-$(CH_3)_2$, 4-Br |
| 243 | 2,3,6-$(CH_3)_3$, 4-F |
| 244 | 2,3,6-$(CH_3)_3$, 4-Cl |
| 245 | 2,3,6-$(CH_3)_3$, 4-Br |
| 246 | 2,4-$(CH_3)_2$, 6-F |
| 247 | 2,4-$(CH_3)_2$, 6-Cl |
| 248 | 2,4-$(CH_3)_2$, 6-Br |
| 249 | 2-i-$C_3H_7$, 4-Cl, 5-$CH_3$ |
| 250 | 2-Cl, 4-$NO_2$ |
| 251 | 2-$NO_2$, 4-Cl |
| 252 | 2-$OCH_3$, 5-$NO_2$ |
| 253 | 2,4-$Cl_2$, 5-$NO_2$ |
| 254 | 2,4-$Cl_2$, 6-$NO_2$ |
| 255 | 2,6-$Cl_2$, 4-$NO_2$ |
| 256 | 2,6-$Br_2$, 4-$NO_2$ |
| 257 | 2,6-$J_2$, 4-$NO_2$ |
| 258 | 2-$CH_3$, 5-i-$C_3H_7$, 4-Cl |
| 259 | 2-$CO_2CH_3$ |
| 260 | 3-$CO_2CH_3$ |
| 261 | 4-$CO_2CH_3$ |
| 262 | 2-$CO_2(C_2H_5)$ |
| 263 | 3-$CO_2(C_2H_5)$ |

| Nummer | $X_m$ |
|---|---|
| 264 | 4-$CO_2(C_2H_5)$ |
| 265 | 2-$CO_2$(n-$C_3H_7$) |
| 266 | 3-$CO_2$(n-$C_3H_7$) |
| 267 | 4-$CO_2$(n-$C_3H_7$) |
| 268 | 2-$CO_2$(i-$C_3H_7$) |
| 269 | 3-$CO_2$(i-$C_3H_7$) |
| 270 | 4-$CO_2$(i-$C_3H_7$) |
| 271 | 2-$CO_2$(n-$C_6H_{13}$) |
| 272 | 3-$CO_2$(n-$C_6H_{13}$) |
| 273 | 4-$CO_2$(n-$C_6H_{13}$) |
| 274 | 2-$CH_2$-$OCH_3$ |
| 275 | 3-$CH_2$-$OCH_3$ |
| 276 | 4-$CH_2$-$OCH_3$ |
| 277 | 2-$CH_2O(C_2H_5)$ |
| 278 | 3-$CH_2O(C_2H_5)$ |
| 279 | 4-$CH_2O(C_2H_5)$ |
| 280 | 2-$CH_2O$(n-$C_3H_7$) |
| 281 | 3-$CH_2O$(n-$C_3H_7$) |
| 282 | 4-$CH_2O$(n-$C_3H_7$) |
| 283 | 2-$CH_2O$(i-$C_3H_7$) |
| 284 | 3-$CH_2O$(i-$C_3H_7$) |
| 285 | 4-$CH_2O$(i-$C_3H_7$) |
| 286 | 2-CHO |
| 287 | 3-CHO |
| 288 | 4-CHO |
| 289 | 2-CO-$CH_3$ |
| 290 | 3-CO-$CH_3$ |
| 291 | 4-CO-$CH_3$ |
| 292 | 2-CO-$CH_2$-$CH_3$ |
| 293 | 3-CO-$CH_2$-$CH_3$ |
| 294 | 4-CO-$CH_2$-$CH_3$ |
| 295 | 2-CO-$CH_2$-$CH_2$-$CH_3$ |
| 296 | 3-CO-$CH_2$-$CH_2$-$CH_3$ |
| 297 | 4-CO-$CH_2$-$CH_2$-$CH_3$ |
| 298 | 2-CO-$CH(CH_3)$-$CH_3$ |
| 299 | 3-CO-$CH(CH_3)$-$CH_3$ |

| Nummer | $X_m$ |
|---|---|
| 300 | 4-CO-CH($CH_3$)-$CH_3$ |
| 301 | 2-Me-4-CHO |
| 302 | 2-Me-4-$CH_3$-CO |
| 303 | 2-Me-4-$CH_3$-$CH_2$-CO |
| 304 | 2-Me-4-$CH_3$-$CH_2$-$CH_2$-CO |
| 305 | 2-Me-4-$CH_3$-CH($CH_3$)-CO |
| 306 | 2,5-$Me_2$-4-CHO |
| 307 | 2,5-$Me_2$-4-$CH_3$-CO |
| 308 | 2,5-$Me_2$-4-$CH_3$-$CH_2$-CO |
| 309 | 2,3-$Me_2$-4-$CH_3$-$CH_2$-$CH_2$-CO |
| 310 | 2,5-$Me_2$-4-$CH_3$-CH($CH_3$)-CO |
| 311 | 2-Cl-4-CHO |
| 312 | 2-Cl-4-$CH_3$-CO |
| 313 | 2-Cl-4-$CH_3$-$CH_2$-CO |
| 314 | 2-Cl-4-$CH_3$-CH($CH_3$)-CO |
| 315 | 2,5-$Cl_2$-4-CHO |
| 316 | 2,5-$Cl_2$-4-CHO |
| 317 | 2,5-$Cl_2$-4-$CH_3$-$CH_2$-CO |
| 318 | 2,5-$Cl_2$-4-$CH_3$-$CH_2$-$CH_2$-CO |
| 319 | 2,5-$Cl_2$-4-$CH_3$-CH($CH_3$)-CO |
| 320 | 2-C(=$NOCH_3$)-$CH_3$ |
| 321 | 3-C(=$NOCH_3$)-$CH_3$ |
| 322 | 4-C(=$NOC_2H_5$)-$CH_3$ |
| 323 | 2-C(=$NOC_2H_5$)-$CH_3$ |
| 324 | 3-C(=$NOC_2H_5$)-$CH_3$ |
| 325 | 4-C(=$NOC_2H_5$)-$CH_3$ |
| 326 | 2-C(=NO-n-$C_3H_7$)-$CH_3$ |
| 327 | 3-C(=NO-n-$C_3H_7$)-$CH_3$ |
| 328 | 4-C(=NO-n-$C_3H_7$)-$CH_3$ |
| 329 | 2-C(=NO-i-$C_3H_7$)-$CH_3$ |
| 330 | 3-C(=NO-i-$C_3H_7$)-$CH_3$ |
| 331 | 4-C(=NO-i-$C_3H_7$)-$CH_3$ |
| 332 | 2-C(=NO-Allyl)-$CH_3$ |
| 333 | 3-C(=NO-Allyl)-$CH_3$ |
| 334 | 4-C(=NO-Allyl)-$CH_3$ |
| 335 | 2-C(=NO-trans-Chlorallyl)-$CH_3$ |

| Nummer | $X_m$ |
|---|---|
| 336 | 3-C(=NO-trans-Chlorallyl)-$CH_3$ |
| 337 | 4-C(=NO-trans-Chlorallyl)-$CH_3$ |
| 338 | 2-C(=NO-Propargyl)-$CH_3$ |
| 339 | 3-C(=NO-Propargyl)-$CH_3$ |
| 340 | 4-C(=NO-Propargyl)-$CH_3$ |
| 341 | 2-C(=NO-n-$C_4H_9$)-$CH_3$ |
| 342 | 3-C(=NO-n-$C_4H_9$)-$CH_3$ |
| 343 | 4-C(=NO-n-$C_4H_9$)-$CH_3$ |
| 344 | 2-C(=NO-$CH_2$-$C_6H_5$)-$CH_3$ |
| 345 | 3-C(=NO-$CH_2$-$C_6H_5$)-$CH_3$ |
| 346 | 4-C(=NO-$CH_2$-$C_6H_5$)-$CH_3$ |
| 347 | 2-$CH_3$-4-CH=$NOCH_3$ |
| 348 | 2-$CH_3$-4-CH=$NOC_2H_5$ |
| 349 | 2-$CH_3$-4-CH=NO-n-$C_3H_7$ |
| 350 | 2-$CH_3$-4-CH=NO-i-$C_3H_7$ |
| 351 | 2-$CH_3$-4-CH=NO-Allyl |
| 352 | 2-$CH_3$-4-CH=NO-(trans-Chlorallyl) |
| 353 | 2-$CH_3$-4-CH=NO-Propargyl |
| 354 | 2-$CH_3$-4-CH=NO-n-$C_4H_9$ |
| 355 | 2-$CH_3$-4-CH=NO-$CH_2$-$C_6H_5$ |
| 356 | 2-$CH_3$-4-($CH_3$-C=$NOCH_3$) |
| 357 | 2-$CH_3$-4-($CH_3$-C=$NOC_2H_5$) |
| 358 | 2-$CH_3$-4-($CH_3$-C=NO-n-$C_3H_7$) |
| 359 | 2-$CH_3$-4-($CH_3$-C=NO-i-$C_3H_7$) |
| 360 | 2-$CH_3$-4-($CH_3$-C=NO-Allyl) |
| 361 | 2-$CH_3$-4-($CH_3$-C=NO-trans-Chlorallyl) |
| 362 | 2-$CH_3$-4-($CH_3$-C=NO-Propargyl) |
| 363 | 2-$CH_3$-4-($CH_3$-C=NO-n-$C_4H_5$) |
| 364 | 2-$CH_3$-4-($CH_3$-C=NO-$CH_2$-$C_6H_5$) |
| 365 | 2-$CH_3$-4-($C_2H_5$-C=NO-$CH_3$) |
| 366 | 2-$CH_3$-4-($C_2H_5$-C=NO-$C_2H_5$) |
| 367 | 2-$CH_3$-4-($C_2H_5$-C=NO-n-$C_3H_7$) |
| 368 | 2-$CH_3$-4-($C_2H_5$-C=NO-i-$C_3H_7$) |
| 369 | 2-$CH_3$-4-($C_2H_5$-C=NO-Allyl) |
| 370 | 2-$CH_3$-4-($C_2H_5$-C=NO-trans-Chlorallyl) |
| 371 | 2-$CH_3$-4-($C_2H_5$-C=NO-Propargyl) |

| Nummer | $X_m$ |
|---|---|
| 372 | 2-$CH_3$-4-($C_2H_5$-C=NO-n-$C_4H_9$) |
| 373 | 2-$CH_3$-4-($C_2H_5$-C=NO-$CH_2$-$C_6H_5$) |
| 374 | 2,5-($CH_3$)2-4-($CH_3$-C=$NOCH_3$) |
| 375 | 2,5-($CH_3$)2-4-($CH_3$-C=$NOC_2H_5$) |
| 376 | 2,5-($CH_3$)2-4-($CH_3$-C=NO-n-$C_3H_7$) |
| 377 | 2,5-($CH_3$)2-4-($CH_3$-C=NO-i-$C_3H_7$) |
| 378 | 2,5-($CH_3$)2-4-($CH_3$-C=NO-Allyl) |
| 379 | 2,5-($CH_3$)2-4-($CH_3$-C=NO-trans-Chlorallyl) |
| 380 | 2,5-($CH_3$)2-4-($CH_3$-C=NO-Proparyl) |
| 381 | 2,5-($CH_3$)2-4-($CH_3$-C=NO-n-$C_4H_9$) |
| 382 | 2,5-($CH_3$)2-4-($CH_3$-C=NO-$CH_2$-$C_6H_5$) |
| 383 | 2-$C_6H_5$ |
| 384 | 3-$C_6H_5$ |
| 385 | 4-$C_6H_5$ |
| 386 | 2-(2'-F-$C_6H_4$) |
| 387 | 2-(3'-F-$C_6H_4$) |
| 388 | 2-(4'-F-$C_6H_4$) |
| 389 | 3-(2'-F-$C_6H_4$) |
| 390 | 3-(3'-F-$C_6H_4$) |
| 391 | 3-(4'-F-$C_6H_4$) |
| 392 | 4-(2'-F-$C_6H_4$) |
| 393 | 4-(3'-F-$C_6H_4$) |
| 394 | 4-(4'-F-$C_6H_4$) |
| 395 | 2-(2'-Cl-$C_6H_4$) |
| 396 | 2-(3'-Cl-$C_6H_4$) |
| 397 | 2-(4'-Cl-$C_6H_4$) |
| 398 | 3-(2'-Cl-$C_6H_4$) |
| 399 | 3-(3'-Cl-$C_6H_4$) |
| 400 | 3-(4'-Cl-$C_6H_4$) |
| 401 | 4-(2'-Cl-$C_6H_4$) |
| 402 | 4-(3'-Cl-$C_6H_4$) |
| 403 | 4-(4'-Cl-$C_6H_4$) |
| 404 | 2-(2'-$CH_3$-$C_6H_4$) |
| 405 | 2-(3'-$CH_3$-$C_6H_4$) |
| 406 | 2-(4'-$CH_3$-$C_6H_4$) |
| 407 | 3-(2'-$CH_3$-$C_6H_4$) |

| Nummer | $X_m$ |
|---|---|
| 408 | 3-(3'-$CH_3$-$C_6H_4$) |
| 409 | 3-(4'-$CH_3$-$C_6H_4$) |
| 410 | 4-(2'-$CH_3$-$C_6H_4$) |
| 411 | 4-(3'-$CH_3$-$C_6H_4$) |
| 412 | 4-(4'-$CH_3$-$C_6H_4$) |
| 413 | 2-(2'-$CH_3$-CO-$C_6H_4$) |
| 414 | 2-(3'-$CH_3$-CO-$C_6H_4$) |
| 415 | 2-(4'-$CH_3$-CO-$C_6H_4$) |
| 416 | 3-(2'-$CH_3$-CO-$C_6H_4$) |
| 417 | 3-(3'-$CH_3$-CO-$C_6H_4$) |
| 418 | 3-(4'-$CH_3$-CO-$C_6H_4$) |
| 419 | 4-(2'-$CH_3$-CO-$C_6H_4$) |
| 420 | 4-(3'-$CH_3$-CO-$C_6H_4$) |
| 421 | 4-(4'-$CH_3$-CO-$C_6H_4$) |
| 422 | 2-(2'-($CH_3$-C(=NOAllyl))-$C_6H_4$) |
| 423 | 2-(3'-($CH_3$-C(=NOAllyl))-$C_6H_4$) |
| 424 | 2-(4'-($CH_3$-C(=NOAllyl))-$C_6H_4$) |
| 425 | 3-(2'-($CH_3$-C(=NOAllyl))-$C_6H_4$) |
| 426 | 3-(3'-($CH_3$-C(=NOAllyl))-$C_6H_4$) |
| 427 | 3-(4'-($CH_3$-C(=NOAllyl))-$C_6H_4$) |
| 428 | 4-(2'-($CH_3$-C(=NOAllyl))-$C_6H_4$) |
| 429 | 4-(3'-($CH_3$-C(=NOAllyl))-$C_6H_4$) |
| 430 | 4-(4'-($CH_3$-C(=NOAllyl))-$C_6H_4$) |
| 431 | 2-(2'-$CH_3O_2C$-$C_6H_4$) |
| 432 | 2-(3'-$CH_3O_2C$-$C_6H_4$) |
| 433 | 2-(4'-$CH_3O_2C$-$C_6H_4$) |
| 434 | 3-(2'-$CH_3O_2C$-$C_6H_4$) |
| 435 | 3-(3'-$CH_3O_2C$-$C_6H_4$) |
| 436 | 3-(4'-$CH_3O_2C$-$C_6H_4$) |
| 437 | 4-(2'-$CH_3O_2C$-$C_6H_4$) |
| 438 | 4-(3'-$CH_3O_2C$-$C_6H_4$) |
| 439 | 4-(4'-$CH_3O_2C$-$C_6H_4$) |
| 440 | 2-(2'-$CH_3O$-$C_6H_4$) |
| 441 | 2-(3'-$CH_3O$-$C_6H_4$) |
| 442 | 2-(4'-$CH_3O$-$C_6H_4$) |
| 443 | 3-(2'-$CH_3O$-$C_6H_4$) |

| Nummer | $X_m$ |
|---|---|
| 444 | 3-(3'-$CH_3O$-$C_6H_4$) |
| 445 | 3-(4'-$CH_3O$-$CH_4$) |
| 446 | 4-(2'-$CH_3O$-$C_6H_4$) |
| 447 | 4-(3'-$CH_3O$-$C_6H_4$) |
| 448 | 4-(4'-$CH_3O$-$C_6H_4$) |
| 449 | 2-(2'-$O_2N$-$C_6H_4$) |
| 450 | 2-(3'-$O_2N$-$C_6H_4$) |
| 451 | 2-(4'-$O_2N$-$C_6H_4$) |
| 452 | 3-(2'-$O_2N$-$C_6H_4$) |
| 453 | 3-(3'-$O_2N$-$C_6H_4$) |
| 454 | 3-(4'-$O_2N$-$C_6H_4$) |
| 455 | 4-(2'-$O_2N$-$C_6H_4$) |
| 456 | 4-(3'-$O_2N$-$C_6H_4$) |
| 457 | 4-(4'-$O_2N$-$C_6H_4$) |
| 458 | 2-(2'-NC-$C_6H_4$) |
| 459 | 2-(3'-NC-$C_6H_4$) |
| 460 | 2-(4'-NC-$C_6H_4$) |
| 461 | 3-(2'-NC-$C_6H_4$) |
| 462 | 3-(3'-NC-$C_6H_4$) |
| 463 | 3-(4'-NC-$C_6H_4$) |
| 464 | 4-(2'-NC-$C_6H_4$) |
| 465 | 4-(3'-NC-$C_6H_4$) |
| 466 | 4-(4'-NC-$C_6H_4$) |
| 467 | 2-(2'-$CF_3$-$C_6H_4$) |
| 468 | 2-(3'-$CF_3$-$C_6H_4$) |
| 469 | 2-(4'-$CF_3$-$C_6H_4$) |
| 470 | 3-(2'-$CF_3$-$C_6H_4$) |
| 471 | 3-(3'-$CF_3$-$C_6H_4$) |
| 472 | 3-(4'-$CF_3$-$C_6H_4$) |
| 473 | 4-(2'-$CF_3$-$C_6H_4$) |
| 474 | 4-(3'-$CF_3$-$C_6H_4$) |
| 475 | 4-(4'-$CF_3$-$C_6H_4$) |
| 476 | 2-O-$C_6H_5$ |
| 477 | 3-O-$C_6H_5$ |
| 478 | 4-O-$C_6H_5$ |
| 479 | 2-O-(2'-F-$C_6H_4$) |

| Nummer | $X_m$ |
|---|---|
| 480 | 2-O-(3'-F-$C_6H_4$) |
| 481 | 2-O-(4'-F-$C_6H_4$) |
| 482 | 3-O-(2'-F-$C_6H_4$) |
| 483 | 3-O-(3'-F-$C_6H_4$) |
| 484 | 3-O-(4'-F-$C_6H_4$) |
| 485 | 4-O-(2'-F-$C_6H_4$) |
| 486 | 4-O-(3'-F-$C_6H_4$) |
| 487 | 4-O-(4'-F-$C_6H_4$) |
| 488 | 2-O-(2'-Cl-$C_6H_4$) |
| 489 | 2-O-(3'-Cl-$C_6H_4$) |
| 490 | 2-O-(4'-Cl-$C_6H_4$) |
| 491 | 3-O-(2'-Cl-$C_6H_4$) |
| 492 | 3-O-(3'-Cl-$C_6H_4$) |
| 493 | 3-O-(4'-Cl-$C_6H_4$) |
| 494 | 4-O-(2'-Cl-$C_6H_4$) |
| 495 | 4-O-(3'-Cl-$C_6H_4$) |
| 496 | 4-O-(4'-Cl-$C_6H_4$) |
| 497 | 2-O-(2'-$CH_3$-$C_6H_4$) |
| 498 | 2-O-(3'-$CH_3$-$C_6H_4$) |
| 499 | 2-O-(4'-$CH_3$-$C_6H_4$) |
| 500 | 3-O-(2'-$CH_3$-$C_6H_4$) |
| 501 | 3-O-(3'-$CH_3$-$C_6H_4$) |
| 502 | 3-O-(4'-$CH_3$-$C_6H_4$) |
| 503 | 4-O-(2'-$CH_3$-$C_6H_4$) |
| 504 | 4-O-(3'-$CH_3$-$C_6H_4$) |
| 506 | 4-O-(4'-$CH_3$-$C_6H_4$) |
| 507 | 2-O-(2'-$CH_3$-CO-$C_6H_4$) |
| 508 | 2-O-(3'-$CH_3$-CO-$C_6H_4$) |
| 509 | 2-O-(4'-$CH_3$-CO-$C_6H_4$) |
| 510 | 3-O-(2'-$CH_3$-CO-$C_6H_4$) |
| 511 | 3-O-(3'-$CH_3$-CO-$C_6H_4$) |
| 512 | 3-O-(4'-$CH_3$-CO-$C_6H_4$) |
| 513 | 4-O-(2'-$CH_3$-CO-$C_6H_4$) |
| 514 | 4-O-(3'-$CH_3$-CO-$C_6H_4$) |
| 515 | 4-O-(4'-$CH_3$-CO-$C_6H_4$) |
| 516 | 2-O-(2'-($CH_3$-C(=NOAllyl))-$C_6H_4$) |

| Nummer | $X_m$ |
|---|---|
| 517 | 2-O-(3'-($CH_3$-C(=NOAllyl))-$C_6H_4$) |
| 518 | 2-O-(4'-($CH_3$-C(=NOAllyl))-$C_6H_4$) |
| 519 | 3-O-(2'-($CH_3$-C(=NOAllyl))-$C_6H_4$) |
| 520 | 3-O-(3'-($CH_3$-C(=NOAllyl))-$C_6H_4$) |
| 521 | 3-O-(4'-($CH_3$-C(=NOAllyl))-$C_6H_4$) |
| 522 | 4-O-(2'-($CH_3$-C(=NOAllyl))-$C_6H_4$) |
| 523 | 4-O-(3'-($CH_3$-C(=NOAllyl))-$C_6H_4$) |
| 524 | 4-O-(4'-($CH_3$-C(=NOAllyl))-$C_6H_4$) |
| 525 | 2-O-(2'-$CH_3O_2C$-$C_6H_4$) |
| 526 | 2-O-(3'-$CH_3O_2C$-$C_6H_4$) |
| 527 | 2-O-(4'-$CH_3O_2C$-$C_6H_4$) |
| 528 | 3-O-(2'-$CH_3O_2C$-$C_6H_4$) |
| 529 | 3-O-(3'-$CH_3O_2C$-$C_6H_4$) |
| 530 | 3-O-(4'-$CH_3O_2C$-$C_6H_4$) |
| 531 | 4-O-(2'-$CH_3O_2C$-$C_6H_4$) |
| 532 | 4-O-(3'-$CH_3O_2C$-$C_6H_4$) |
| 533 | 4-O-(4'-$CH_3O_2C$-$C_6H_4$) |
| 534 | 2-O-(2'-$CH_3O$-$C_6H_4$) |
| 535 | 2-O-(3'-$CH_3O$-$C_6H_4$) |
| 536 | 2-O-(4'-$CH_3O$-$C_6H_4$) |
| 537 | 3-O-(2'-$CH_3O$-$C_6H_4$) |
| 538 | 3-O-(3'-$CH_3O$-$C_6H_4$) |
| 539 | 3-O-(4'-$CH_3O$-$C_6H_4$) |
| 540 | 4-O-(2'-$CH_3O$-$C_6H_4$) |
| 541 | 4-O-(3'-$CH_3O$-$C_6H_4$) |
| 542 | 4-O-(4'-$CH_3O$-$C_6H_4$) |
| 543 | 2-O-(2'-$O_2N$-$C_6H_4$) |
| 544 | 2-O-(3'-$O_2N$-$C_6H_4$) |
| 545 | 2-O-(4'-$O_2N$-$C_6H_4$) |
| 546 | 3-O-(2'-$O_2N$-$C_6H_4$) |
| 547 | 3-O-(3'-$O_2N$-$C_6H_4$) |
| 548 | 3-O-(4'-$O_2N$-$C_6H_4$) |
| 549 | 4-O-(2'-$O_2N$-$C_6H_4$) |
| 550 | 4-O-(3'-$O_2N$-$C_6H_4$) |
| 551 | 4-O-(4'-$O_2N$-$C_6H_4$) |
| 552 | 2-O-(2'-NC-$C_6H_4$) |

| Nummer | $X_m$ |
|---|---|
| 553 | 2-O-(3'-NC-$C_6H_4$) |
| 554 | 2-O-(4'-NC-$C_6H_4$) |
| 555 | 3-O-(2'-NC-$C_6H_4$) |
| 556 | 3-O-(3'-NC-$C_6H_4$) |
| 557 | 3-O-(4'-NC-$C_6H_4$) |
| 558 | 4-O-(2'-NC-$C_6H_4$) |
| 559 | 4-O-(3'-NC-$C_6H_4$) |
| 560 | 2-O-(2'-$CF_3$-$C_6H_4$) |
| 561 | 2-O-(3'-$CF_3$-$C_6H_4$) |
| 562 | 2-O-(4'-$CF_3$-$C_6H_4$) |
| 563 | 3-O-(2'-$CF_3$-$C_6H_4$) |
| 564 | 3-O-(3'-$CF_3$-$C_6H_4$) |
| 565 | 3-O-(4'-$CF_3$-$C_6H_4$) |
| 566 | 4-O-(2'-$CF_3$-$C_6H_4$) |
| 567 | 4-O-(3'-$CF_3$-$C_6H_4$) |
| 568 | 4-O-(4'-$CF_3$-$C_6H_4$) |
| 569 | 2-Pyridinyl-2' |
| 570 | 2-Pyridinyl-3' |
| 571 | 2-Pyridinyl-4' |
| 572 | 3-Pyridinyl-2' |
| 573 | 3-Pyridinyl-3' |
| 574 | 3-Pyridinyl-4' |
| 575 | 4-Pyridinyl-2' |
| 576 | 4-Pyridinyl-3' |
| 577 | 4-Pyridinyl-4' |
| 578 | 2-Pyrimidinyl-2' |
| 579 | 2-Pyrimidinyl-5' |
| 580 | 2-Pyrimidinyl-4' |
| 581 | 3-Pyrimidinyl-2' |
| 582 | 3-Pyrimidinyl-5' |
| 583 | 3-Pyrimidinyl-4' |
| 584 | 4-Pyrimidinyl-2' |
| 585 | 4-Pyrimidinyl-5' |
| 586 | 4-Pyrimidinyl-4' |
| 587 | 2-Pyrazolyl-1' |
| 588 | 2-Pyrazolyl-3' |

| Nummer | $X_m$ |
|---|---|
| 589 | 2-Pyrazolyl-4' |
| 590 | 3-Pyrazolyl-1' |
| 591 | 3-Pyrazolyl-3' |
| 592 | 3-Pyrazolyl-4' |
| 593 | 4-Pyrazolyl-1' |
| 594 | 4-Pyrazolyl-3' |
| 595 | 4-Pyrazolyl-4' |
| 596 | 2-Isoxazolyl-3' |
| 597 | 2-Isoxazolyl-4' |
| 598 | 2-Isoxazolyl-5' |
| 599 | 3-Isoxazolyl-3' |
| 600 | 3-Isoxazolyl-4' |
| 601 | 3-Isoxazolyl-5' |
| 602 | 4-Isoxazolyl-3' |
| 603 | 4-Isoxazolyl-4' |
| 604 | 4-Isoxazolyl-5' |
| 605 | 2-Isothiazolyl-3' |
| 606 | 2-Isothiazolyl-4' |
| 607 | 2-Isothiazolyl-5' |
| 608 | 3-Isothiazolyl-3' |
| 609 | 3-Isothiazolyl-4' |
| 610 | 3-Isothiazolyl-5' |
| 611 | 4-Isothiazolyl-3' |
| 612 | 4-Isothiazolyl-4' |
| 613 | 4-Isothiazolyl-5' |
| 614 | 2-Imidazolyl-1' |
| 615 | 2-Imidazolyl-2' |
| 616 | 2-Imidazolyl-4' |
| 617 | 3-Imidazolyl-1' |
| 618 | 3-Imidazolyl-2' |
| 619 | 3-Imidazolyl-4' |
| 620 | 4-Imidazolyl-1' |
| 621 | 4-Imidazolyl-2' |
| 622 | 4-Imidazolyl-4' |
| 623 | 2-Oxazolyl-2' |
| 624 | 2-Oxazolyl-4' |

| Nummer | $X_m$ |
|---|---|
| 625 | 2-Oxazolyl-5' |
| 626 | 3-Oxazolyl-2' |
| 627 | 3-Oxazolyl-4' |
| 628 | 3-Oxazolyl-5' |
| 629 | 4-Oxazolyl-2' |
| 630 | 4-Oxazolyl-4' |
| 631 | 4-Oxazolyl-5' |
| 632 | 2-Thiazolyl-2' |
| 633 | 2-Thiazolyl-4' |
| 634 | 2-Thiazolyl-5' |
| 635 | 3-Thiazolyl-2' |
| 636 | 3-Thiazolyl-4' |
| 637 | 3-Thiazolyl-5' |
| 638 | 4-Thiazolyl-2' |
| 639 | 4-Thiazolyl-4' |
| 640 | 4-Thiazolyl-5' |

Tabelle 2

I:

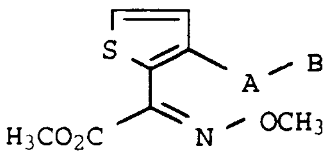

II:

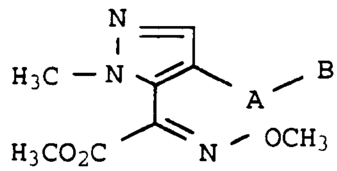

III:

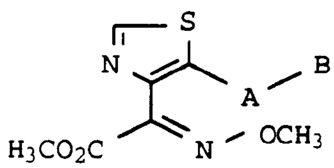

a) A = $-CH_2-O-$

b) A = 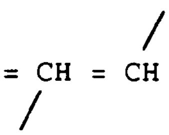

c) A = 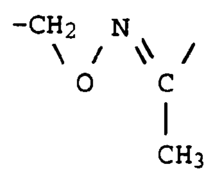

| Nummer | B |
|---|---|
| 1 | Pyrrolyl-3 |
| 2 | N-$CH_3$-Pyrrolyl-3 |
| 3 | N-$C_6H_5$-Pyrrolyl-3 |
| 4 | N-(4'-$CH_3$-$C_6H_4$)-Pyrrolyl-3 |
| 5 | N-(3'-$CH_3$-$C_6H_4$)-Pyrrolyl-3 |
| 6 | N-(2'-$CH_3$-$C_6H_4$)-Pyrrolyl-3 |
| 7 | N-(4'-$CH_3O$-$C_6H_4$)-Pyrrolyl-3 |
| 8 | N-(3'-$CH_3O$-$C_6H_4$)-Pyrrolyl-3 |
| 9 | N-(2'-$CH_3O$-$C_6H_4$)-Pyrrolyl-3 |
| 10 | N-(4'-$NO_2$-$C_6H_4$)-Pyrrolyl-3 |
| 11 | N-(3'-$NO_2$-$C_6H_4$)-Pyrrolyl-3 |

| Nummer | B |
|---|---|
| 12 | N-(2'-$NO_2$-$C_6H_4$)-Pyrrolyl-3 |
| 13 | N-(4'-CN-$C_6H_4$)-Pyrrolyl-3 |
| 14 | N-(3'-CN-$C_6H_4$)-Pyrrolyl-3 |
| 15 | N-(2'-CN-$C_6H_4$)-Pyrrolyl-3 |
| 16 | N-(4'-Cl-$C_6H_4$)-Pyrrolyl-3 |
| 17 | N-(3'-Cl-$C_6H_4$)-Pyrrolyl-3 |
| 18 | N-(2'-Cl-$C_6H_4$)-Pyrrolyl-3 |
| 19 | Pyrrolyl-2 |
| 20 | N-$CH_3$-Pyrrolyl-2 |
| 21 | N-$C_6H_5$-Pyrrolyl-2 |
| 22 | N-(4'-$CH_3$-$C_6H_4$)-Pyrrolyl-2 |
| 23 | N-(3'-$CH_3$-$C_6H_4$)-Pyrrolyl-2 |
| 24 | N-(2'-$CH_3$-$C_6H_4$)-Pyrrolyl-2 |
| 25 | N-(4'-$CH_3O$-$C_6H_4$)-Pyrrolyl-2 |
| 26 | N-(3'-$CH_3O$-$C_6H_4$)-Pyrrolyl-2 |
| 27 | N-(2'-$CH_3O$-$C_6H_4$)-Pyrrolyl-2 |
| 28 | N-(4'-$NO_2$-$C_6H_4$)-Pyrrolyl-2 |
| 29 | N-(3'-$NO_2$-$C_6H_4$)-Pyrrolyl-2 |
| 30 | N-(2'-$NO_2$-$C_6H_4$)-Pyrrolyl-2 |
| 31 | N-(4'-CN-$C_6H_4$)-Pyrrolyl-2 |
| 32 | N-(3'-CN-$C_6H_4$)-Pyrrolyl-2 |
| 33 | N-(2'-CN-$C_6H_4$)-Pyrrolyl-2 |
| 34 | N-(4'-Cl-$C_6H_4$)-Pyrrolyl-2 |
| 35 | N-(3'-Cl-$C_6H_4$)-Pyrrolyl-2 |
| 36 | N-(2'-Cl-$C_6H_4$)-Pyrrolyl-2 |
| 37 | Furyl-2 |
| 38 | 5-$CH_3$-Furyl-2 |
| 39 | 5-$C_6H_5$-Furyl-2 |
| 40 | 5-(4'-$CH_3$-$C_6H_4$)-Furyl-2 |
| 41 | 5 -(3'-$CH_3$-$C_6H_4$)-Furyl-2 |
| 42 | 5-(2'-$CH_3$-$C_6H_4$)-Furyl-2 |
| 43 | 5-(4'-$CH_3O$-$C_6H_4$)-Furyl-2 |
| 44 | 5-(3'-$CH_3O$-$C_6H_4$)-Furyl-2 |
| 45 | 5-(2'-CH3O-C6H4)-Furyl-2 |
| 46 | 5-(4'-$NO_2$-$C_6H_4$)-Furyl-2 |
| 47 | 5-(3'-$NO_2$-$C_6H_4$)-Furyl-2 |

| Nummer | B |
|---|---|
| 48 | 5-(2'-$NO_2$-$C_6H_4$)-Furyl-2 |
| 49 | 5-(4'-CN-$C_6H_4$)-Furyl-2 |
| 50 | 5-(3'-CN-$C_6H_4$)-Furyl-2 |
| 51 | 5-(2'-CN-$C_6H_4$)-Furyl-2 |
| 52 | 5-(4'-Cl-$C_6H_4$)-Furyl-2 |
| 53 | 5-(3'-Cl-$C_6H_4$)-Furyl-2 |
| 54 | 5-(2'-Cl-$C_6H_4$)-Furyl-2 |
| 55 | 4-$CH_3$-Furyl-2 |
| 56 | 4-$C_6H_5$-Furyl-2 |
| 57 | 4-(4'-$CH_3$-$C_6H_4$)-Furyl-2 |
| 58 | 4 -(3'-$CH_3$-$C_6H_4$)-Furyl-2 |
| 59 | 4-(2'-$CH_3$-$C_6H_4$)-Furyl-2 |
| 60 | 4-(4'-$CH_3O$-$C_6H_4$)-Furyl-2 |
| 61 | 4-(3'-$CH_3O$-$C_6H_4$)-Furyl-2 |
| 62 | 4-(2'-$CH_3O$-$C_6H_4$)-Furyl-2 |
| 63 | 4-(4'-$NO_2$-$C_6H_4$)-Furyl-2 |
| 64 | 4-(3'-$NO_2$-$C_6H_4$)-Furyl-2 |
| 65 | 4-(2'-$NO_2$-$C_6H_4$)-Furyl-2 |
| 66 | 4-(4'-CN-$C_6H_4$)-Furyl-2 |
| 67 | 4-(3'-CN-$C_6H_4$)-Furyl-2 |
| 68 | 4-(2'-CN-$C_6H_4$)-Furyl-2 |
| 69 | 4-(4'-Cl-$C_6H_4$)-Furyl-2 |
| 70 | 4-(3'-Cl-$C_6H_4$)-Furyl-2 |
| 71 | 4-(2'-Cl-$C_6H_4$)-Furyl-2 |
| 72 | Thienyl-2 |
| 73 | 5-$CH_3$-Thienyl-2 |
| 74 | 5-$C_6H_5$-Thienyl-2 |
| 75 | 5-(4'-$CH_3$-$C_6H_4$)-Thienyl-2 |
| 76 | 5-(3'-$CH_3$-$C_6H_4$)-Thienyl-2 |
| 77 | 5-(2'-$CH_3$-$C_6H_4$)-Thienyl-2 |
| 78 | 5-(4'-$CH_3O$-$C_6H_4$)-Thienyl-2 |
| 79 | 5-(3'-$CH_3O$-$C_6H_4$)-Thienyl-2 |
| 80 | 5-(2'-$CH_3O$-$C_6H_4$)-Thienyl-2 |
| 81 | 5-(4'-$NO_2$-$C_6H_4$)-Thienyl-2 |
| 82 | 5-(3'-$NO_2$-$C_6H_4$)-Thienyl-2 |
| 83 | 5-(2'-$NO_2$-$C_6H_4$)-Thienyl-2 |

| Nummer | B |
|---|---|
| 84 | 5-(4'-CN-$C_6H_4$)-Thienyl-2 |
| 85 | 5-(3'-CN-$C_6H_4$)-Thienyl-2 |
| 86 | 5-(2'-CN-$C_6H_4$)-Thienyl-2 |
| 87 | 5-(4'-Cl-$C_6H_4$)-Thienyl-2 |
| 88 | 5-(3'-Cl-$C_6H_4$)-Thienyl-2 |
| 89 | 5-(2'-Cl-$C_6H_4$)-Thienyl-2 |
| 90 | 4-$CH_3$-Thienyl-2 |
| 91 | 4-$C_6H_5$-Thienyl-2 |
| 92 | 4-(4'-$CH_3$-$C_6H_4$)-Thienyl-2 |
| 93 | 4-(3'-$CH_3$-$C_6H_4$)-Thienyl-2 |
| 94 | 4-(2'-$CH_3$-$C_6H_4$)-Thienyl-2 |
| 95 | 4-(4'-$CH_3O$-$C_6H_4$)-Thienyl-2 |
| 96 | 4-(3'-$CH_3O$-$C_6H_4$)-Thienyl-2 |
| 97 | 4-(2'-$CH_3O$-$C_6H_4$)-Thienyl-2 |
| 98 | 4-(4'-$NO_2$-$C_6H_4$)-Thienyl-2 |
| 99 | 4-(3'-$NO_2$-$C_6H_4$)-Thienyl-2 |
| 100 | 4-(2'-$NO_2$-$C_6H_4$)-Thienyl-2 |
| 101 | 4-(4'-CN-$C_6H_4$)-Thienyl-2 |
| 102 | 4-(3'-CN-$C_6H_4$)-Thienyl-2 |
| 103 | 4-(2'-CN-$C_6H_4$)-Thienyl-2 |
| 104 | 4-(4'-Cl-$C_6H_4$)-Thienyl-2 |
| 105 | 4-(3'-Cl-$C_6H_4$)-Thienyl-2 |
| 106 | 4-(2'-Cl-$C_6H_4$)-Thienyl-2 |
| 107 | Thienyl-3 |
| 108 | 5-$CH_3$-Thienyl-3 |
| 109 | 5-$C_6H_5$-Thienyl-3 |
| 110 | 5-(4'-$CH_3$-$C_6H_4$)-Thienyl-3 |
| 111 | 5-(3'-$CH_3$-$C_6H_4$)-Thienyl-3 |
| 112 | 5-(2'-$CH_3$-$C_6H_4$)-Thienyl-3 |
| 113 | 5-(4'-$CH_3O$-$C_6H_4$)-Thienyl-3 |
| 114 | 5-(3'-$CH_3O$-$C_6H_4$)-Thienyl-3 |
| 115 | 5-(2'-$CH_3O$-$C_6H_4$)-Thienyl-3 |
| 116 | 5-(4'-$NO_2$-$C_6H_4$)-Thienyl-3 |
| 117 | 5-(3'-$NO_2$-$C_6H_4$)-Thienyl-3 |
| 118 | 5-(2'-$NO_2$-$C_6H_4$)-Thienyl-3 |
| 119 | 5-(4'-CN-$C_6H_4$)-Thienyl-3 |

| Nummer | B |
|---|---|
| 120 | 5-(3'-CN-$C_6H_4$)-Thienyl-3 |
| 121 | 5-(2'-CN-$C_6H_4$)-Thienyl-3 |
| 122 | 5-(4'-Cl-$C_6H_4$)-Thienyl-3 |
| 123 | 5-(3'-Cl-$C_6H_4$)-Thienyl-3 |
| 124 | 5-(2'-Cl-$C_6H_4$)-Thienyl-3 |
| 125 | Pyrazolyl-4 |
| 126 | N-$CH_3$-Pyrazolyl-4 |
| 127 | N-$C_6H_5$-Pyrazolyl-4 |
| 128 | N-(4'-$CH_3$-$C_6H_4$)-Pyrazolyl-4 |
| 129 | N-(3'-$CH_3$-$C_6H_4$)-Pyrazolyl-4 |
| 130 | N-(2'-$CH_3$-$C_6H_4$)-Pyrazolyl-4 |
| 131 | N-(4'-$CH_3O$-$C_6H_4$)-Pyrazolyl-4 |
| 132 | N-(3'-$CH_3O$-$C_6H_4$)-Pyrazolyl-4 |
| 133 | N-(2'-$CH_3O$-$C_6H_4$)-Pyrazolyl-4 |
| 134 | N-(4'-$NO_2$-$C_6H_4$)-Pyrazolyl-4 |
| 135 | N-(3'-$NO_2$-$C_6H_4$)-Pyrazolyl-4 |
| 136 | N-(2'-$NO_2$-$C_6H_4$)-Pyrazolyl-4 |
| 137 | N-(4'-CN-$C_6H_4$)-Pyrazolyl-4 |
| 138 | N-(3'-CN-$C_6H_4$)-Pyrazolyl-4 |
| 139 | N-(2'-CN-$C_6H_4$)-Pyrazolyl-4 |
| 140 | N-(4'-Cl-$C_6H_4$)-Pyrazolyl-4 |
| 141 | N-(3'-Cl-$C_6H_4$)-Pyrazolyl-4 |
| 142 | N-(2'-Cl-$C_6H_4$)-Pyrazolyl-4 |
| 143 | 3-$CH_3$-N-Methylpyrazolyl-4 |
| 144 | 3-$C_6H_5$-N-Methylpyrazolyl-4 |
| 145 | 3-(4'-$CH_3$-$C_6H_4$)-N-Methylpyrazolyl-4 |
| 146 | 3-(3'-$CH_3$-$C_6H_4$)-N-Methylpyrazolyl-4 |
| 147 | 3-(2'-$CH_3$-$C_6H_4$)-N-Methylpyrazolyl-4 |
| 148 | 3-(4'-$CH_3O$-$C_6H_4$)-N-Methylpyrazolyl-4 |
| 149 | 3-(3'-$CH_3O$-$C_6H_4$)-N-Methylpyrazolyl-4 |
| 150 | 3-(2'-$CH_3O$-$C_6H_4$)-N-Methylpyrazolyl-4 |
| 151 | 3-(4'-$NO_2$-$C_6H_4$)-N-Methylpyrazolyl-4 |
| 152 | 3-(3'-$NO_2$-$C_6H_4$)-N-Methylpyrazolyl-4 |
| 153 | 3-(2'-$NO_2$-$C_6H_4$)-N-Methylpyrazolyl-4 |
| 154 | 3-(4'-CN-$C_6H_4$)-N-Methylpyrazolyl-4 |
| 155 | 3-(3'-CN-$C_6H_4$)-N-Methylpyrazolyl-4 |

| Nummer | B |
|---|---|
| 156 | 3-(2'-CN-$C_6H_4$)-N-Methylpyrazolyl-4 |
| 157 | 3-(4'-Cl-$C_6H_4$)-N-Methylpyrazolyl-4 |
| 158 | 3-(3'-Cl-$C_6H_4$)-N-Methylpyrazolyl-4 |
| 159 | 3-(2'-Cl-$C_6H_4$)-N-Methylpyrazolyl-4 |
| 160 | Isoxazolyl-5 |
| 161 | 3-$CH_3$-Isoxazolyl-5 |
| 162 | 3-$C_6H_5$-Isoxazolyl-5 |
| 163 | 3-(4'-$CH_3$-$C_6H_4$)-Isoxazolyl-5 |
| 164 | 3-(3'-$CH_3$-$C_6H_4$)-Isoxazolyl-5 |
| 165 | 3-(2'-$CH_3$-$C_6H_4$)-Isoxazolyl-5 |
| 166 | 3-(4'-$CH_3O$-$C_6H_4$)-Isoxazolyl-5 |
| 167 | 3-(3'-$CH_3O$-$C_6H_4$)-Isoxazolyl-5 |
| 168 | 3-(2'-$CH_3O$-$C_6H_4$)-Isoxazolyl-5 |
| 169 | 3-(4'-$NO_2$-$C_6H_4$)-Isoxazolyl-5 |
| 170 | 3-(3'-$NO_2$-$C_6H_4$)-Isoxazolyl-5 |
| 171 | 3-(2'-$NO_2$-$C_6H_4$)-Isoxazolyl-5 |
| 172 | 3-(4'-CN-$C_6H_4$)-Isoxazolyl-5 |
| 173 | 3-(3'-CN-$C_6H_4$)-Isoxazolyl-5 |
| 174 | 3-(2'-CN-$C_6H_4$)-Isoxazolyl-5 |
| 175 | 3-(4'-Cl-$C_6H_4$)-Isoxazolyl-5 |
| 176 | 3-(3'-Cl-$C_6H_4$)-Isoxazolyl-5 |
| 177 | 3-(2'-Cl-$C_6H_4$)-Isoxazolyl-5 |
| 178 | 4-Chlorisoxazolyl-5 |
| 179 | 3-$CH_3$-4-Chlorisoxazolyl-5 |
| 180 | 3-$C_6H_5$-4-Chlorisoxazolyl-5 |
| 181 | 3-(4'-$CH_3$-$C_6H_4$)-Chlorisoxazolyl-5 |
| 182 | 3-(3'-$CH_3$-$C_6H_4$)-Chlorisoxazolyl-5 |
| 183 | 3-(2'-$CH_3$-$C_6H_4$)-Chlorisoxazolyl-5 |
| 184 | 3-(4'-$CH_3O$-$C_6H_4$)-Chlorisoxazolyl-5 |
| 185 | 3-(3'-$CH_3O$-$C_6H_4$)-Chlorisoxazolyl-5 |
| 186 | 3-(2'-$CH_3O$-$C_6H_4$)-Chlorisoxazolyl-5 |
| 187 | 3-(4'-$NO_2$-$C_6H_4$)-Chlorisoxazolyl-5 |
| 188 | 3-(3'-$NO_2$-$C_6H_4$)-Chlorisoxazolyl-5 |
| 189 | 3-(2'-$NO_2$-$C_6H_4$)-Chlorisoxazolyl-5 |
| 190 | 3-(4'-CN-$C_6H_4$)-Chlorisoxazolyl-5 |
| 191 | 3-(3'-CN-$C_6H_4$)-Chlorisoxazolyl-5 |

| Nummer | B |
|---|---|
| 192 | 3-(2'-CN-$C_6H_4$)-Chlorisoxazolyl-5 |
| 193 | 3-(4'-Cl-$C_6H_4$)-Chlorisoxazolyl-5 |
| 194 | 3-(3'-Cl-$C_6H_4$)-Chlorisoxazolyl-5 |
| 195 | 3-(2'-Cl-$C_6H_4$)-Chlorisoxazolyl-5 |
| 196 | Isoxazolyl-3 |
| 197 | 5-$CH_3$-Isoxazolyl-3 |
| 198 | 5-$C_6H_5$-Isoxazolyl-3 |
| 199 | 5-(4'-$CH_3$-$C_6H_4$)-Isoxazolyl-3 |
| 200 | 5-(3'-$CH_3$-$C_6H_4$)-Isoxazolyl-3 |
| 201 | 5-(2'-$CH_3$-$C_6H_4$)-Isoxazolyl-3 |
| 202 | 5-(4'-$CH_3O$-$C_6H_4$)-Isoxazolyl-3 |
| 203 | 5-(3'-$CH_3O$-$C_6H_4$)-Isoxazolyl-3 |
| 204 | 5-(2'-$CH_3O$-$C_6H_4$)-Isoxazolyl-3 |
| 205 | 5-(4'-$NO_2$-$C_6H_4$)-Isoxazolyl-3 |
| 206 | 5-(3'-$NO_2$-$C_6H_4$)-Isoxazolyl-3 |
| 207 | 5-(2'-$NO_2$-$C_6H_4$)-Isoxazolyl-3 |
| 208 | 5-(4'-CN-$C_6H_4$)-Isoxazolyl-3 |
| 209 | 5-(3'-CN-$C_6H_4$)-Isoxazolyl-3 |
| 210 | 5-(2'-CN-$C_6H_4$)-Isoxazolyl-3 |
| 211 | 5-(4'-Cl-$C_6H_4$)-Isoxazolyl-3 |
| 212 | 5-(3'-Cl-$C_6H_4$)-Isoxazolyl-3 |
| 213 | 5-(2'-Cl-$C_6H_4$)-Isoxazolyl-3 |
| 214 | Isothiazolyl-5 |
| 215 | 3-$CH_3$-Isothiazolyl-5 |
| 216 | 3-$C_6H_5$-Isothiazolyl-5 |
| 217 | 3-(4'-$CH_3$-C6H4)-Isothiazolyl-5 |
| 218 | 3-(3'-CH3-C6H4)-Isothiazolyl-5 |
| 219 | 3-(2'-CH3-C6H4)-Isothiazolyl-5 |
| 220 | 3-(4'-CH3O-C6H4)-Isothiazolyl-5 |
| 221 | 3-(3'-CH3O-$C_6H_4$)-Isothiazolyl-5 |
| 222 | 3-(2'-$CH_3O$-$C_6H_4$)-Isothiazolyl-5 |
| 223 | 3-(4'-$NO_2$-$C_6H_4$)-Isothiazolyl-5 |
| 224 | 3-(3'-$NO_2$-$C_6H_4$)-Isothiazolyl-5 |
| 225 | 3-(2'-$NO_2$-$C_6H_4$)-Isothiazolyl-5 |
| 226 | 3-(4'-CN-$C_6H_4$)-Isothiazolyl-5 |
| 227 | 3-(3'-CN-$C_6H_4$)-Isothiazolyl-5 |

| Nummer | B |
|---|---|
| 228 | 3-(2'-CN-$C_6H_4$)-Isothiazolyl-5 |
| 229 | 3-(4'-Cl-$C_6H_4$)-Isothiazolyl-5 |
| 230 | 3-(3'-Cl-$C_6H_4$)-Isothiazolyl-5 |
| 231 | 3-(2'-Cl-$C_6H_4$)-Isothiazolyl-5 |
| 232 | Oxazolyl-4 |
| 233 | 2-$CH_3$-Oxazolyl-4 |
| 234 | 2-$C_6H_5$-Oxazolyl-4 |
| 235 | 2-(4'-$CH_3$-$C_6H_4$)-Oxazolyl-4 |
| 236 | 2-(3'-$CH_3$-$C_6H_4$)-Oxazolyl-4 |
| 237 | 2-(2'-$CH_3$-$C_6H_4$)-Oxazolyl-4 |
| 238 | 2-(4'-$CH_3O$-$C_6H_4$)-Oxazolyl-4 |
| 239 | 2-(3'-$CH_3O$-$C_6H_4$)-Oxazolyl-4 |
| 240 | 2-(2'-$CH_3O$-$C_6H_4$)-Oxazolyl-4 |
| 241 | 2-(4'-$NO_2$-$C_6H_4$)-Oxazolyl-4 |
| 242 | 2-(3'-$NO_2$-$C_6H_4$)-Oxazolyl-4 |
| 243 | 2-(2'-$NO_2$-$C_6H_4$)-Oxazolyl-4 |
| 244 | 2-(4'-CN-$C_6H_4$)-Oxazolyl-4 |
| 245 | 2-(3'-CN-$C_6H_4$)-Oxazolyl-4 |
| 246 | 2-(2'-CN-$C_6H_4$)-Oxazolyl-4 |
| 247 | 2-(4'-Cl-$C_6H_4$)-Oxazolyl-4 |
| 248 | 2-(3'-Cl-$C_6H_4$)-Oxazolyl-4 |
| 249 | 2-(2'-Cl-$C_6H_4$)-Oxazolyl-4 |
| 250 | Thiazolyl-4 |
| 251 | 2-$CH_3$-Thiazolyl-4 |
| 252 | 2-$C_6H_5$-Thiazolyl |
| 253 | 2-(4'-$CH_3$-$C_6H_4$)-Thiazolyl-4 |
| 254 | 2-(3'-$CH_3$-$C_6H_4$)-Thiazolyl-4 |
| 255 | 2-(2'-$CH_3$-$C_6H_4$)-Thiazolyl-4 |
| 256 | 2-(4'-$CH_3O$-$C_6H_4$)-Thiazolyl-4 |
| 257 | 2-(3'-$CH_3O$-$C_6H_4$)-Thiazolyl-4 |
| 258 | 2-(2'-$CH_3O$-$C_6H_4$)-Thiazolyl-4 |
| 259 | 2-(4'-$NO_2$-$C_6H_4$)-Thiazolyl-4 |
| 260 | 2-(3'-NO2-C6H4)-Thiazolyl-4 |
| 261 | 2-(2'-$NO_2$-$C_6H_4$)-Thiazolyl-4 |
| 262 | 2-(4'-CN-$C_6H_4$)-Thiazolyl-4 |
| 263 | 2-(3'-CN-$C_6H_4$)-Thiazolyl-4 |

| Nummer | B |
|---|---|
| 264 | 2-(2'-CN-$C_6H_4$)-Thiazolyl-4 |
| 265 | 2-(4'-Cl-$C_6H_4$)-Thiazolyl-4 |
| 266 | 2-(3'-Cl-$C_6H_4$)-Thiazolyl-4 |
| 267 | 2-(2'-Cl-$C_6H_4$)-Thiazolyl-4 |
| 268 | N-$CH_3$-1,2,4-Triazolyl-5 |
| 269 | 3-$CH_3$-N-$CH_3$-1,2,4-Triazolyl-5 |
| 270 | 3-$C_6H_5$-N-$CH_3$-1,2,4-Triazolyl-5 |
| 271 | 3-(4'-$CH_3$-$C_6H_4$)-N-$CH_3$-1,2,4-Triazolyl-5 |
| 272 | 3-(3'-$CH_3$-$C_6H_4$)-N-$CH_3$-1,2,4-Triazolyl-5 |
| 273 | 3-(2'-$CH_3$-$C_6H_4$)-N-$CH_3$-1,2,4-Triazolyl-5 |
| 274 | 3-(4'-$CH_3O$-$C_6H_4$)-N-$CH_3$-1,2,4-Triazolyl-5 |
| 275 | 3-(3'-$CH_3O$-$C_6H_4$)-N-$CH_3$-1,2,4-Triazolyl-5 |
| 276 | 3-(2'-$CH_3O$-$C_6H_4$)-N-$CH_3$-1,2,4-Triazolyl-5 |
| 277 | 3-(4'-$NO_2$-$C_6H_4$)-N-$CH_3$-1,2,4-Triazolyl-5 |
| 278 | 3-(3'-$NO_2$-$C_6H_4$)-N-$CH_3$-1,2,4-Triazolyl-5 |
| 279 | 3-(2'-$NO_2$-$C_6H_4$)-N-$CH_3$-1,2,4-Triazolyl-5 |
| 280 | 3-(4'-CN-$C_6H_4$)-N-$CH_3$-1,2,4-Triazolyl-5 |
| 281 | 3-(3'-CN-$C_6H_4$)-N-$CH_3$-1,2,4-Triazolyl-5 |
| 282 | 3-(2'-CN-$C_6H_4$)-N-$CH_3$-1,2,4-Triazolyl-5 |
| 283 | 3-(4'-Cl-$C_6H_4$)-N-$CH_3$-1,2,4-Triazolyl-5 |
| 284 | 3-(3'-Cl-$C_6H_4$)-N-$CH_3$-1,2,4-Triazolyl-5 |
| 285 | 3-(2'-Cl-$C_6H_4$)-N-$CH_3$-1,2,4-Triazolyl-5 |
| 286 | 1,3,4-Oxadiazolyl-2 |
| 287 | 5-$CH_3$-1,3,4-Oxadiazolyl-2 |
| 288 | 5-$C_6H_5$-1,3,4-Oxadiazolyl-2 |
| 289 | 5-(4'-$CH_3$-$C_6H_4$)-1,3,4-Oxadiazolyl-2 |
| 290 | 5-(3'-$CH_3$-$C_6H_4$)-1,3,4-Oxadiazolyl-2 |
| 291 | 5-(2'-$CH_3$-$C_6H_4$)-1,3,4-Oxadiazolyl-2 |
| 292 | 5-(4'-$CH_3O$-$C_6H_4$)-1,3,4-Oxadiazolyl-2 |
| 293 | 5-(3'-$CH_3O$-$C_6H_4$)-1,3,4-Oxadiazolyl-2 |
| 294 | 5-(2'-$CH_3O$-$C_6H_4$)-1,3,4-Oxadiazolyl-2 |
| 295 | 5-(4'-$NO_2$-$C_6H_4$)-1,3,4-Oxadiazolyl-2 |
| 296 | 5-(3'-$NO_2$-$C_6H_4$)-1,3,4-Oxadiazolyl-2 |
| 297 | 5-(2'-$NO_2$-$C_6H_4$)-1,3,4-Oxadiazolyl-2 |
| 298 | 5-(4'-CN-$C_6H_4$)-1,3,4-Oxadiazolyl-2 |
| 299 | 5-(3'-CN-$C_6H_4$)-1,3,4-Oxadiazolyl-2 |

| Nummer | B |
|---|---|
| 300 | 5-(2'-CN-$C_6H_4$)-1,3,4-Oxadiazolyl-2 |
| 301 | 5-(4'-Cl-$C_6H_4$)-1,3,4-Oxadiazolyl-2 |
| 302 | 5-(3'-Cl-$C_6H_4$)-1,3,4-Oxadiazolyl-2 |
| 303 | 5-(2'-Cl-C6H4)-1,3,4-Oxadiazolyl-2 |
| 304 | 1,2,4-Oxadiazolyl-3 |
| 305 | 5-$CH_3$-1,2,4-Oxadiazolyl-3 |
| 306 | 5-$C_6H_5$-1,2,4-Oxadiazolyl-3 |
| 307 | 5-(4'-$CH_3$-$C_6H_4$)-1,2,4-Oxadiazolyl-3 |
| 308 | 5-(3'-$CH_3$-$C_6H_4$)-1,2,4-Oxadiazolyl-3 |
| 309 | 5-(2'-$CH_3$-$C_6H_4$)-1,2,4-Oxadiazolyl-3 |
| 310 | 5-(4'-$CH_3O$-$C_6H_4$)-1,2,4-Oxadiazolyl-3 |
| 311 | 5-(3'-$CH_3O$-$C_6H_4$)-1,2,4-Oxadiazolyl-3 |
| 312 | 5-(2'-$CH_3O$-$C_6H_4$)-1,2,4-Oxadiazolyl-3 |
| 313 | 5-(4'-$NO_2$-$C_6H_4$)-1,2,4-Oxadiazolyl-3 |
| 314 | 5-(3'-$NO_2$-$C_6H_4$)-1,2,4-Oxadiazolyl-3 |
| 315 | 5-(2'-$NO_2$-$C_6H_4$)-1,2,4-Oxadiazolyl-3 |
| 316 | 5-(4'-CN-$C_6H_4$)-1,2,4-Oxadiazolyl-3 |
| 317 | 5-(3'-CN-$C_6H_4$)-1,2,4-Oxadiazolyl-3 |
| 318 | 5-(2'-CN-$C_6H_4$)-1,2,4-Oxadiazolyl-3 |
| 319 | 5-(4'-Cl-$C_6H_4$)-1,2,4-Oxadiazolyl-3 |
| 320 | 5-(3'-Cl-$C_6H_4$)-1,2,4-Oxadiazolyl-3 |
| 321 | 5-(2'-Cl-$C_6H_4$)-1,2,4-Oxadiazolyl-3 |
| 322 | 1,2,4-Oxadiazolyl-5 |
| 323 | 3-$CH_3$-1,2,4-Oxadiazolyl-5 |
| 324 | 3-$C_6H_5$-1,2,4-Oxadiazolyl-5 |
| 325 | 3-(4'-$CH_3$-$C_6H_4$)-1,2,4-Oxadiazolyl-5 |
| 326 | 3-(3'-$CH_3$-$C_6H_4$)-1,2,4-Oxadiazolyl-5 |
| 327 | 3-(2'-$CH_3$-$C_6H_4$)-1,2,4-Oxadiazolyl-5 |
| 328 | 3-(4'-$CH_3O$-$C_6H_4$)-1,2,4-Oxadiazolyl-5 |
| 329 | 3-(3'-$CH_3O$-$C_6H_4$)-1,2,4-Oxadiazolyl-5 |
| 330 | 3-(2'-$CH_3O$-$C_6H_4$)-1,2,4-Oxadiazolyl-5 |
| 331 | 3-(4'-$NO_2$-$C_6H_4$)-1,2,4-Oxadiazolyl-5 |
| 332 | 3-(3'-$NO_2$-$C_6H_4$)-1,2,4-Oxadiazolyl-5 |
| 333 | 3-(2'-$NO_2$-$C_6H_4$)-1,2,4-Oxadiazolyl-5 |
| 334 | 3-(4'-CN-$C_6H_4$)-1,2,4-Oxadiazolyl-5 |
| 335 | 3-(3'-CN-$C_6H_4$)-1,2,4-Oxadiazolyl-5 |

| Nummer | B |
|---|---|
| 336 | 3-(2'-CN-$C_6H_4$)-1,2,4-Oxadiazolyl-5 |
| 337 | 3-(4'-Cl-$C_6H_4$)-1,2,4-Oxadiazolyl-5 |
| 338 | 3-(3'-Cl-$C_6H_4$)-1,2,4-Oxadiazolyl-5 |
| 339 | 3-(2'-Cl-$C_6H_4$)-1,2,4-Oxadiazolyl-5 |
| 340 | 1,2,4-Thiadiazolyl-3 |
| 341 | 5-$CH_3$-1,2,4-Thiadiazolyl-3 |
| 342 | 5-$C_6H_5$-1,2,4-Thiadiazolyl-3 |
| 343 | 5-(4'-$CH_3$-$C_6H_4$)-1,2,4-Thiadiazolyl-3 |
| 344 | 5-(3'-$CH_3$-$C_6H_4$)-1,2,4-Thiadiazolyl-3 |
| 345 | 5-(2'-$CH_3$-$C_6H_4$)-1,2,4-Thiadiazolyl-3 |
| 346 | 5-(4'-$CH_3O$-$C_6H_4$)-1,2,4-Thiadiazolyl-3 |
| 347 | 5-(3'-$CH_3O$-$C_6H_4$)-1,2,4-Thiadiazolyl-3 |
| 348 | 5-(2'-$CH_3O$-$C_6H_4$)-1,2,4-Thiadiazolyl-3 |
| 349 | 5-(4'-$NO_2$-$C_6H_4$)-1,2,4-Thiadiazolyl-3 |
| 350 | 5-(3'-$NO_2$-$C_6H_4$)-1,2,4-Thiadiazolyl-3 |
| 351 | 5-(2'-$NO_2$-$C_6H_4$)-1,2,4-Thiadiazolyl-3 |
| 352 | 5-(4'-CN-$C_6H_4$)-1,2,4-Thiadiazolyl-3 |
| 353 | 5-(3'-CN-$C_6H_4$)-1,2,4-Thiadiazolyl-3 |
| 354 | 5-(2'-CN-$C_6H_4$)-1,2,4-Thiadiazolyl-3 |
| 355 | 5-(4'-Cl-$C_6H_4$)-1,2,4-Thiadiazolyl-3 |
| 356 | 5-(3'-Cl-$C_6H_4$)-1,2,4-Thiadiazolyl-3 |
| 357 | 5-(2'-Cl-$C_6H_4$)-1,2,4-Thiadiazolyl-3 |
| 358 | 1,3,4-Thiadiazolyl-2 |
| 359 | 5-$CH_3$-1,3,4-Thiadiazolyl-2 |
| 360 | 5-$C_6H_5$-1,3,4-Thiadiazolyl-2 |
| 361 | 5-(4'-$CH_3$-$C_6H_4$)-1,3,4-Thiadiazolyl-2 |
| 362 | 5-(3'-$CH_3$-$C_6H_4$)-1,3,4-Thiadiazolyl-2 |
| 363 | 5-(2'-$CH_3$-$C_6H_4$)-1,3,4-Thiadiazolyl-2 |
| 364 | 5-(4'-$CH_3O$-$C_6H_4$)-1,3,4-Thiadiazolyl-2 |
| 365 | 5-(3'-$CH_3O$-$C_6H_4$)-1,3,4-Thiadiazolyl-2 |
| 366 | 5-(2'-$CH_3O$-$C_6H_4$)-1,3,4-Thiadiazolyl-2 |
| 367 | 5-(4'-$NO_2$-$C_6H_4$)-1,3,4-Thiadiazolyl-2 |
| 368 | 5-(3'-$NO_2$-$C_6H_4$)-1,3,4-Thiadiazolyl-2 |
| 369 | 5-(2'-$NO_2$-$C_6H_4$)-1,3,4-Thiadiazolyl-2 |
| 370 | 5-(4'-CN-$C_{6H4}$)-1,3,4-Thiadiazolyl-2 |
| 371 | 5-(3'-CN-$C_6H_4$)-1,3,4-Thiadiazolyl-2 |

| Nummer | B |
|---|---|
| 372 | 5-(2'-CN-$C_6H_4$)-1,3,4-Thiadiazolyl-2 |
| 373 | 5-(4'-Cl-$C_6H_4$)-1,3,4-Thiadiazolyl-2 |
| 374 | 5-(3'-Cl-$C_6H_4$)-1,3,4-Thiadiazolyl-2 |
| 375 | 5-(2'-Cl-$C_6H_4$)-1,3,4-Thiadiazolyl-2 |
| 376 | Pyridinyl-2 |
| 377 | Pyridinyl-4 |
| 378 | Pyridazinyl-3 |
| 379 | Pyridazinyl-4 |
| 380 | Pyridinyl-3 |
| 381 | Pyrimidinyl-4 |
| 382 | Pyrimidinyl-5 |
| 383 | Pyrimidinyl-2 |

Tabelle 3: Ausgewählte physikalische Daten einiger Verbindungen
Isomer A: unpolares Isomeres
Isomer B: polares Isomeres

| Nr. | Verbindung | IR ($cm^{-1}$) oder $^1H$-NMR (ppm) | Fp (° C) |
|---|---|---|---|
| 1 | 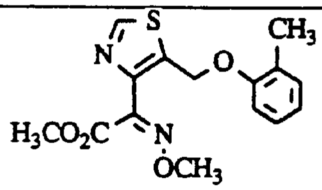 | | 90 |
| 2 | 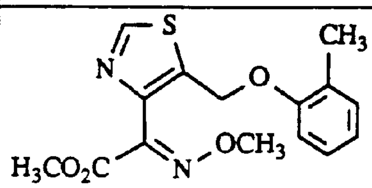 | | 95 |
| 3 | 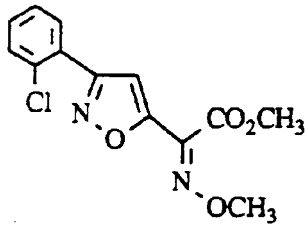 | | 67 |
| 4 | 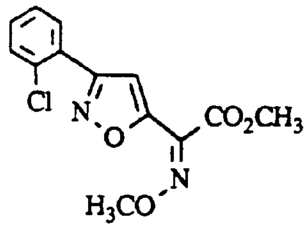 | | 98 |

Tabelle 3: Fortsetzung

| Nr. | Verbindung | IR ($cm^{-1}$) oder $^1H$-NMR (ppm) | Fp (° C) |
|---|---|---|---|
| 5 | 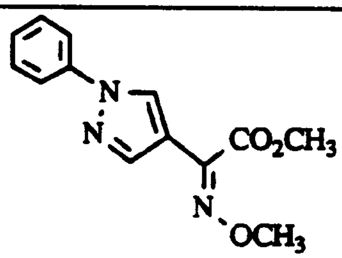 | | 71 |
| 6 | 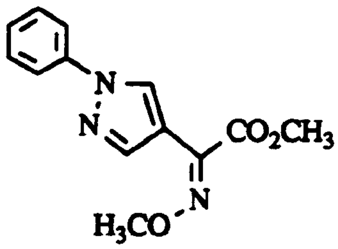 | | 45 |
| 7 | 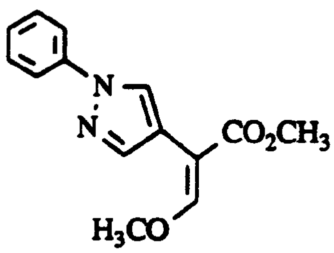 | 7,55 (s, 1 H); 4,0 (s, 3 H); 3,85 (s, 3 H) | |
| 8 | 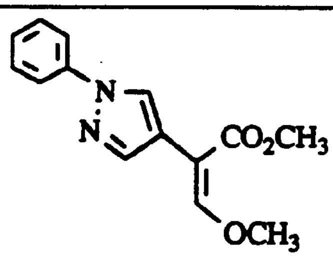 | 6,9 (s, 1 H); 3,95 (s, 3 H); 3,85 (s, 3 H) | |
| 9 | 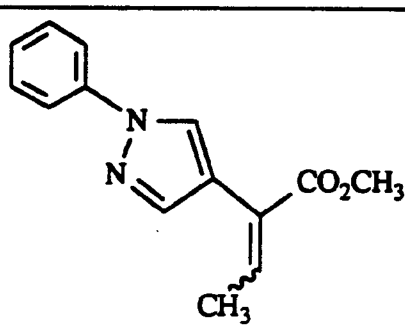 | trans-Isomeres: 7,15 (q, 1 H, J = 7 Hz); 3,85 (s, 3 H); 3,8 (s, 3 H) cis-Isomeres: 6,5 (q, 1 H, J = 7 Hz); 3,85 (s, 3 H); 3,8 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 10 | 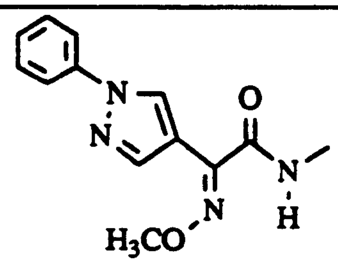 | | 97 |
| 11 | 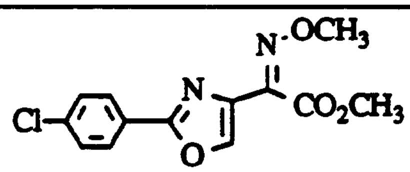 | | 94 |
| 12 | 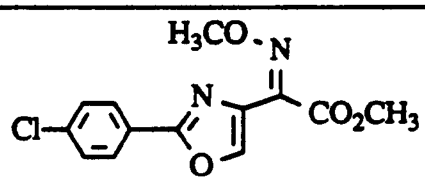 | | 76 |
| 13 | 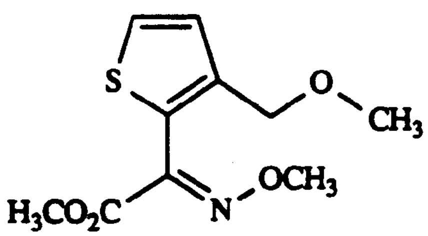 | 4,1 (s, 3 H); 3,9 (s, 3 H) | |
| 14 | 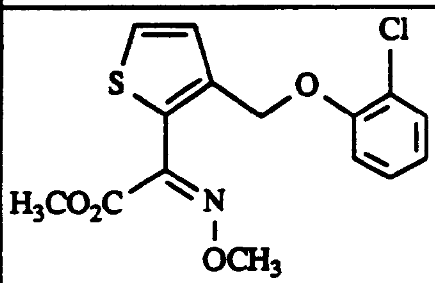 | 4,12 (s, 3 H); 3,86 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 15 | 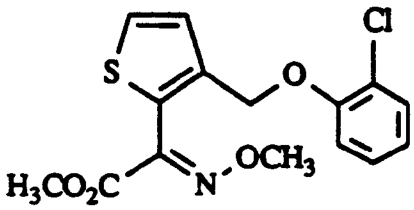 | 4,02 (s, 3 H); 3,90 (s, 3 H) | |
| 16 | 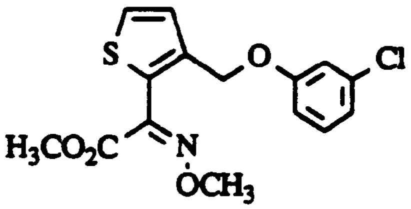 | 4,1 (s, 3 H); 3,79 (s, 3 H) | |
| 17 | 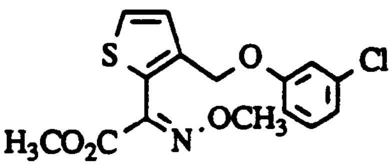 | 4,01 (s, 3 H); 3,86 (s, 3 H) | |
| 18 | 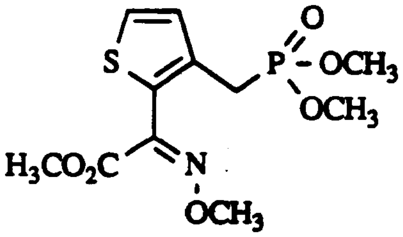 | | 75° C |
| 19 | 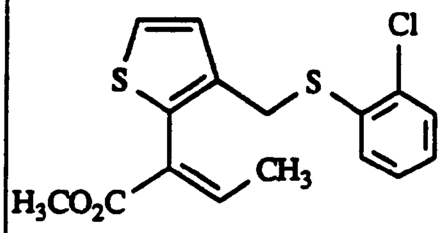 | 1717, 1451, 1432, 1257, 1237, 1196, 1175, 1035, 747, 730 | |

Tabelle 3: Fortsetzung

| Nr. | Verbindung | IR ($cm^{-1}$) oder $^1H$-NMR (ppm) | Fp (° C) |
| --- | --- | --- | --- |
| 20 | 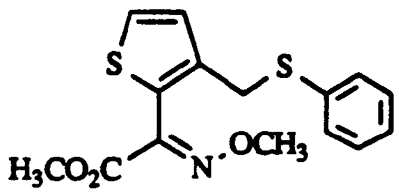 | 4,03 (s, 3 H); 3,84 (s 3 H) | |
| 21 | 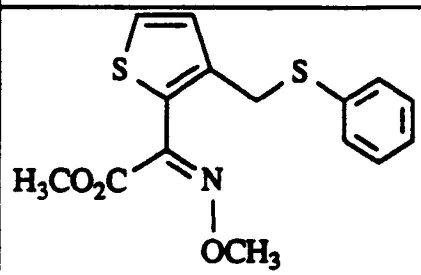 | 3,79 (s 3 H); 3,87 (s 3 H) | |
| 22 | 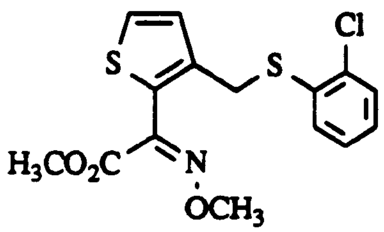 | 4,01 (s 3 H); 3,90 (s 3 H) | |
| 23 | 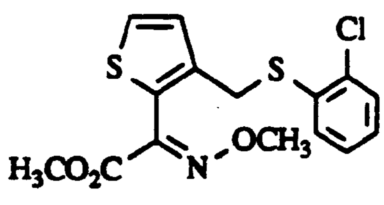 | 4,12 (s, 3 H); 3,91 (s, 3 H) | |
| 24 | 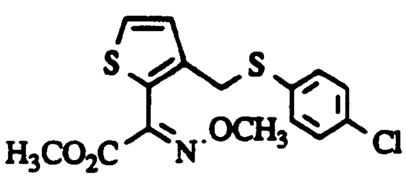 | 4,10 (s, 3 H); 3,87 (s, 3 H) | |

Tabelle 3: Fortsetzung

| Nr. | Verbindung | IR ($cm^{-1}$) oder $^1$H-NMR (ppm) | Fp (° C) |
|---|---|---|---|
| 25 | 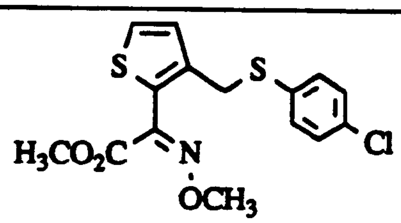 | 3,99 (s, 3 H); 3,89 (s, 3 H) | |
| 26 | 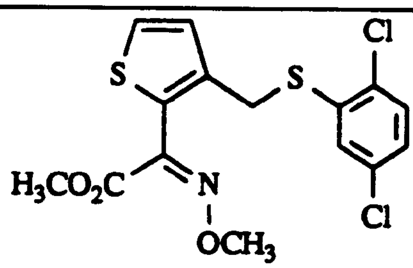 | 4,02 (s, 3 H); 3,92 (s, 3 H) | |
| 27 | 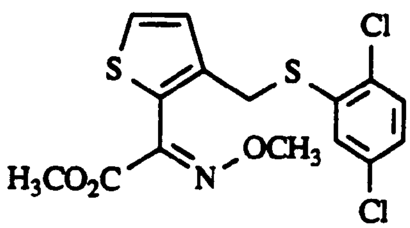 | 4,14 (s, 3 H); 3,91 (s, 3 H) | |
| 28 | 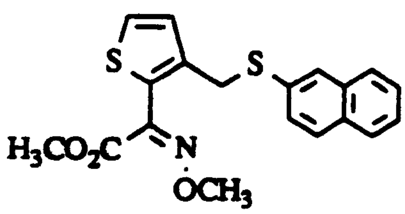 | 3,99 (s, 3 H); 3,88 (s, 3 H) | |
| 29 | 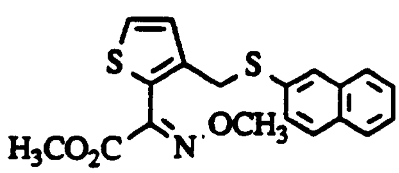 | 4,04 (s, 3 H); 3,76 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 30 | 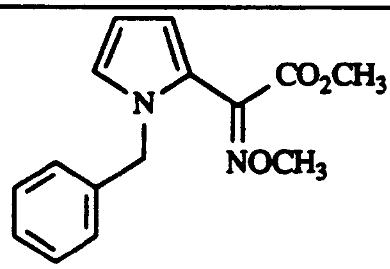 | Isomer A:<br>3,88 (s, 3 H);<br>3,82 (s, 3 H)<br><br>Isomer B:<br>4,01 (s, 3 H);<br>3,77 (s, 3 H) | |
| 31 | 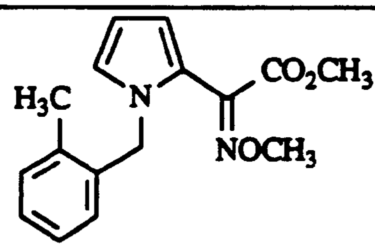 | 1733, 1461,<br>1438, 1319,<br>1286, 1221,<br>1072, 1035<br>776, 742, | |
| 32 | 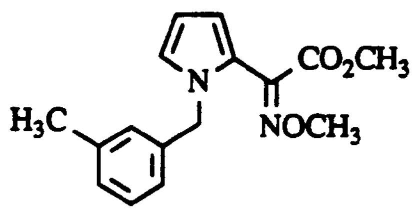 | 1733, 1464,<br>1438, 1319,<br>1285, 1214,<br>1072, 1035,<br>776, 729 | |
| 33 | 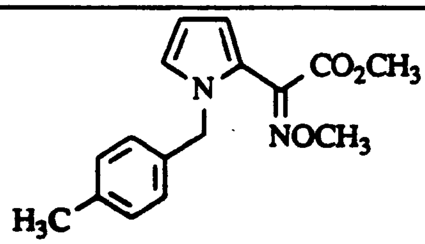 | 1733, 1438,<br>1322, 1285,<br>1217, 1072,<br>1035, 1005,<br>776, 726 | |
| 34 | 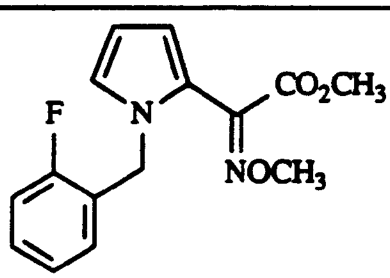 | 1732, 1492,<br>1458, 1438,<br>1285, 1230,<br>1215, 1071,<br>1034, 758 | |

Tabelle 3: Fortsetzung

| Nr. | Verbindung | IR ($cm^{-1}$) oder $^{1}H$-NMR (ppm) | Fp (° C) |
|---|---|---|---|
| 35 | 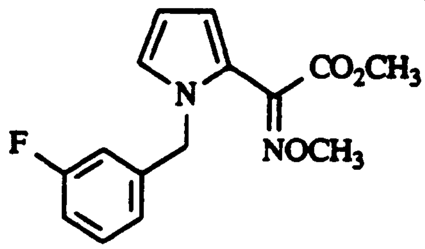 | 1733, 1591, 1489, 1452, 1438, 1286, 1216, 1072, 1035, 777 | |
| 36 | 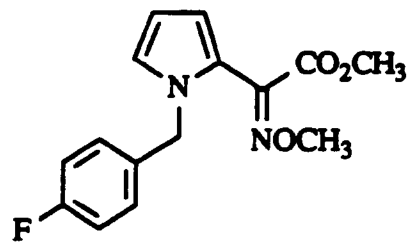 | 1733, 1510, 1439, 1321, 1285, 1223, 1159, 1072, 1035, 727 | |
| 37 | 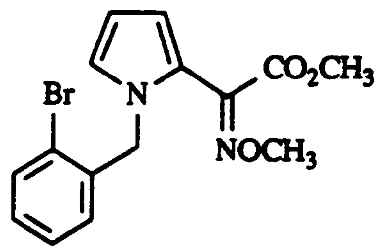 | 1731, 1439, 1320, 1287, 1218, 1071, 1035, 776, 747, 727 | |
| 38 | 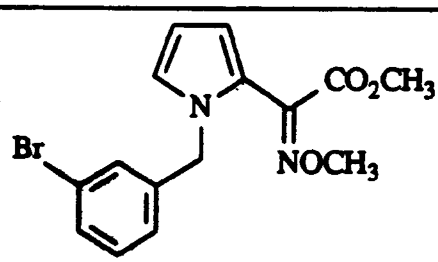 | 1733, 1437, 1429, 1318, 1285, 1217, 1071, 1035, 776, 727 | |
| 39 | 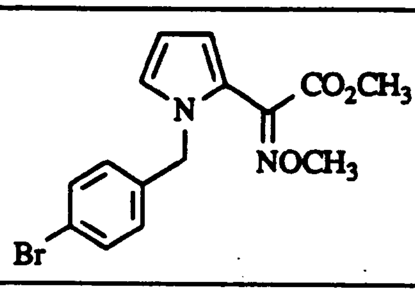 | 1731, 1488, 1437, 1285, 1217, 1071, 1035, 1011, 726 | |

Tabelle 3: Fortsetzung

| Nr. | Verbindung | IR ($cm^{-1}$) oder $^1H$-NMR (ppm) | Fp (° C) |
|---|---|---|---|
| 40 | 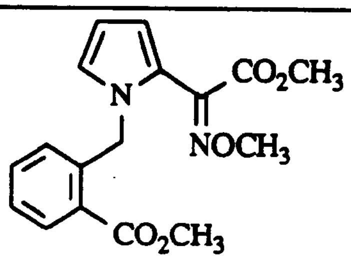 | 1723, 1436, 1285, 1215, 1201, 1072, 1035, 736 | |
| 41 | 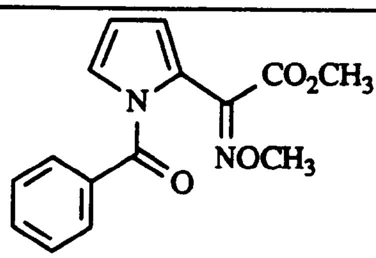 | 4,01 (s, 3 H); 3,77 (s, 3 H) | |
| 42 | 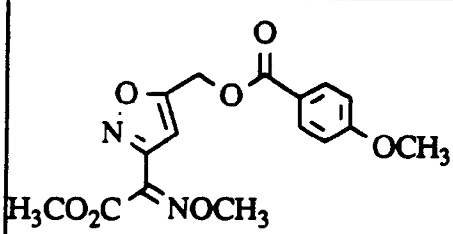 | Isomer A: 4,06 (s, 3 H); 3,96 (s, 3 H); 3,87 (s, 3 H) Isomer B: 4,17 (s, 3 H); 3,92 (s, 3 H); 3,86 (s, 3 H) | |
| 43 | 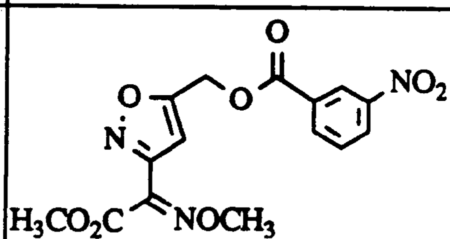 | Isomer A: 4,07 (s, 3 H); 3,96 (s, 3 H) Isomer B: 4,20 (s, 3 H); 3,94 (s, 3 H) | |
| 44 | 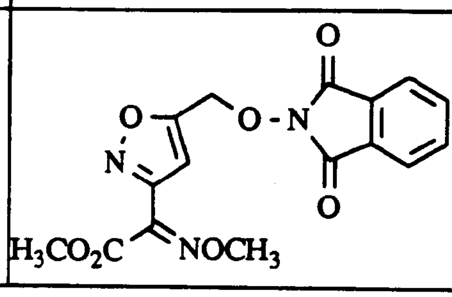 | 4,17 (s, 3 H); 3,92 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 45 | 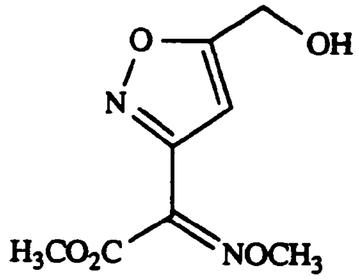 | 4,16 (s, 3 H); 3,92 (s, 3 H) | |
| 46 | 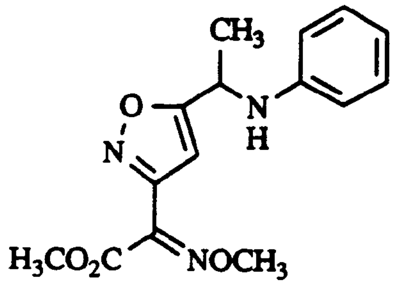 | 4,12 (s, 3 H); 3,91 (s, 3 H) | |
| 47 | 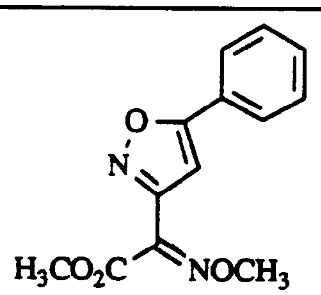 | 4,21 (s, 3 H); 3,96 (s, 3 H) | |
| 48 | 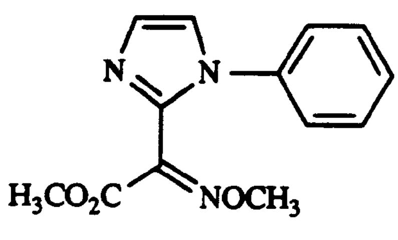 | Isomer A: 3,85 (s, 3 H); 3,65 (s, 3 H) Isomer B: | 49 |
| 49 | 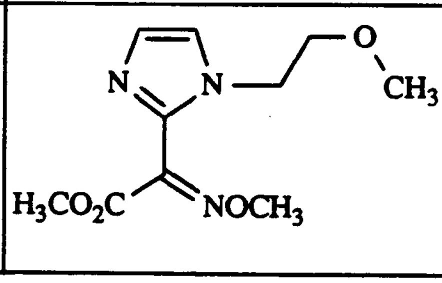 | Isomer A: 4,0 (s, 3 H); 3,95 (s, 3 H) Isomer B: | 56 |

Tabelle 3: Fortsetzung

| Nr. | Verbindung | IR ($cm^{-1}$) oder $^1H$-NMR (ppm) | Fp (° C) |
|---|---|---|---|
| 50 | 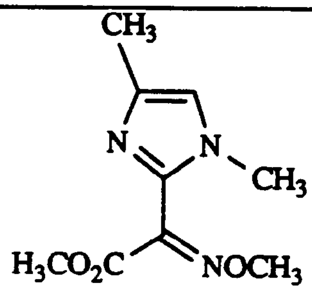 | | 72 |
| 51 | 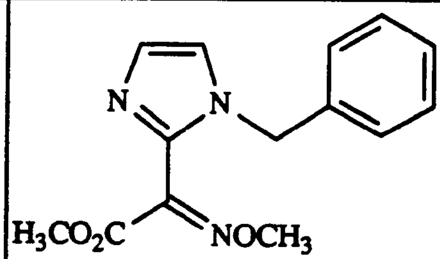 | Isomer A: 3,95 (s, 3 H); 3,95 (s, 3 H) Isomer B: | 58 |
| 52 | 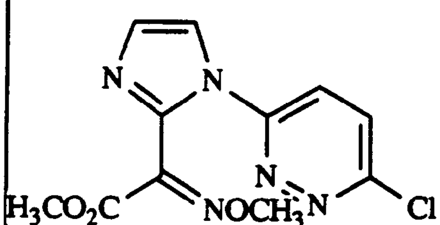 | Isomer A: Isomer B: | 136 113 |
| 53 | 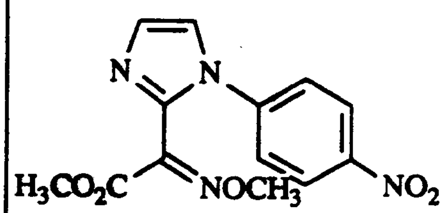 | Isomer A: Isomer B: | 150 112 |
| 54 | 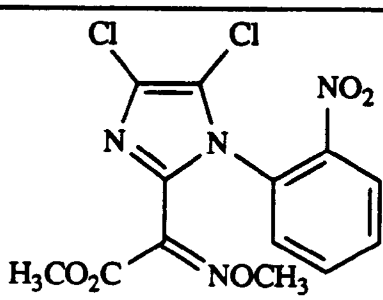 | Isomer A: Isomer B: | 138 112 |

Tabelle 3: Fortsetzung

| Nr. | Verbindung | IR ($cm^{-1}$) oder $^1H$-NMR (ppm) | Fp (° C) |
|---|---|---|---|
| 55 | 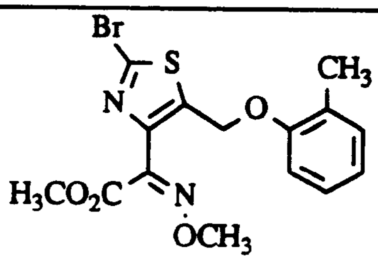 | | 110 |
| 56 | 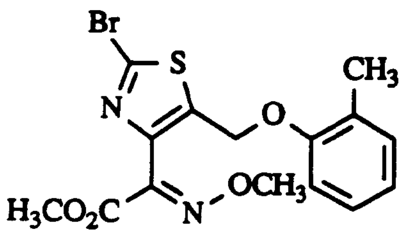 | | 84 |
| 57 | 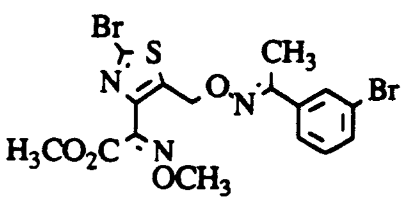 | 4,05 (s, 3 H); 3,95 (s, 3 H) | |
| 58 | 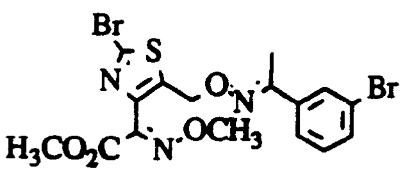 | 4,15 (s, 3 H); 3,85 (s, 3 H) | |
| 59 | 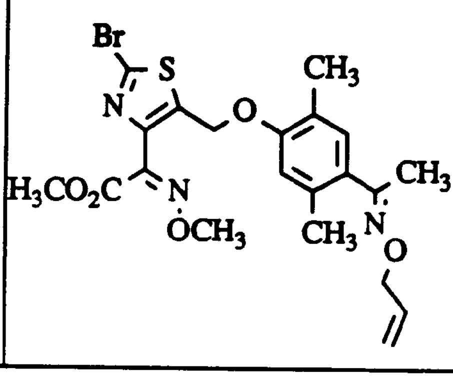 | | 106 |

Tabelle 3: Fortsetzung

| Nr. | Verbindung | IR ($cm^{-1}$) oder $^1H$-NMR (ppm) | Fp (° C) |
|---|---|---|---|
| 60 | 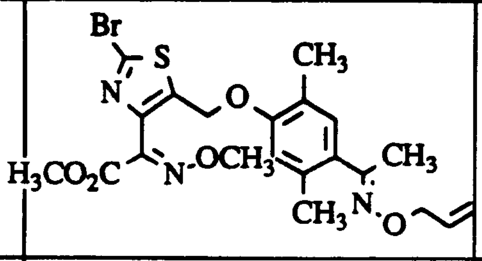 | 4,1 (s, 3 H); 3,9 (s, 3 H) | |
| 61 | 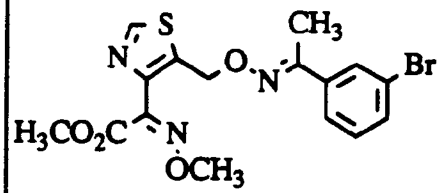 | 4,1 (s, 3 H); 4,0 (s, 3 H) | |
| 62 | 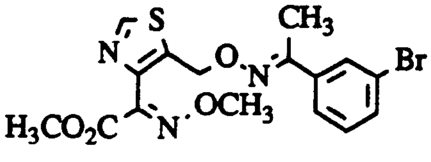 | 4,1 (s, 3 H); 3,9 (s, 3 H) | |
| 63 | 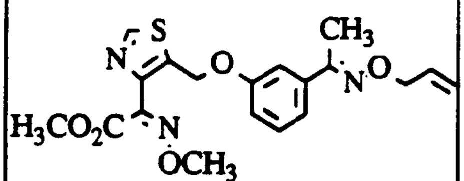 | | 81 |
| 64 | 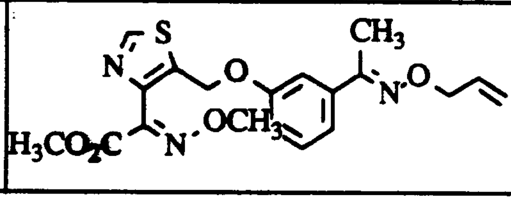 | | 85 |

Tabelle 3: Fortsetzung

| Nr. | Verbindung | IR ($cm^{-1}$) oder $^1H$-NMR (ppm) | Fp (° C) |
|---|---|---|---|
| 65 | 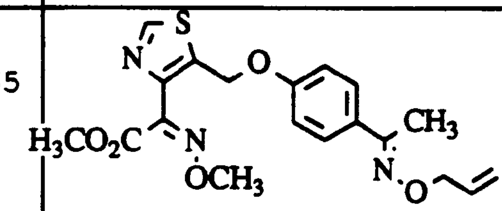 | | 85 |
| 66 | 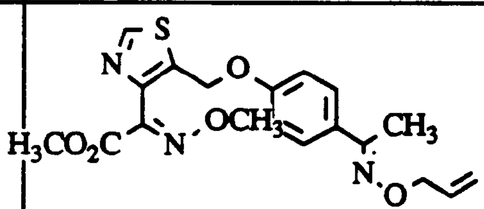 | 4,1 (s, 3 H); 3,9 (s, 3 H) | |
| 67 | 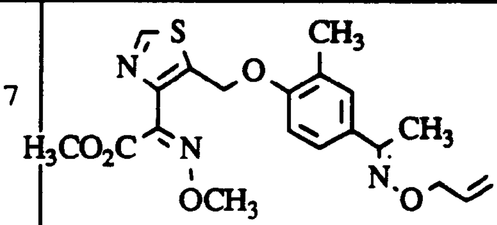 | | 64 |
| 68 | 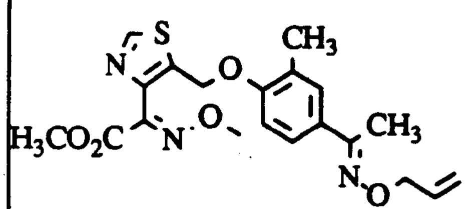 | 4,1 (s, 3 H); 3,9 (s, 3 H) | |
| 69 | 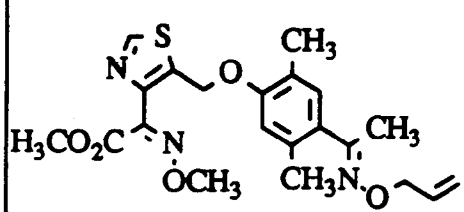 | 4,05 (s, 3 H); 3,95 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 70 | 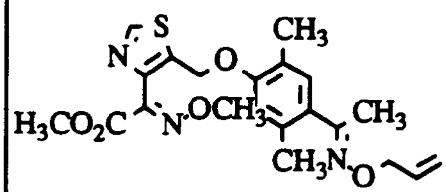 | 4,1 (s, 3 H); 3,9 (s, 3 H) | |
| 71 | 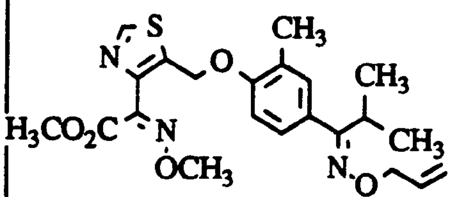 | 4,05 (s, 3 H); 3,95 (s, 3 H) | |
| 72 | 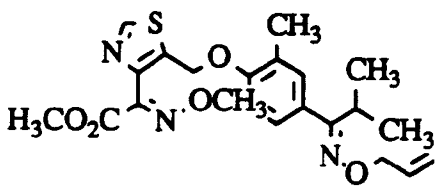 | 4,1 (s, 3 H); 3,9 (s, 3 H) | |
| 73 | 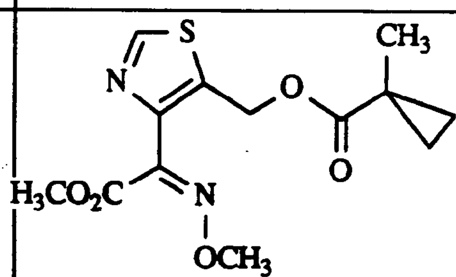 | | 61 |
| 74 | 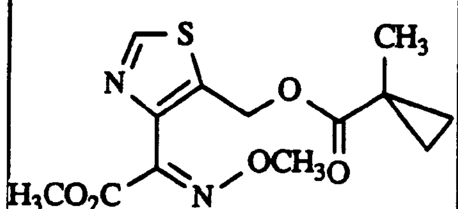 | | 74 |

Tabelle 3: Fortsetzung

| Nr. | Verbindung | IR ($cm^{-1}$) oder $^{1}H$-NMR (ppm) | Fp (° C) |
|---|---|---|---|
| 75 | 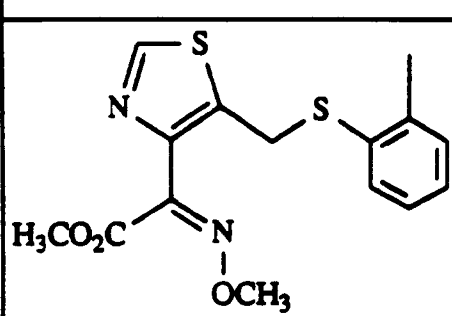 | | 65 |
| 76 | 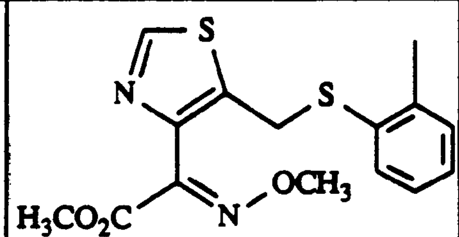 | | 73 |
| 77 | 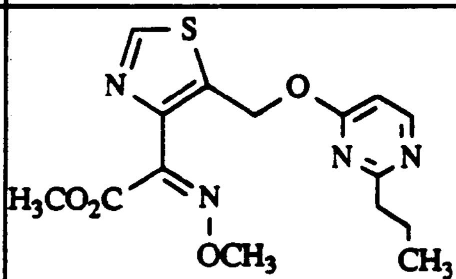 | 4,05 (s, 3 H); 3,95 (s, 3 H) | |
| 78 | 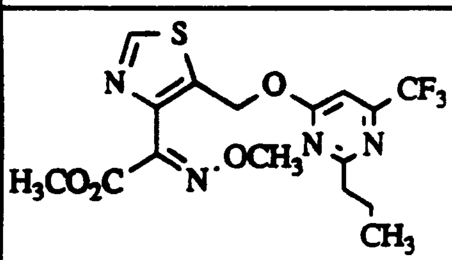 | | 59 |
| 79 | 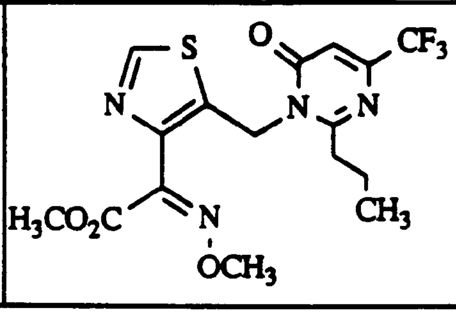 | | 107 |

Tabelle 3: Fortsetzung

| Nr. | Verbindung | IR ($cm^{-1}$) oder $^1H$-NMR (ppm) | Fp (° C) |
|---|---|---|---|
| 80 | 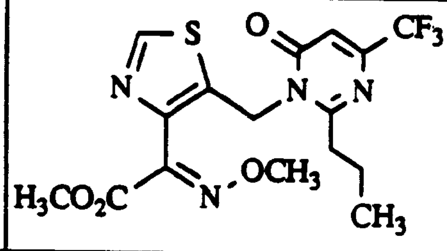 | | 94 |
| 81 | 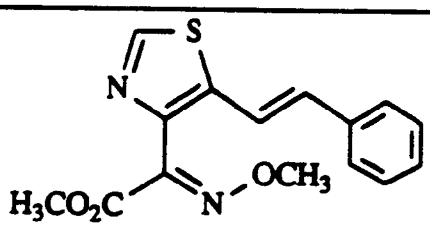 | | 108 |
| 82 | 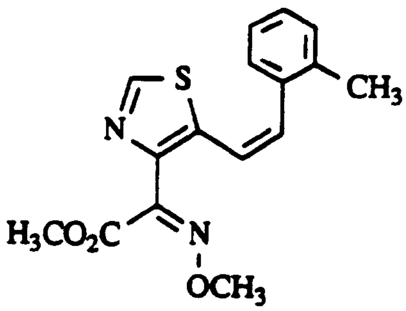 | | 67 |
| 83 | 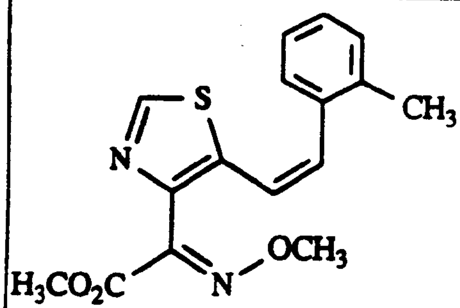 | 4,1 (s, 3 H); 3,9 (s, 3 H) | |
| 84 | 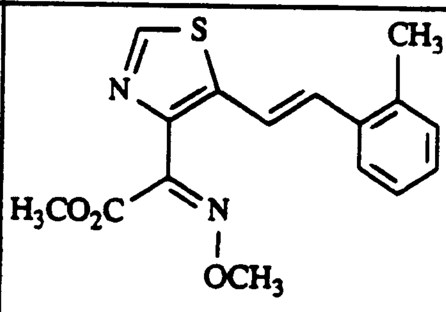 | | 86 |

Tabelle 3: Fortsetzung

| Nr. | Verbindung | IR ($cm^{-1}$) oder $^1H$-NMR (ppm) | Fp (° C) |
| --- | --- | --- | --- |
| 85 | 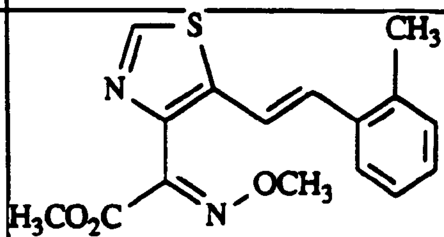 | | 94 |
| 86 | 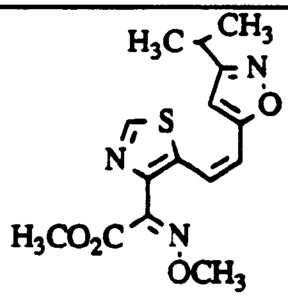 | 4,05 (s, 3 H); 3,95 (s, 3 H) | |
| 87 | 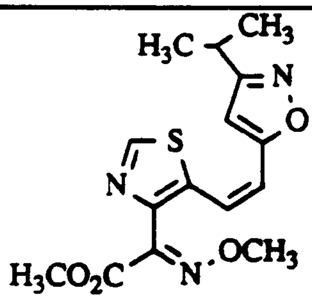 | 4,1 (s, 3 H); 3,9 (s, 3 H) | |
| 88 | 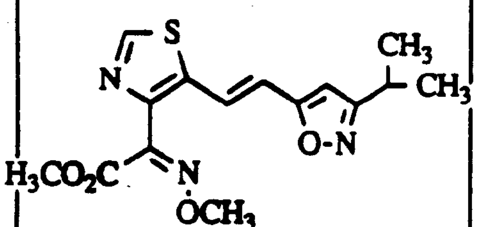 | | 87 |
| 89 | 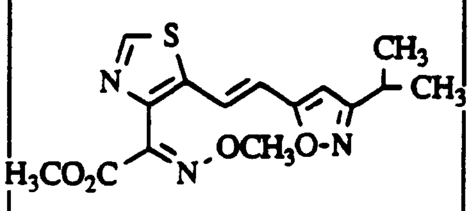 | | 139 |

Tabelle 3: Fortsetzung

| Nr. | Verbindung | IR ($cm^{-1}$) oder $^1$H-NMR (ppm) | Fp (° C) |
|---|---|---|---|
| 90 | 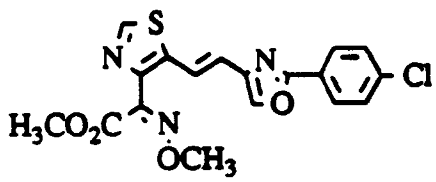 | | 156 |
| 91 | 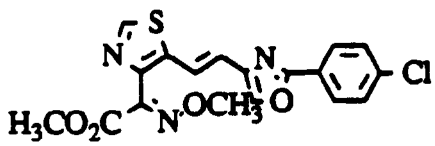 | | 169 |
| 92 | 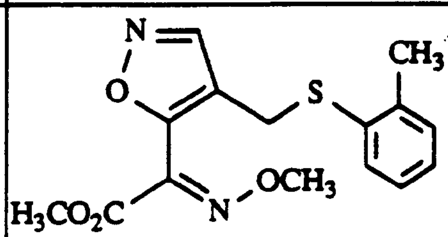 | | 82 |
| 93 | 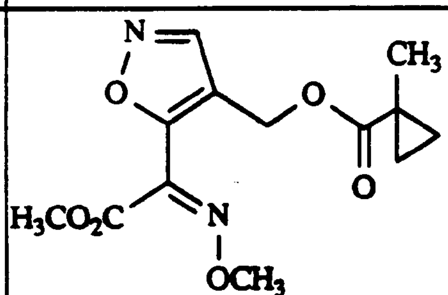 | 4,1 (s, 3 H); 3,95 (s, 3 H) | |
| 94 | 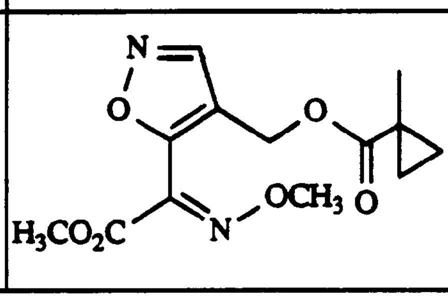 | 4,2 (s, 3 H); 3,95 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 95 | 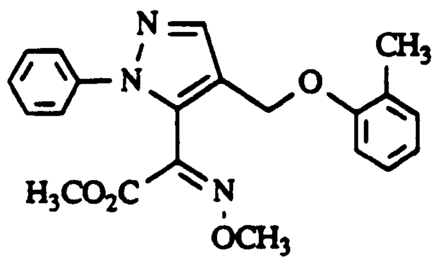 | | 77 |
| 96 | 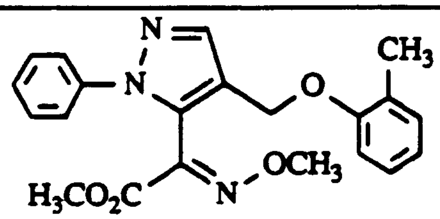 | | 87 |
| 97 | 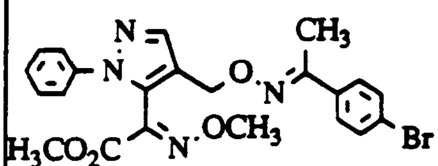 | 4,0 (s, 3 H); 3,55 (s, 3 H) | |
| 98 | 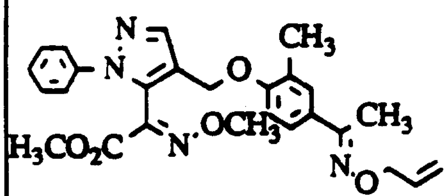 | | 98 |
| 99 | 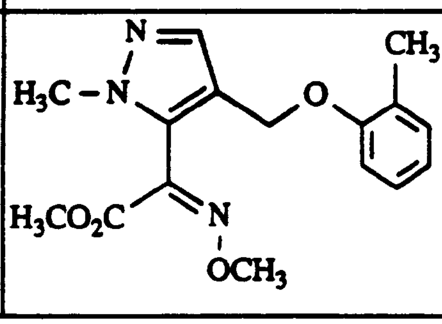 | 4,05 (s, 3 H); 4,0 (s, 3 H); 3,7 (s, 3 H) | |

Tabelle 3: Fortsetzung

| Nr. | Verbindung | IR ($cm^{-1}$) oder $^1$H-NMR (ppm) | Fp (° C) |
|---|---|---|---|
| 100 | 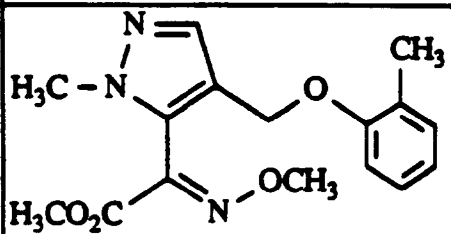 | 4,05 (s, 3 H); 3,8 (s, 3 H); 3,75 (s, 3 H) | |
| 101 | 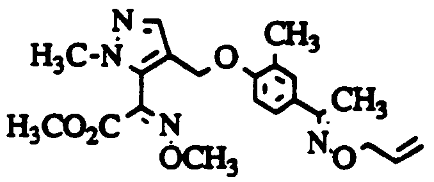 | 4,05 (s, 3 H); 3,8 (s, 3 H); 3,7 (s, 3 H) | |
| 102 | 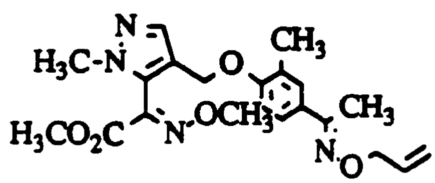 | 4,1 (s, 3 H); 3,85 (s, 3 H); 3,75 (s, 3 H) | |
| 103 | 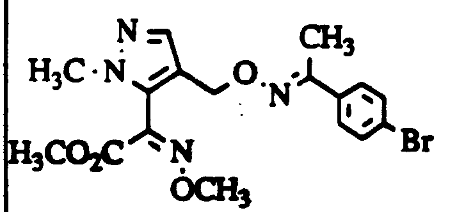 | 4,05 (s, 3 H); 3,95 (s, 3 H); 3,8 (s, 3 H) | |
| 104 | 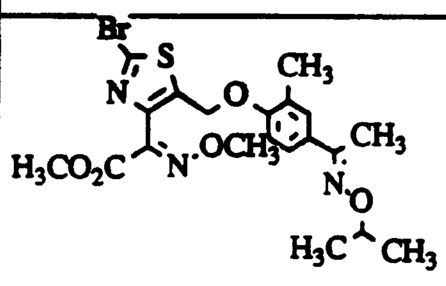 | | 129 |

Tabelle 3: Fortsetzung

| Nr. | Verbindung | IR ($cm^{-1}$) oder $^1H$-NMR (ppm) | Fp (° C) |
|---|---|---|---|
| 105 | 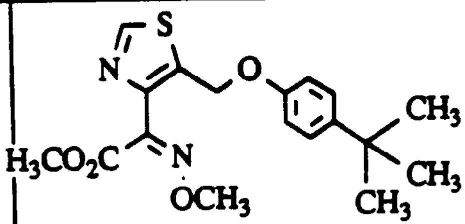 | 4,05 (s, 3 H); 3,95 (s, 3 H) | |
| 106 | 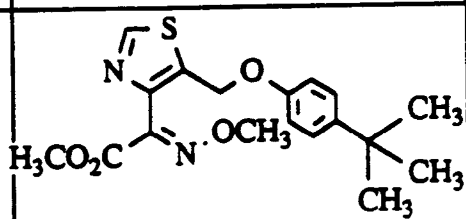 | 4,1 (s, 3 H); 3,9 (s, 3 H) | |
| 107 | 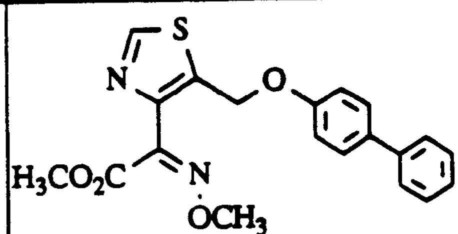 | | 116 |
| 108 | 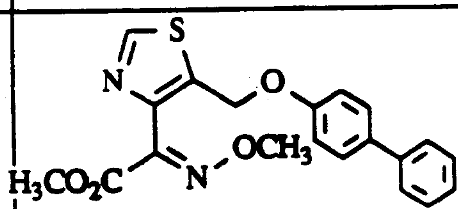 | | 98 |
| 109 | 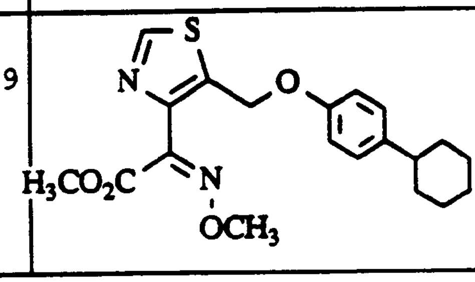 | | 136 |

Tabelle 3: Fortsetzung

| Nr. | Verbindung | IR ($cm^{-1}$) oder $^{1}H$-NMR (ppm) | Fp (° C) |
|---|---|---|---|
| 110 | 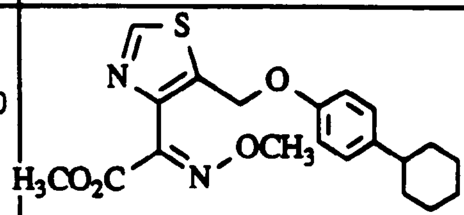 | | 82 |
| 111 | 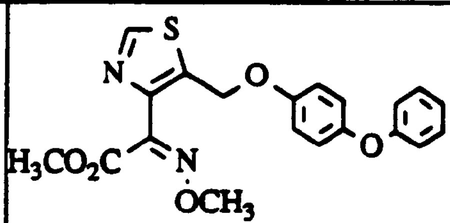 | | 79 |
| 112 | 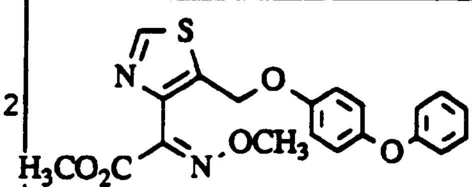 | 4,1 (s, 3 H); 3,9 (s, 3 H) | |
| 113 | 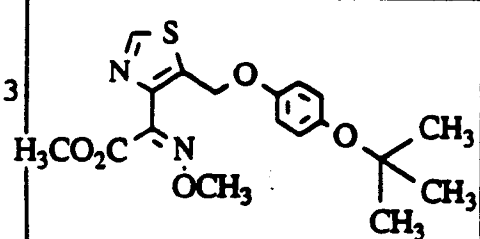 | 4,05 (s, 3 H); 3,9 (s, 3 H) | |
| 114 | 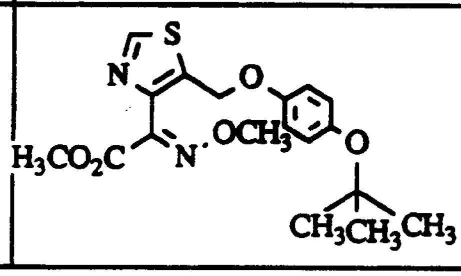 | 4,1 (s, 3 H); 3,9 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 115 | 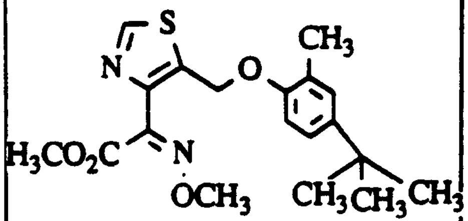 | | 85 |
| 116 | 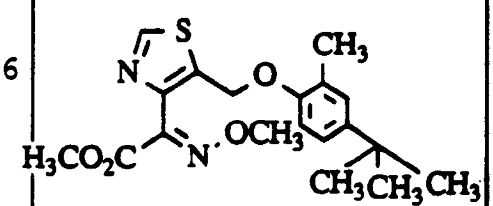 | 4,1 (s, 3 H); 3,9 (s, 3 H) | |
| 117 | 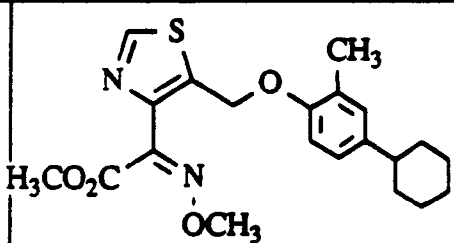 | | 100 |
| 118 | 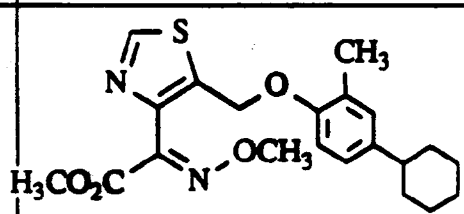 | 4,1 (s, 3 H); 3,9 (s, 3 H) | |
| | | | |

Tabelle 3: Fortsetzung

| Nr. | Verbindung | IR ($cm^{-1}$) oder $^1$H-NMR (ppm) | Fp (° C) |
|---|---|---|---|
| 119 | 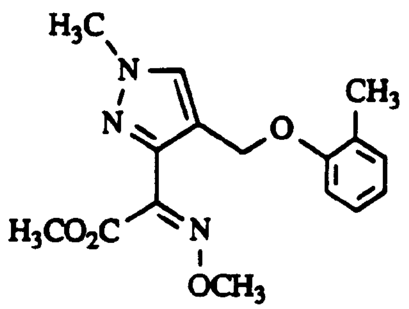 | | 78 |
| 120 | 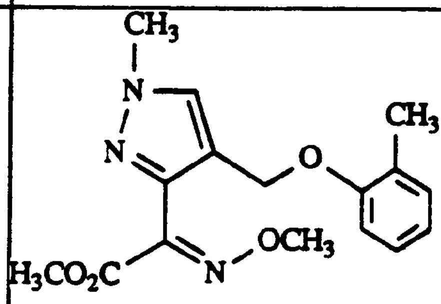 | | 93 |
| 121 | 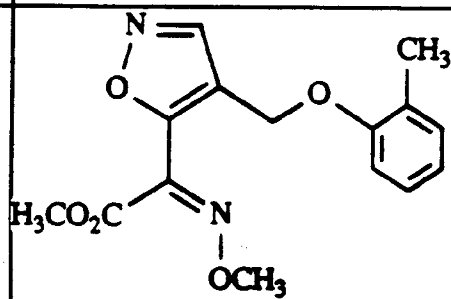 | | 63 |
| 122 | 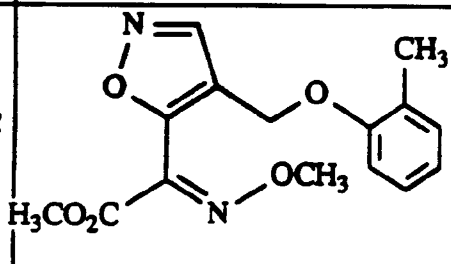 | | 88 |
| 123 | 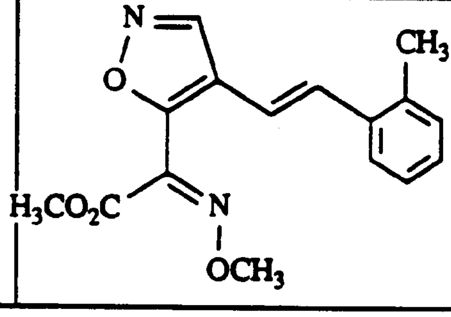 | | 93 |

Tabelle 3: Fortsetzung

| Nr. | Verbindung | IR ($cm^{-1}$) oder $^{1}H$-NMR (ppm) | Fp (° C) |
|---|---|---|---|
| 124 | 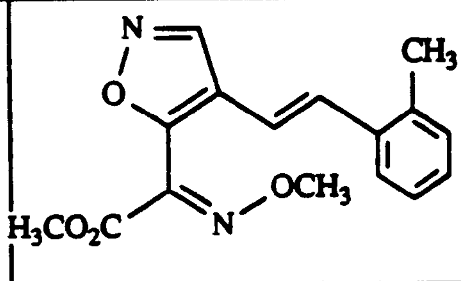 | | 93 |
| 125 | 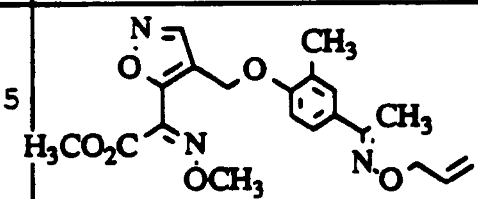 | 4,1 (s, 3 H); 3,95 (s, 3 H) | |
| 126 | 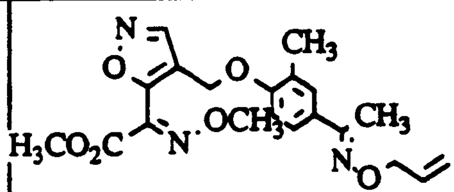 | | 58 |
| 127 | 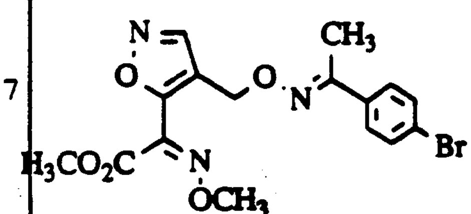 | | 74 |
| 128 | 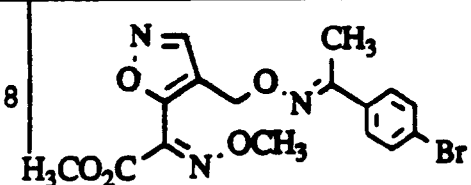 | | 72 |

Tabelle 3: Fortsetzung

| Nr. | Verbindung | IR ($cm^{-1}$) oder $^1H$-NMR (ppm) | Fp (° C) |
|---|---|---|---|
| 129 | 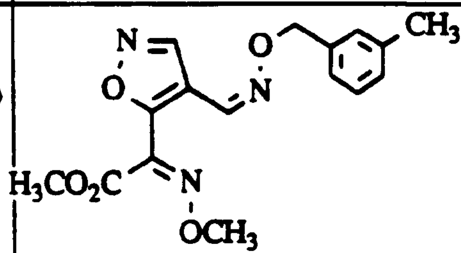 | 4,1 (s, 3 H); 3,95 (s, 3 H) | |
| 130 | 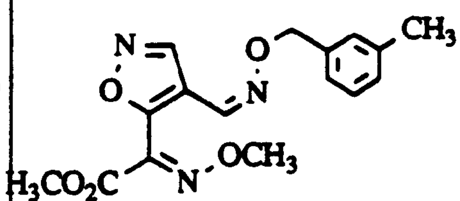 | | 91 |
| 131 | 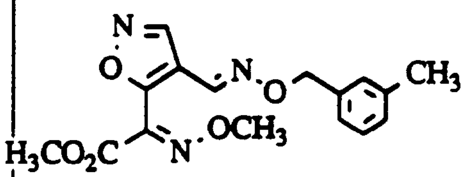 | 4,15 (s, 3 H); 3,9 (s, 3 H) | |
| 132 | 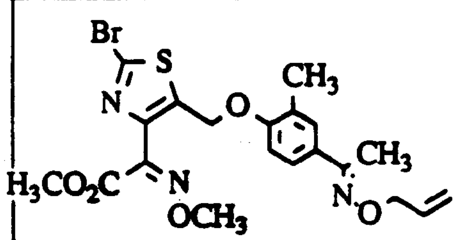 | | 106 |
| 133 | 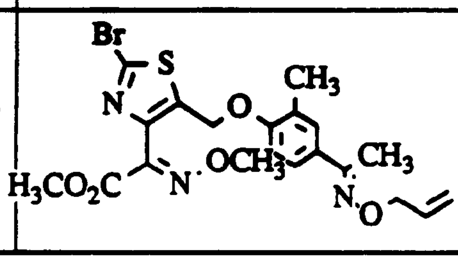 | | 125 |

Tabelle 3: Fortsetzung

| Nr. | Verbindung | IR ($cm^{-1}$) oder $^1H$-NMR (ppm) | Fp (° C) |
|---|---|---|---|
| 134 | 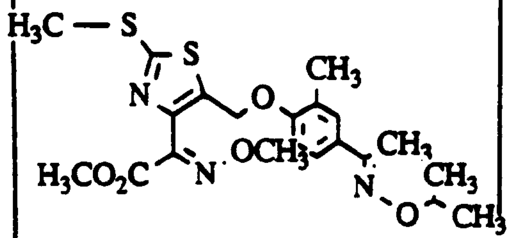 | | 83 |
| 135 | 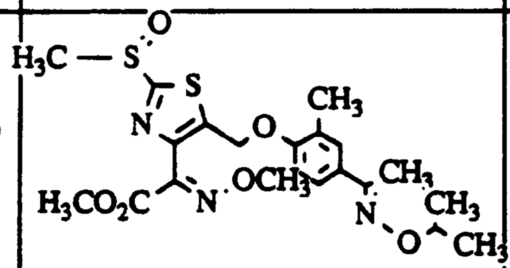 | | 99 |
| 136 | 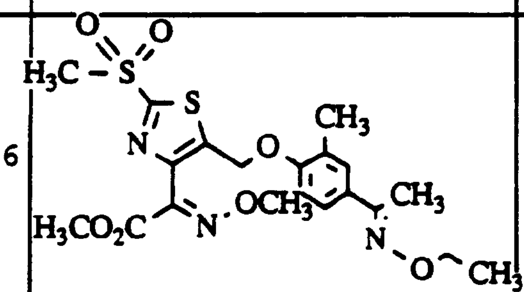 | | 126 |
| 137 | 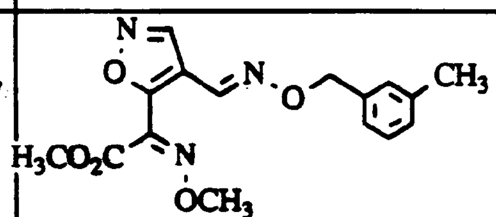 | 4,1 (s, 3 H); 3,9 (s, 3 H) | |
| 138 | 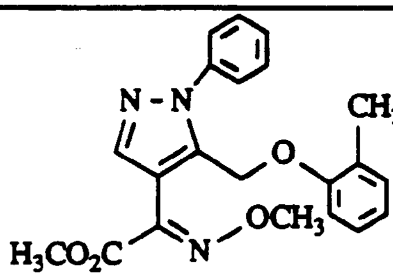 | 4,05 (s, 3 H); 3,7 (s, 3 H) | |

Tabelle 3: Fortsetzung

| Nr. | Verbindung | IR ($cm^{-1}$) oder $^1$H-NMR (ppm) | Fp (° C) |
|---|---|---|---|
| 139 | 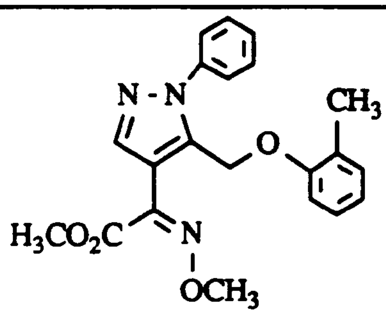 | 3,82 (s, 3 H); 3,80 (s, 3 H) | |
| 140 | 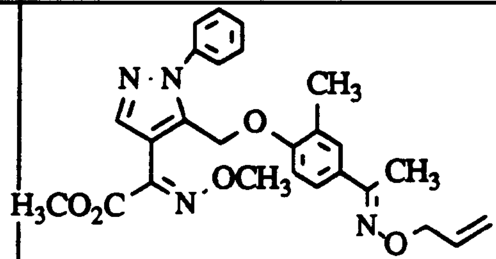 | 4,05 (s, 3 H); 3,75 (s, 3 H) | |
| 141 | 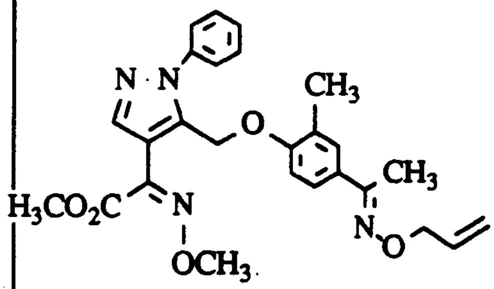 | 3,82 (s, 3 H); 3,80 (s, 3 H) | |
| 142 | 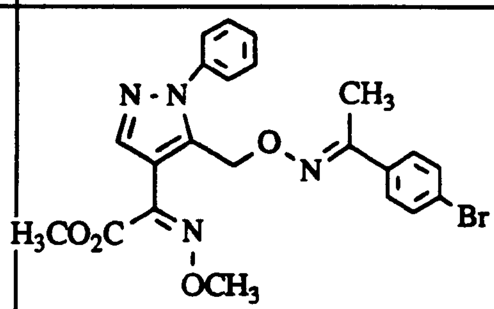 | 4,0 (s, 3 H); 3,85 (s, 3 H) | |
| 143 | 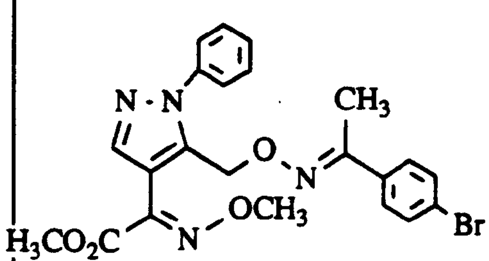 | 4,1 (s, 3 H); 3,85 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 144 | 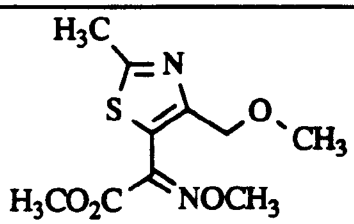 | 4,15 (s, 3 H); 3,9 (s, 3 H) | |
| 145 | 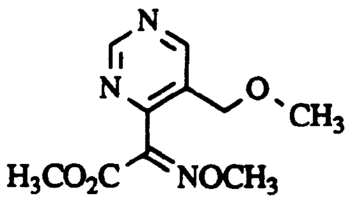 | | 82 |
| 146 | 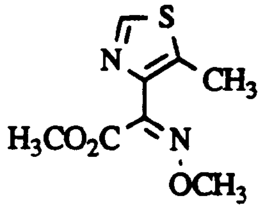 | 4,05 (s, 3 H); 3,95 (s, 3 H) | |
| 147 | 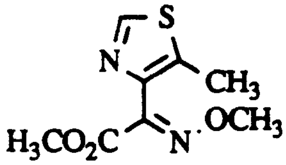 | | 65 |
| 148 | 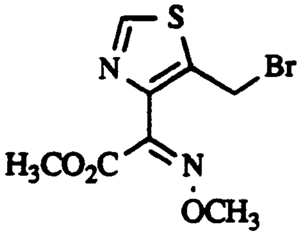 | 4,1 (s, 3 H); 3,95 (s, 3 H) | |

Tabelle 3: Fortsetzung

| Nr. | Verbindung | IR ($cm^{-1}$) oder $^{1}H$-NMR (ppm) | Fp (° C) |
|---|---|---|---|
| 149 | 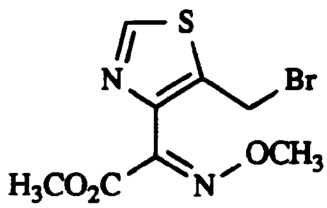 | 4,15 (s, 3 H); 3,9 (s, 3 H) | |
| 150 | 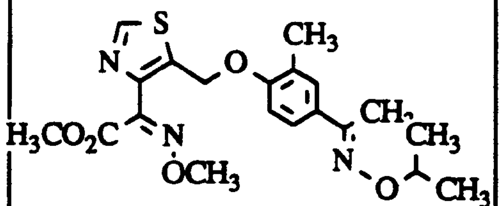 | | 107 |
| 151 | 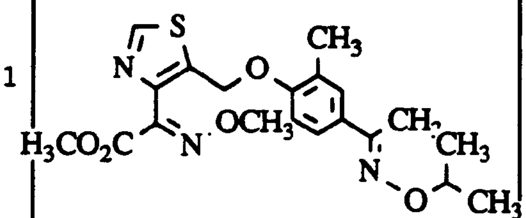 | | 87 |
| 152 | 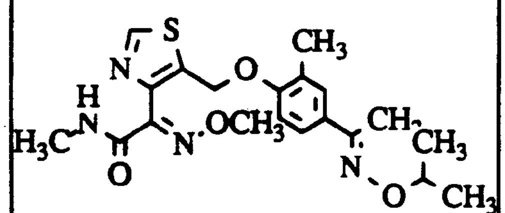 | | 147 |
| 153 | 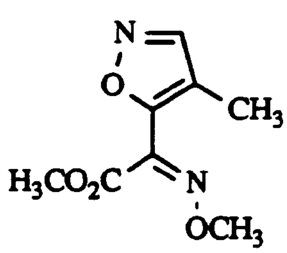 | 4,1 (s, 3 H); 3,95 (s 3 H) | |

Tabelle 3: Fortsetzung

| Nr. | Verbindung | IR ($cm^{-1}$) oder $^1H$-NMR (ppm) | Fp (° C) |
|---|---|---|---|
| 154 | 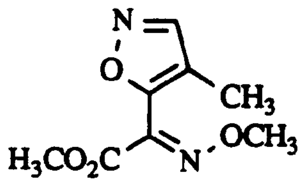 | 4,2 (s, 3 H); 3,9 (s, 3 H) | |
| 155 | 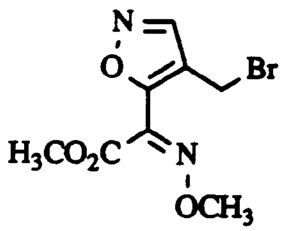 | 4,15 (s, 3 H); 4,0 (s, 3 H) | |
| 156 | 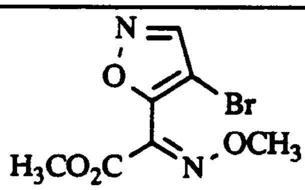 | | 45 |
| 157 | 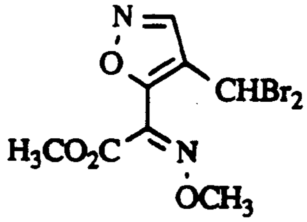 | 4,2 (s, 3 H); 3,95 (s, 3 H) | |
| 158 | 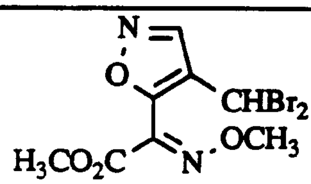 | 4,25 (s, 3 H); 3,95 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 159 | 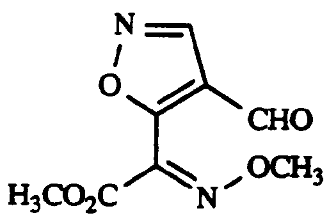 | 4,25 (s, 3 H); 3,95 (s, 3 H) | |
| 160 | 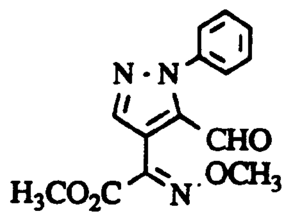 | 4,15 (s, 3 H); 3,9 (s, 3 H) | |
| 161 | 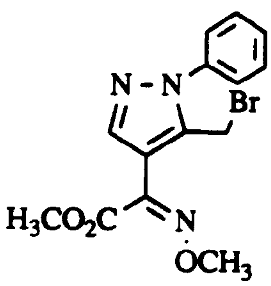 | 4,05 (s, 3 H); 3,95 (s, 3 H) | |
| 162 | 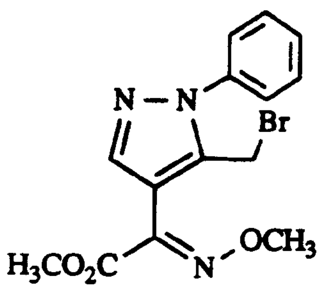 | 4,2 (s, 3 H); 3,95 (s, 3 H) | |
| 163 | 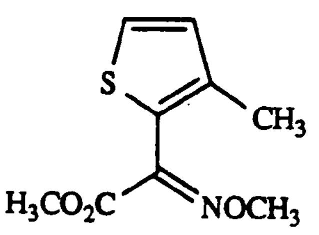 | Isomer A: 4,1 (s, 3 H); 3,9 (s, 3 H) Isomer B: 4,0 (s, 3 H); 3,9 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 164 | 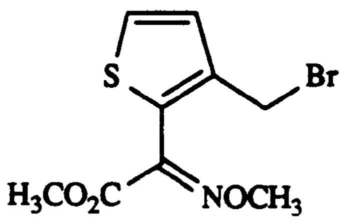 | Isomer A:<br>4,15 (s, 3 H);<br>3,9 (s, 3 H)<br><br>Isomer B:<br>4,05 (s, 3 H);<br>3,95 (s, 3 H) | |
| 165 | 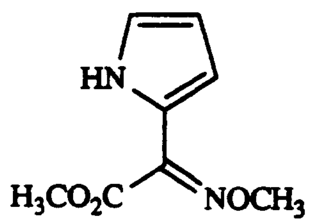 | 4,15 (s, 3 H);<br>3,9 (s, 3 H) | |
| 166 | 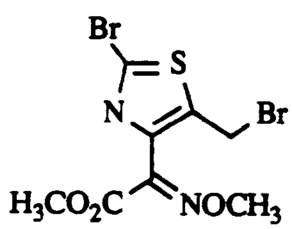 | 2 Isomere:<br>4,2 (s, 3 H);<br>3,9 (s, 3 H)<br><br>und<br><br>4,1 (s, 3 H);<br>4,0 (s, 3 H) | |
| 167 | 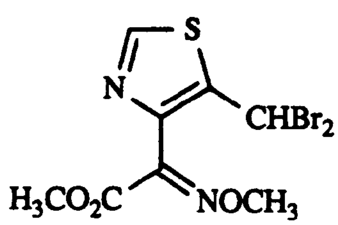 | 2 Isomere:<br>4,2 (s, 3 H);<br>3,95 (s, 3 H)<br><br>und<br><br>4,15 (s, 3 H);<br>4,0 (s, 3 H) | |
| 168 | 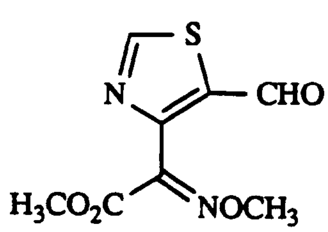 | 4,15 (s, 3 H);<br>4,0 (s, 3 H) | |

Tabelle 3: Fortsetzung

| Nr. | Verbindung | IR ($cm^{-1}$) oder $^{1}H$-NMR (ppm) | Fp (° C) |
|---|---|---|---|
| 169 | 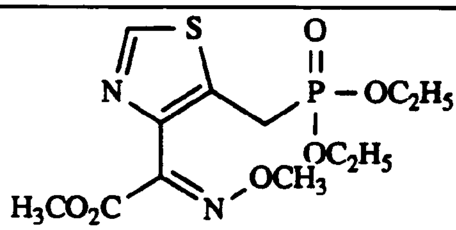 | 4,15 (s, 3 H); 3,9 (s, 3 H) | |
| 170 | 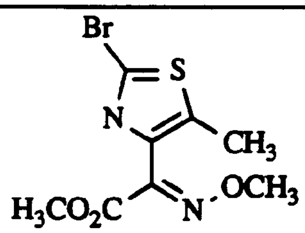 | 4,15 (s, 3 H); 3,9 (s, 3 H) | |
| 171 | 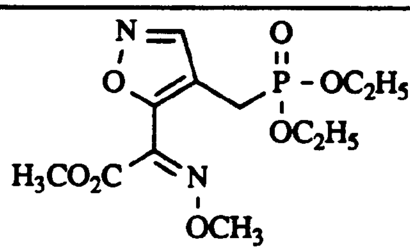 | 4,1 (s, 3 H); 3,95 (s, 3 H) | |
| 172 | 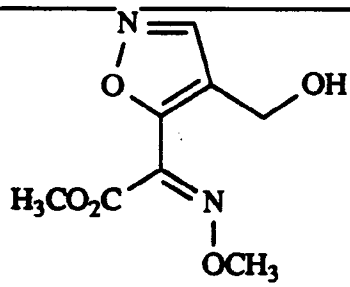 | 4,1 (s 3 H); 4,0 (s 3 H) | |
| 173 | 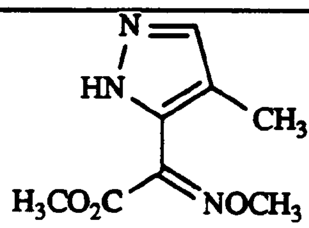 | 2 Isomere: 4,0 (s, 3 H); 3,8 (s, 3 H) und 4,1 (s, 3 H); 3,9 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 174 | 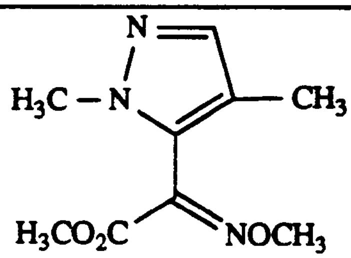 | 2 Isomere: 4,02 (s, 3 H); 3,95 (s, 3 H); 3,88 (s, 3 H) und 4,15 (s, 3 H); 3,9 (s, 3 H); 3,7 (s, 3 H) | |
| 175 | 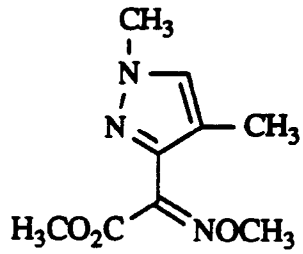 | 2 Isomere: 3,97 (s, 3 H); 3,92 (s, 3 H); 3,8 (s, 3 H) und 4,1 (s, 3 H); 3,85 (s, 6 H) | |
| 176 | 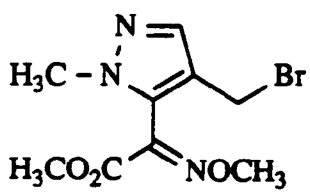 | 4,1 (s, 3 H); 3,95 (s, 6 H) | |
| 177 | 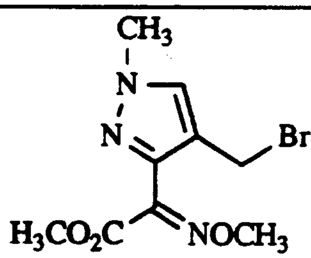 | Isomer A: 4,05 (s, 3 H); 4,95 (s, 3 H); 4,85 (s, 3 H) Isomer B: 4,15 (s, 3 H); 3,9 (s, 6 H) | |
| 178 | 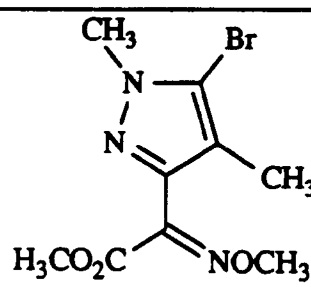 | 4,0 (s, 3 H); 3,95 (s, 3 H); 3,85 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 179 | 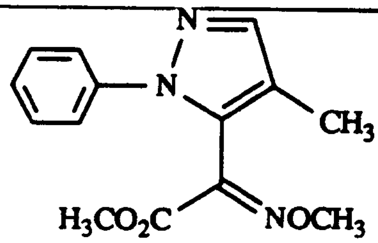 | 2 Isomere: 4,05 (s, 3 H); 3,65 (s, 3 H) und 4,0 (s, 3 H); 3,45 (s, 3 H) | |
| 180 | 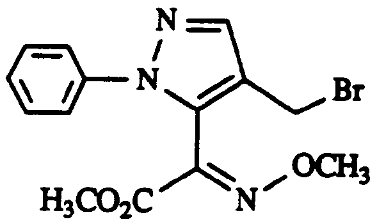 | 4,1 (s, 3 H); 3,65 (s, 3 H) | |
| 181 | 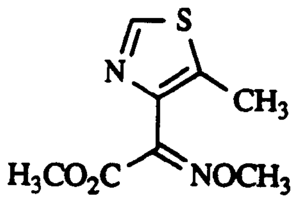 | 4,15 (s, 3 H); 3,95 (s, 3 H) | |
| 182 | 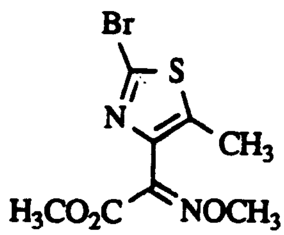 | 4,15 (s, 3 H); 3,9 (s, 3 H) | |
| 183 | 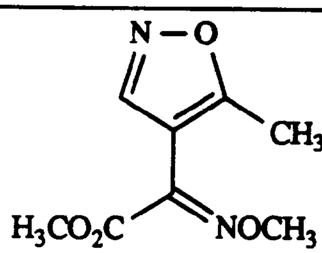 | 4,0 (s, 6 H) | |

Tabelle 3: Fortsetzung

| Nr. | Verbindung | IR ($cm^{-1}$) oder $^1H$-NMR (ppm) | Fp (° C) |
|---|---|---|---|
| 184 | 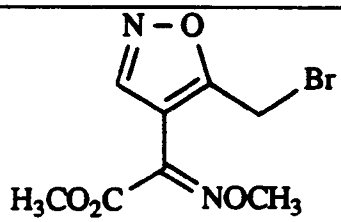 | 2 Isomere: 4,1 (s, 3 H); 4,0 (s, 3 H) und 4,05 (s, 3 H); 3,9 (s, 3 H) | |
| 185 | 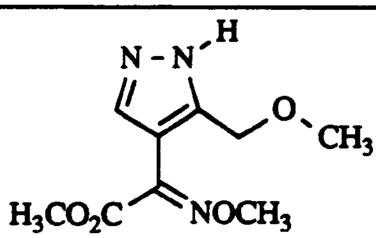 | 4,1 (s, 3 H); 3,9 (s, 3 H) | |
| 186 | 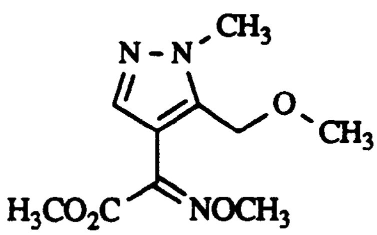 | 4,1 (s, 3 H); 3,9 (s, 6 H) | |
| 187 | 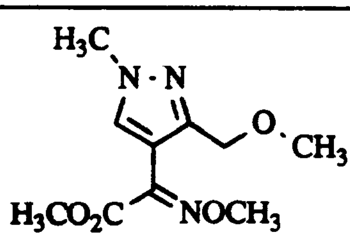 | 4,1 (s, 3 H); 3,9 (2 s, 6 H) | |
| 188 | 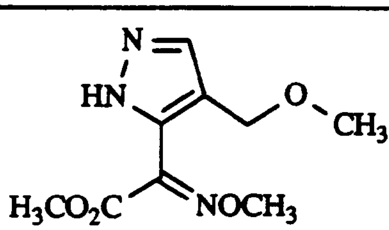 | 4,0 (s, 3 H); 3,9 (s, 3 H) | |

Tabelle 3: Fortsetzung

| Nr. | Verbindung | IR ($cm^{-1}$) oder $^1H$-NMR (ppm) | Fp (° C) |
|---|---|---|---|
| 189 | 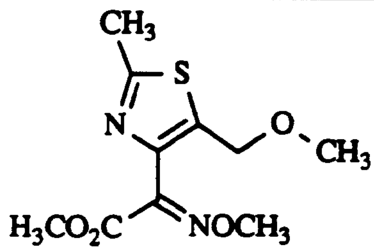 | 4,0 (s, 3 H); 3,95 (s, 3 H) | |
| 190 | 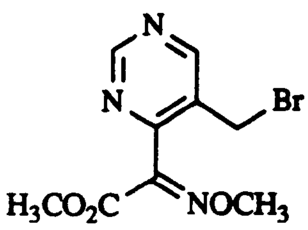 | 2 Isomere: 4,3 (s, 3 H); 4,0 (s, 3 H) und 4,15 (S, 3 H) 3,95 (S, 3 H) | |
| 191 | 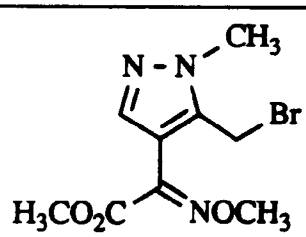 | 2 Isomere: 4,1 (s, 2 x 3 H); 4,05 (s, 3 H); 3,9 (s, 3 H); 3,35 (s, 2 x 3 H) | |
| 192 | 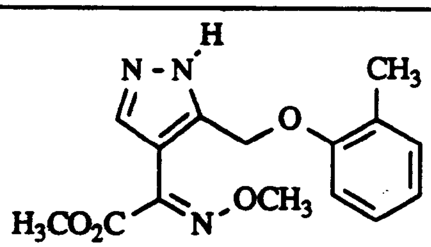 | 4,05 (s, 3H) 3,7 (s, 3H) | |
| 193 | 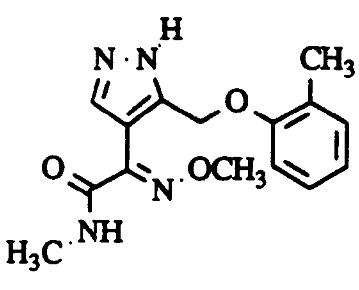 | | 47 |

Tabelle 3: Fortsetzung

| Nr. | Verbindung | IR (cm$^{-1}$) oder $^{1}$H-NMR (ppm) | Fp (°C) |
|---|---|---|---|
| 194 | 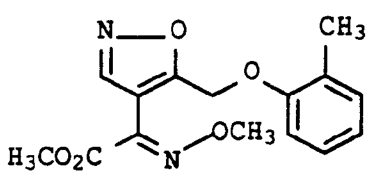 | 4,1 (s,3H); 3,75 (s,3H) | |
| 195 | 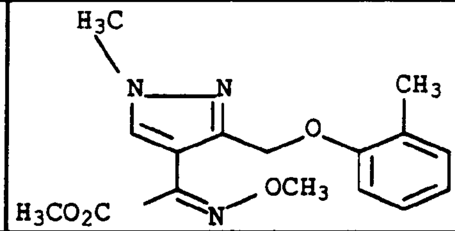 | | 54 |
| 196 | 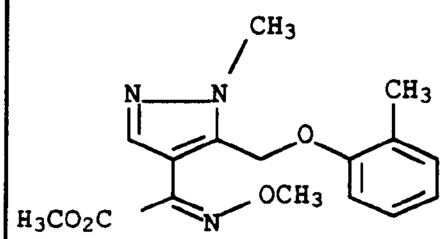 | 4,0 (s,3H); 3,95 (s,3H); 3,8 (s,3H) | |
| 197 | 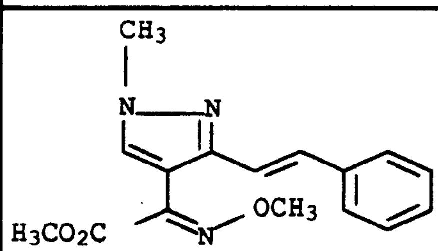 | 4,1 (s,3H); 3,9 (s,3H); 3,85 (s,3H) | |
| 198 | 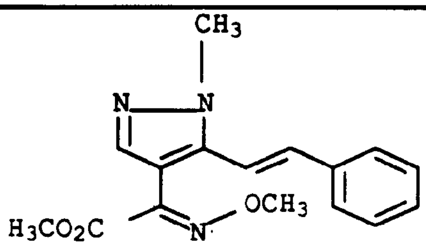 | 4,1 (s,3H); 3,9 (s,3H); 3,75 (s,3H) | |

Die neuen Verbindungen eignen sich als Fungizide, Insektizide, Nematizide und zur Regulierung des Pflanzenwachstums.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwekken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der

Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen der Verbindung Tabelle 3, Nr. 1 und 10 Gew.-Teilen N-Methyl-a-pyrrolidon, die zur Anwendung in Form kleinster Tropfen geeignet ist;

II. eine Mischung aus 20 Gew.-Teilen der Verbindung Tabelle 3, Nr. 2, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Tabelle 3, Nr. 3, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Tabelle 3, Nr. 4, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Tabelle 3, Nr. 5, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Tabelle 3, Nr. 6 und 97 Gew.-Teilen feinteiligem Kaolin; dieses Stäubemittel enthält 3 Gew.-% Wirkstoff;

VII. eine innige Mischung aus 30 Gew.-Teilen der Verbindung Tabelle 3, Nr. 7, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Tabelle 3, Nr. 8, 10 Gew.-Teilen des Natriumsalzes eines Phenolsulfonsäure-harnstoffformaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Tabelle 3, Nr. 9, 2 Gew.-Teilen des Calciumsalzes der Dodecylbenzolsulfonsäure, 8 Gew.-Teilen Fettalkohol-polyglykolether, 20 Gew.-Teilen

des Natriumsalzes eines Phenolsulfonsäure-harnstoff-formaldehydKondensates und 68 Gew.-Teilen eines paraffinischen Mineralöls.

Die Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Anwendungsbeispiele

Als Vergleichswirkstoff wurde 2-(2-Methylphenyl)-3-methoxyacrylsäuremethylester (A) - bekannt aus EP 178 826 - benutzt

Anwendungsbeispiel 1

Wirksamkeit gegen Rebenperosnopora

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Das Ergebnis zeigt, daß die Wirkstoffe aus Tabelle 3, Nr. 17, 23, 24, 27, 29, 60, 62, 64, 66, 70, 91, 101, 102, 112, 113, 122, 123, 124, 126, 130 und 134 bei der Anwendung als 250 ppm (Gew.-Teile) Wirkstoff enthaltende Spritzbrühe eine bessere fungizide Wirkung zeigen (90 %) als der bekannte Vergleichswirkstoff A (0 %).

Anwendungsbeispiel 2

Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24°C und 95 bis 99 % relativer Luftfeuchtigkeit aufgestellt. Nach 6 Tagen wurde das Ausmaß des Krankheitsbefalls ermittelt.

Das Ergebnis zeigt, daß die Wirkstoffe aus Tabelle 3, Nr. 17, 19, 20, 23, 90, 91 und 102 bei der Anwendung als 500 ppm (Gew.-Teile) Wirkstoff enthaltende Spritzbrühe eine bessere fungizide Wirkung zeigen (90 %) als der bekannte Vergleichswirkstoff A (10 %).

## Patentansprüche

1. Heteroaromatische Verbindungen der allgemeinen Formel IA und IB

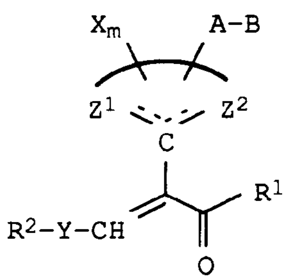

IA

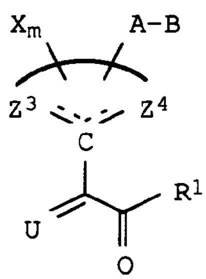

IB

in denen die gestrichelte Linie für eine Doppelbindung zwischen dem C-Atom und $Z^1$ oder $Z^3$ oder zwischen dem C-Atom und $Z^2$ oder $Z^4$ steht, und der Index und die Substituenten die folgende Bedeutung haben:

$R^1$

$C_1$-$C_6$-Alkyl, $C_1$-$C_6$-Alkoxy, $C_1$-$C_6$-Alkylamino oder Di-($C_1$-$C_6$-alkyl)-amino;

$R^2$

$C_1$-$C_6$-Alkyl;

A

eine direkte Bindung; ggf. subst. Alkylen, Alkenylen, Alkinylen; $-(CHR^4)_n$-O-, $-(CHR^4)_n$-S-, $-(CHR^4)_n$-NH-, $-(CHR^4)_n$-$NR^5$-, $-(CHR^4)_n$-S(=O)-, $-(CHR^4)_n$-$S(=O)_2$-, $-(CHR^4)_n$-O-S(=O)-, $-(CHR^4)_n$-S(=O)-O-, $-(CHR^4)_n$-O-$S(=O)_2$-, $-(CHR^4)_n$-$S(=O)_2$-O-, $-(CHR^4(_n$-C(=O)-, $-(CHR^4)_n$-C(=O)O-, $-(CHR^4)_n$-O-C(=O)-, $-(CHR^4)_n$-$CR^5$=N-O-, $-(CHR^4)_n$-$CR^5$=N-N=$CR^6$-, $-(CHR^4)_n$-O-N=$CR^7$-,

| | |
|---|---|
| n | 0, 1, 2, 3 oder 4, wobei die Reste $R^4$ verschieden sein können, wenn der Wert von n größer als 1 ist; |
| $R^4$, $R^5$ und $R^6$ | unabhängig voneinander Wasserstoff; ggf. subst. $C_1$-$C_6$-Alkyl, $C_2$-$C_6$-Alkenyl, $C_2$-$C_6$-Alkinyl oder Aryl; |
| $R^7$ | Wasserstoff, Cyano; |

ggf. subst. Alkyl, Alkenyl, Alkinyl, Cycloalkyl, Cycloalkenyl, Cycloalkinyl, Heterocyclyl, Aryl, Heteroaryl;
ggf. subst. Alkoxy, Alkenyloxy, Alkinyloxy, Cycloalkoxy, Cycloalkenyloxy, Cycloalkinyloxy, Heterocyclyloxy, Aryloxy, Heteroaryloxy;
oder $R^7$ und B gemeinsam mit dem C-Atom an das sie gebunden sind ein ggf. subst., gesättigtes oder teilweise ungesättigtes, alicyclisches oder heterocyclisches System;

B
Wasserstoff, Halogen,
ggf. subst. Alkyl, Alkenyl, Alkinyl;
Cycloalkyl, Cycloalkenyl, Cycloalkinyl, Heterocyclyl, Aryl, Heteroaryl, $-(CHR^4)_p$-Cycloalkyl, $-(CHR^4)_p$-Cycloalkenyl, $-(CHR^4)_p$-Cycloalkinyl, $-(CHR^4)_p$-Heterocyclyl, $-(CHR^4)_p$-Aryl, $-(CHR^4)_p$-Heteroaryl, $-(CHR^4)_p$-O-Cycloalkyl, $-(CHR^4)_p$-O-Cycloalkenyl, $-(CHR^4)_p$-O-Cycloalkinyl, $-(CHR^4)_p$-O-Hetetocyclyl, $-(CHR^4)_p$-O-Aryl, $-(CHR^4)_p$-O-Heteroaryl, $-(CHR^4)_p$-S-Cylcloalkyl, $-(CHR^4)_p$-S-Cylcoalkenyl, $-(CHR^4)_p$-S-Cylcloalkinyl, $-(CHR^4)_p$-S-Heterocyclyl, $-(CHR^4)_p$-S-Aryl, $-(CHR^4)_p$-S-Heteroaryl, $-(CHR^4)_p$-NH-Cycloakyl, $-(CHR^4)_p$-NH-Cycloalkenyl, $-(CHR^4)_p$-NH-Cycloalkinyl, $-(CHR^4)_p$-NH-Heterocyclyl, $-(CHR^4)_p$-NH-Aryl, $-(CHR^4)_p$-NH-Heteroaryl, $-(CHR^4)_p$-$NR^5$-Cycloalkyl, $-(CHR^4)_p$-$NR^5$-Cycloalkenyl, $-(CHR^4)_p$-$NR^5$-Cycloalkinyl, $-(CHR^4)_p$-$NR^5$-Heterocyclyl, $-(CHR^4)_p$-$NR^5$-Aryl, $-(CHR^4)_p$-$NR^5$-Heteroaryl, wobei die vorstehend genannten cyclischen Reste ihrerseits Substituenten tragen können;

p 1, 2, 3 oder 4, wobei die Reste $R^4$ verschieden sein können, wenn der Wert von n größer als 1 ist;

U
eine Gruppe $CH_2$, CHCl, $CHR^2$ oder $NOR^2$;

X
Wasserstoff, Cyano, Nitro, Halogen, Haloalkyl, Haloalkoxy,
ggf. subst. Alkyl, Alkenyl, Alkinyl, Cycloalkyl, Cycloalkenyl, Heterocyclyl, Aryl, Heteroaryl;
ggf. subst. Alkoxy, Alkenyloxy, Alkinyloxy, Cycloalkoxy, Cycloalkenyloxy, Heterocyclyloxy, Aryloxy, Heteroaryloxy;
ggf. subst. Alkylthio, Alkenylthio, Alkinylthio, Cycloalkylthio, Cycloalkenylthio, Heterocyclylthio, Arylthio, Heteroarylthio;
Amino, welches ein oder zwei der folgenden Gruppen tragen kann: ggf. subst. Alkyl, Alkenyl, Alkinyl, Cycloalkyl, Cycloalkenyl, Heterocyclyl, Aryl und/oder Heteroaryl;
ggf. subst. Alkylcarbonyl, Alkenylcarbonyl, Alkinylcarbonyl, Cycloalkylcarbonyl, Cycloalkenylcarbonyl, Heterocyclylcarbonyl, Arylcarbonyl, Heteroarylcarbonyl;
ggf. subst. Alkylcarbonyloxy, Alkenylcarbonyloxy, Alkinylcarbonyloxy, Cycloalkylcarbonyloxy, Cycloalkenylcarbonyloxy, Heterocyclylcarbonyloxy, Arylcarbonyloxy, Heteroarylcarbonyloxy;
ggf. subst. Alkoxycarbonyl, Alkenyloxycarbonyl, Alkinyloxycarbonyl, Cycloalkoxycarbonyl, Cycloalkenyloxycarbonyl, Heterocyclyloxycarbonyl, Aryloxycarbonyl, Heteroaryloxycarbonyl;
ggf. subst. Alkylcarbonylamino, Alkenylcarbonylamino, Alkinylcarbonylamino, Cycloalkylcarbonylamino, Cycloalkenylcarbonylamino, Heterocyclylcarbonylamino, Arylcarbonylamino, Heteroarylcarbonylamino, wobei diese Reste an der Aminogruppe zusätzlich eine der folgenden Gruppen tragen können: ggf. subst. Alkyl, Alkenyl, Alkinyl, Cycloalkyl, Cycloalkenyl, Heterocyclyl, Aryl und/oder Heteroaryl;
Aminocarbonyl, welches an der Aminogruppe ein oder zwei der folgenden Gruppen tragen kann: ggf. subst. Alkyl, Alkenyl, Alkinyl, Cycloalkyl, Cycloalkenyl, Heterocyclyl, Aryl, und/oder Heteroaryl;
ggf. subst. Alkyl-S(=O)-, Alkenyl-S(=O)-, Alkinyl-S(=O)-, Cycloalkyl-S(=O)-, Cycloalkenyl-S(=O)-, Heterocyclyl-S(=O)-, Aryl-S(=O)-, Heteroaryl-S(=O)-;
ggf. subst. Alkyl-S(=O)-O-, Alkenyl-S(=O)-O-, Alkinyl-S(=O)-O-, Cycloalkyl-S(=O)-O-, Cycloalkenyl-S(=O)-O-, Heterocyclyl-S(=O)-O-, Aryl-S(=O)-O-, Heteroaryl-S(=O)-O-,;
ggf. subst. Alkyl-O-S(=O)-, Alkenyl-O-S(=O)-, Alkinyl-O-S(=O)-, Cycloalkyl-O-S(=O)-, Cycloalkenyl-O-S(=O)-, Heterocyclyl-O-S(=O)-, Aryl-O-S(=O)-, Heteroaryl-O-S(=O)-;
ggf. subst. Alkyl-$S(=O)_2$-, Alkenyl-$S(=O)_2$-, Alkinyl-$S(=O)_2$-, Cycloalkyl-$S(=O)_2$-, Cycloalkenyl-$S(=O)_2$-, Heterocyclyl-$S(=O)_2$-, Aryl-$S(=O)_2$-, Heteroaryl-$S(=O)_2$-;
ggf. subst. Alkyl-$S(=O)_2$-O-, Alkenyl-S $(=O)_2$-O-, Alkinyl-$S(=O)_2$-O-, Cycloalkyl-$S(=O)_2$-O-, Cycloalkenyl-$S(=O)_2$-O-, Heteroxyclyl-$S(=O)_2$-O-, Aryl-$S(=O)_2$-O-, Heteroaryl-$S(=O)_2$-O-;
ggf. subst. Alkyl-O-$S(=O)_2$-, Alkenyl-O-$S(=O)_2$-, Alkinyl-O-$S(=O)_2$-,Cycloalkyl-O-$S(=O)_2$-, Cycloalkenyl-O-$S(=O)_2$-, Heterocyclyl-O-$S(=O)_2$-, Aryl-O-$S(=O)_2$-, Heteroaryl-O-$S(=O)_2$-;
ggf. subst. Alkyl-ON=$CR^8$-, Alkenyl-ON=$CR^8$-, Alkinyl-ON=$CR^8$-, Cycloalkyl-ON=$CR^8$-, Cycloalkenyl-ON=$CR^8$-, Heterocyclyl-ON=$CR^8$-, Aryl-ON=$CR^8$-, Heteroaryl-ON=$CR^8$-;

$R^8$ Wasserstoff; Cyano;
ggf. subst. Alkyl, Alkenyl, Alkinyl, Cycloalkyl, Cycloalkenyl, Cycloalkinyl, Heterocyclyl, Aryl, Heteroaryl;
ggf. subst. Alkoxy, Alkenyloxy, Alkinyloxy, Cycloalkoxy, Cycloalkenyloxy, Cycloalkinyloxy, Heterocyclyloxy, Aryloxy, Heteroaryloxy;

m
1 oder 2, wobei die Reste X verschieden sein können, wenn m den Wert 2 hat;

Y
Sauerstoff oder Schwefel;

$Z^1$-$Z^2$
zusammen mit dem C-Atom an das sie gebunden sind entweder ein 6-gliedriger Heteroaromat enthaltend neben Kohlenstoffringgliedern zwei oder drei Stickstoffatome oder ein 5-gliedriger Heteroaromat enthaltend neben Kohlenstoffringgliedern zwei oder drei Stickstoffatome oder ein oder zwei Stickstoffatome und ein Sauerstoff- oder ein Schwefelatom;

$Z^3$-$Z^4$
zusammen mit dem C-Atom an das sie gebunden sind entweder ein 6-gliedriger Heteroaromat enthaltend neben Kohlenstoffringgliedern ein, zwei oder drei Stickstoffatome oder ein 5-gliedriger Heteroaromat enthaltend neben Kohlenstoffringgliedern ein, zwei oder drei Stickstoffatome oder ein Sauerstoff- oder ein Schwefelatom oder ein oder zwei Stickstoffatome und ein Sauerstoff- oder ein Schwefelatom ;

und ihre pflanzenverträglichen Säureadditionsprodukte und Basenadditionsprodukte,
ausgenommen solche Verbindungen, in denen
der Ring C-$Z^1$-$Z^2$ ein Pyrazolring ist, der Rest -Y-$R^2$ die Bedeutung -O-$CH_3$ hat und -A-B in 4-Stellung am Pyrazolring steht, -A- der Rest -O-$CH_2$- ist, B gegebenenfalls substituiertes Phenyl oder gegebenenfalls substituiertes Thiazolyl bedeutet oder
der Ring C-$Z^1$-$Z^2$ ein Pyrazolring ist, der Rest -Y-$R^2$ die Bedeutung -O-$CH_3$ hat und -A-B in 4-Stellung am Pyrazolring steht, -A- der Rest -O-CO- ist und B die in Anspruch 1 genannte Bedeutung besitzt oder
der Ring C-$Z^1$-$Z^2$ ein Thiazolring ist, Y Sauerstoff bedeutet und $R^1$ die Bedeutung $OCH_3$ hat,
oder
der Ring C-$Z^3$-$Z^4$ ein Thiazolring ist, X an ein N-Atom des Thiazolrings gebunden ist und U den Rest $NOR^2$ bedeutet.

2. Heteroaromatische Verbindungen gemäß den Formeln IA und IB im Anspruch 1, in der A -O-, -S-, -$CH_2$-, -O-$CH_2$-, -$CH_2$-O-, -$CH_2$-S-, -$CH_2$-O-C(=O)-, -CH=CH-, -C≡C-, -$CH_2$-O-N=C($R^6$)- oder -$CH_2$-$CH_2$- bedeutet.

3. Heteroaromatische Verbindungen der Formel II,

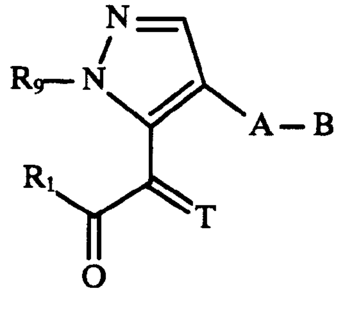

II

in der T den Rest -CH-Y-$R^2$ oder U bedeutet und $R^1$, $R^2$, U, Y, A und B die in Anspruch 1 angegebene Bedeutung besitzen und $R^9$ H, Alkyl, Alkenyl, Alkinyl oder Aryl bedeutet.

4. Heteroaromatische Verbindungen der Formel III

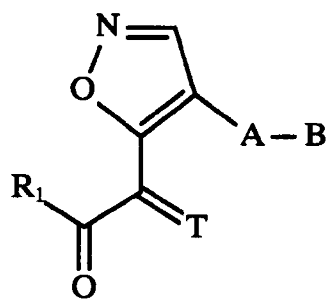

III

in der T den Rest -CH-Y-$R^2$ oder U bedeutet und $R^1$, $R^2$, U, Y, A und B die in Anspruch 1 angegebene Bedeutung besitzen.

5. Heteroaromatische Verbindungen der Formel IV

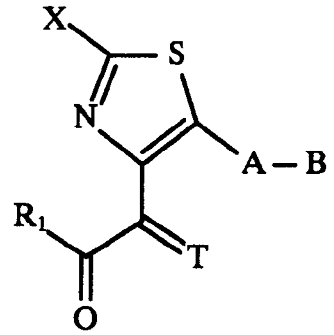

IV

in der T den Rest -CH-Y-$R^2$ oder U bedeutet und $R^1$, $R^2$, U, Y, X, A und B die in Anspruch 1 angegebene Bedeutung besitzen.

6. Heteroaromatische Verbindungen der Formel V

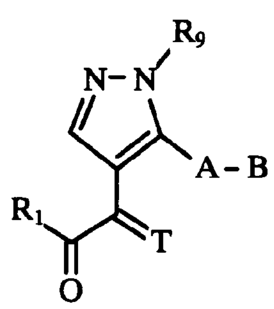

V

in der T den Rest -CH-Y-$R^2$ oder U bedeutet, $R^1$, $R^2$, U, Y, A und B die in Anspruch 1 angegebene Bedeutung besitzen und $R^9$ H, Alkyl, Alkenyl, Alkinyl oder Aryl bedeutet.

7. Heteroaromatische Verbindungen der Formel VI

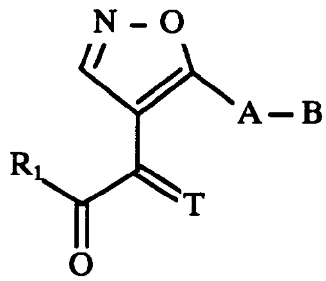

VI

in der T den Rest -CH-Y-$R^2$ oder U bedeutet und $R^1$, $R^2$, U, A und B die in Anspruch 1 angegebene Bedeutung besitzen.

**8.** Heteroaromatische Verbindungen der Formel VII

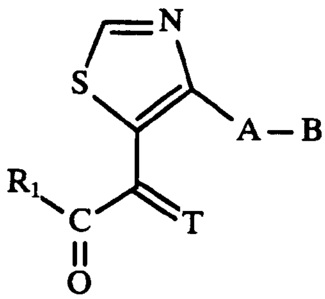

VII

in der T den Rest -CH-Y-$R^2$ oder U bedeutet und $R^1$, $R^2$, U, A und B die in Anspruch 1 angegebene Bedeutung besitzen.

**9.** Heteroaromatische Verbindungen der Formel VIII

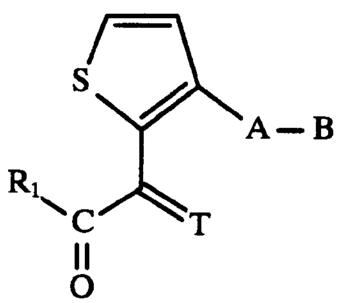

VIII

in der T den Rest -CH-Y-$R^2$ oder U bedeutet und $R^1$, $R^2$, U, Y, A und B die in Anspruch 1 genannte Bedeutung besitzen.

**10.** Heteroaromatiche Verbindungen der Formel IX

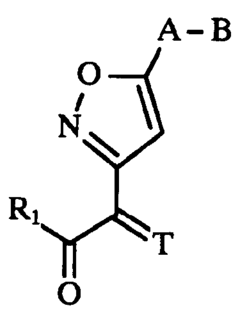

IX

in der T den Rest -CH-Y-$R^2$ oder U bedeutet und $R^1$, $R^2$, U, Y, A und B die in Anspruch 1 angegebene Bedeutung besitzen.

**11.** Heteroaromatische Verbindungen der Formel X

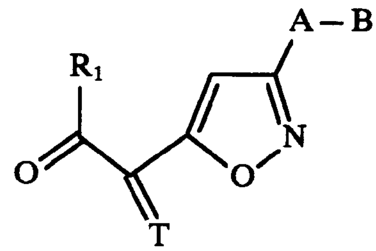

X

in der T den Rest -CH-Y-$R^2$ oder u bedeutet und $R^1$, $R^2$, U, Y, A und B die in Anspruch 1 angegebene Bedeutung besitzen.

**12.** Heteroaromatische Verbindungen der Formel XI

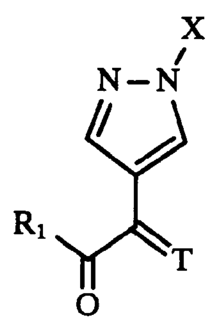

XI

in der T den Rest -CH-Y-$R^2$ oder U bedeutet und $R^1$, $R^2$, U, Y und X die in Anspruch 1 genannte Bedeutung besitzen.

**13.** Heteroaromatische Verbindungen der Formel XII

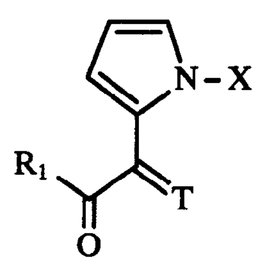

XII

in der T den Rest -CH-Y-$R^2$ oder U bedeutet und $R^1$, $R^2$, U, Y und X die in Anspruch 1 genannte Bedeutung besitzen.

**14.** Heteroaromatische Verbindungen der Formel XIII

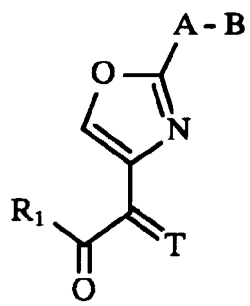

XIII

in der T den Rest -CH-Y-$R^2$ oder U bedeutet und $R^1$, $R^2$, U, Y, A und B die in Anspruch 1 genannte Bedeutung besitzen.

**15.** Heteroaromatische Verbindungen der Formel XIV

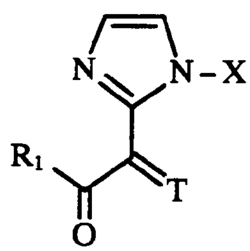

XIV

in der T den Rest -CH-Y-$R^2$ oder U bedeutet und $R^1$, $R^2$, U, Y und X die in Anspruch 1 genannte Bedeutung besitzen.

**16.** Heteroaromatische Verbindungen der Formel XV

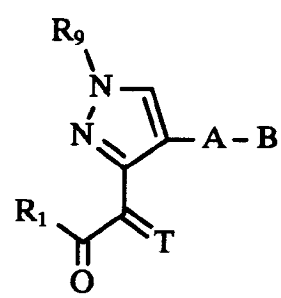

XV

in der T den Rest -CH-Y-$R^2$ oder U bedeutet und $R^1$, $R^2$, U, Y, A und B die in Anspruch 1 genannte Bedeutung besitzen und $R^9$ Wasserstoff, Alkyl, Alkenyl, Alkinyl oder Aryl bedeutet.

**17.** Heteroaromatische Verbindungen der Formeln IA und IB gemäß Anspruch 1, in denen -A-B den Rest $-CH_2$-Br bedeutet.

**18.** Heteroaromatische Verbindungen der Formeln IA und IB gemäß Anspruch 1, in denen -A-B den Rest $-CH_3$ bedeutet.

**19.** Zwischenprodukt der Formel XVI

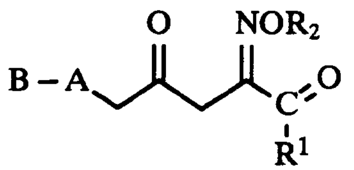

XVI

in der $R^1$ $C_1$-$C_6$-Alkoxy ist und $R^2$, A und B die in Anspruch 1 genannte Bedeutung besitzen.

**20.** Zwischenprodukt der Formel XVII

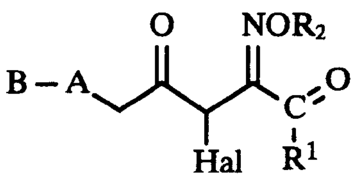

XVII

in der $R^1$ $C_1$-$C_6$-Alkoxy ist und $R^2$, A und B die in Anspruch 1 genannte Bedeutung besitzen und Hal Halogen bedeutet.

**21.** Zwischenprodukt der Formel XVIII

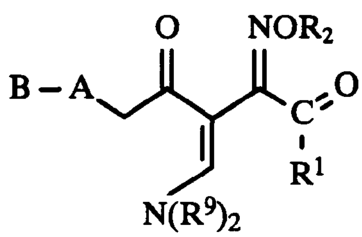

XVIII

in der $R^1$ $C_1$-$C_6$-Alkoxy ist und $R^2$, A und B die in Anspruch 1 genannte Bedeutung besitzen und $R^9$ $C_1$-$C_6$-Alkyl bedeutet.

**22.** Zwischenprodukt der Formel XIX

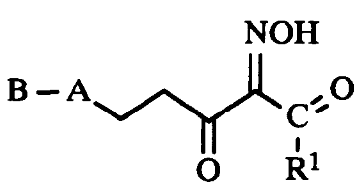

XIX

in der $R^1$ $C_1$-$C_6$-Alkoxy ist und A und B die in Anspruch 1 genannte Bedeutung besitzen.

**23.** Zwischenprodukt der Formel XX

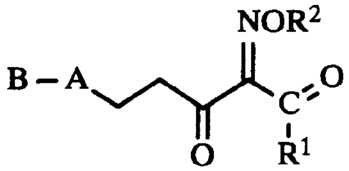

XX

in der $R^1$ $C_1$-$C_6$-Alkoxy ist und $R^2$, A und B die in Anspruch 1 genannte Bedeutung besitzen.

**24.** Zwischenprodukt der Formel XXI

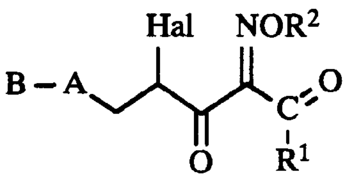

XXI

in der $R^1$ $C_1$-$C_6$-Alkoxy ist und $R^2$, A und B die in Anspruch 1 genannte Bedeutung besitzen und Hal Halogen bedeutet.

**25.** Zwischenprodukt der Formel XXII

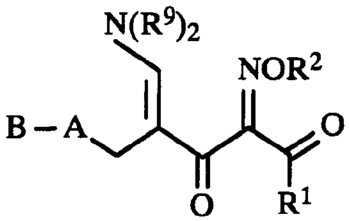

XXII

in der $R^1$ $C_1$-$C_6$-Alkoxy ist und $R^2$, A und B die in Anspruch 1 genannte Bedeutung besitzen und $R^9$ $C_1$-$C_6$-Alkyl bedeutet.

**26.** Heteroaromatische Verbindungen der Formeln IA und IB, in denen -A-B den Rest -CHO bedeutet.

**27.** Zwischenprodukt der Formel IB, in der U Sauerstoff bedeutet und $R^1$, $X_m$, A, B, $Z^3$ und $Z^4$ die in Anspruch 1 benannte Bedeutung besitzen.

**28.** Zwischenprodukt der Formel IB, in der $R^1$ $C_1$-$C_6$-Alkoxy ist, C = U den Rest CH-OH bedeutet und $X_m$, A, B, $Z^3$ und $Z^4$ die in Anspruch 1 genannte Bedeutung besitzen.

**29.** Zwischenprodukt der Formel IB, in der die Gruppe

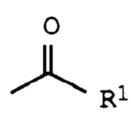

durch den Rest CN ersetzt ist und C = U den Rest CH-OH bedeutet und $X_m$, A, B, $Z^3$ und $Z^4$ die in Anspruch 1 genannte Bedeutung besitzen.

**30.** Zwischenprodukt der Formel IB, in der die Gruppe

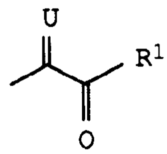

durch den Rest CHO ersetzt ist und $X_m$, A, B, $Z^3$ und $Z^4$ die in Anspruch 1 genannte Bedeutung besitzen.

**31.** Verfahren zur Herstellung von heteroaromatischen Verbindungen der Formeln IA und IB gemäß Anspruch 1, in der A $-CH(R^4)-O-C(=O)-$, $-CH(R^4)-O-N=C(R^7)-$, $-CH(R^4)_n-S-$, $-CH(R^4)_n-O-$ oder $-CH(R^4)_n-NR^5-$ bedeutet, gekennzeichnet dadurch, daß man ein Alkylbromid der Formeln IA und IB, in der -A-B den Rest $-CH(R^4)_n-Br$ bedeutet, mit den entsprechenden Nucleophilen umsetzt.

**32.** Verfahren zur Herstellung einer heteroaromatischen Verbindung der Formeln IA und IB, in der -A-B ein Alkenylenrest bedeutet, gekennzeichnet dadurch, daß man eine Carbonylverbindung der Formel IA und IB, in der A-B den Rest -CO- $C_1$-$C_6$-Alkyl bedeutet, in einer Wittig-Reaktion mit den entsprechenden Phosphoniumsalzen, Phosphinoxiden oder Phosphonaten umsetzt.

**33.** Verfahren zur Herstellung einer heteroaromatischen Verbindung der Formel IA und IB in der -A-B den Rest $-CH(R^4)_n-Br$ bedeutet, gekennzeichnet dadurch, daß man eine heteroaromatische Verbindung der Formel IA und IB, in der -A-B den Rest $-CH_2(R^4)_n$ bedeutet, mit Bromierungsmitteln, umsetzt.

**34.** Verfahren zur Herstellung einer heteroaromatischen Verbindung der Formel IA und IB, in der -A-B den Rest $-CO-R^4$ bedeutet, gekennzeichnet dadurch, daß man eine heteroaromatische Verbindung der Formel IA und IB, in der -A-B den Rest $-CBr_2-R^4$ bedeutet, hydrolysiert.

**35.** Verfahren zur Herstellung einer heteroaromatischen Verbindung der Formel IA und IB, in der -A-B den Rest

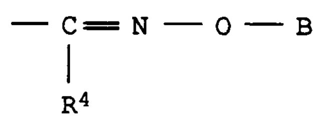

bedeutet, gekennzeichnet dadurch, daß man eine heteroaromatische Verbindung der Formel IA und IB, in der -A-B den Rest

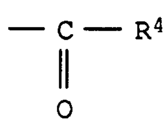

bedeutet, mit den entsprechenden Hydroxylaminen umsetzt.

**36.** Verfahren zur Herstellung einer heteroaromatischen Verbindung der Formel XXIII

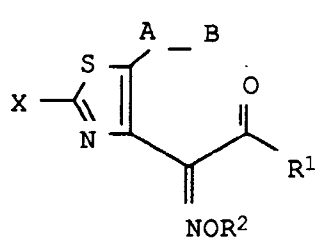

XXIII

in der $R^2$, A und B die in Anspruch 1 genannte Bedeutung haben und X Wasserstoff, oder die ggf. substituierten Reste Alkyl, Alkenyl, Alkinyl, Cycloalkyl, Cycloalkinyl, Aryl, Hetaryl oder Heterocyclyl bedeutet und $R^1$ $C_1$-$C_6$-Alkoxy ist, gekennzeichnet dadurch, daß man eine α-Halogencarbonylverbindung der Formel XXIV

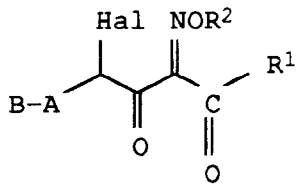

XXIV

in der $R^2$, A und B die in Anspruch 1 genannte Bedeutung besitzen, Hal Halogen bedeutet und $R^1$ $C_1$-$C_6$-Alkoy ist, mit den entsprechenden Thioamiden umsetzt.

**37.** Verfahren zur Herstellung einer α-Halogencarbonylverbindung der Formel XXIV gemäß Anspruch 36, gekennzeichnet dadurch, daß man eine Verbindung der Formel XVI

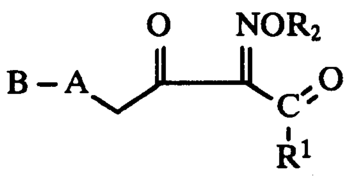

XVI

in der $R^2$, A und B die in Anspruch 1 genannte Bedeutung besitzen und $R^1$ $C_1$-$C_6$-Alkoxy ist, mit Halogenierungsmitteln umsetzt.

**38.** Verfahren zur Herstellung einer heteroaromatischen Verbindung der Formel XXV

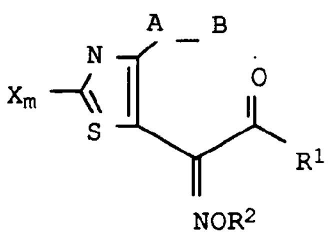

XXV

in der $R^2$, A und B die in Anspruch 1 genannte Bedeutung besitzen und $X_m$ Wasserstoff, Alkyl, Alkenyl, Alkinyl, Cycloalkyl, Cycloalkenyl, Cycloalkinyl, Aryl, Hetaryl oder Heterocyclyl bedeutet und $R^1$ $C_1$-$C_6$-Alkoxy ist, gekennzeichnet dadurch, daß man eine α-Halogencarbonylverbindung der Formel XXVI

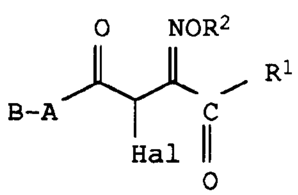

XXVI

in der $R^2$, A und B die in Anspruch 1 genannte Bedeutung besitzen, Hal Halogen bedeutet und $R^1$ $C_1$-$C_6$-Alkoxy ist, mit den entsprechenden Thioamiden umsetzt.

**39.** Verfahren zur Herstellung einer α-Halogenverbindung der Formel XXVI gemäß Anspruch 38, gekennzeichnet dadurch, daß man eine Verbindung der Formel XXVII

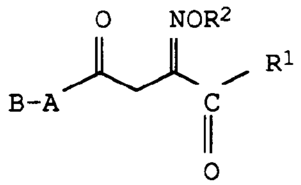

XXVII

in der $R^2$, A und B die in Anspruch 1 genannte Bedeutung besitzen und $R^1$ $C_1$-$C_6$-Alkoxy ist, mit den entsprechenden Halogenierungsmitteln umsetzt.

**40.** Verfahren zur Herstellung einer heteroaromatischen Verbindung der Formel XXVIII

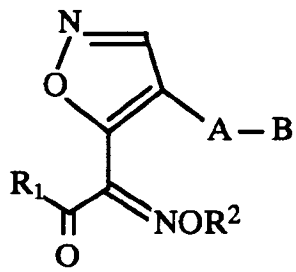

XXVIII

in der $R^2$, A und B die in Anspruch 1 genannte Bedeutung besitzen und $R^1$ $C_1$-$C_6$-Alkoxy bedeutet, gekennzeichnet dadurch, daß man eine $\alpha,\beta$-ungesättigte Carbonylverbindung der Formel XXIX

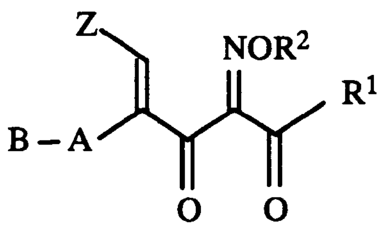

XXIX

in der $R^2$, A und B die in Anspruch 1 genannte Bedeutung besitzen und $R^1$ $C_1$-$C_6$-Alkoxy und Z Alkoxy oder Dialkylamino bedeutet, mit Hydroxylamin umsetzt.

**41.** Verfahren zur Herstellung einer $\alpha,\beta$-ungesättigten Carbonylverbindung der Formel XXIX gemäß Anspruch 40, gekennzeichnet dadurch, daß man eine Carbonylverbindung der Formel XXX

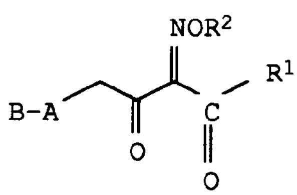

XXX

in der $R^2$, A und B die in Anspruch 1 genannte Bedeutung besitzen und $R^1$ $C_1$-$C_6$-Alkoxy bedeutet, mit Trialkylortho-formiaten oder Dialkylformamid-dialkylacetalen umsetzt.

**42.** Verfahren zur Herstellung einer heteroaromatischen Verbindung der Formel XXXI

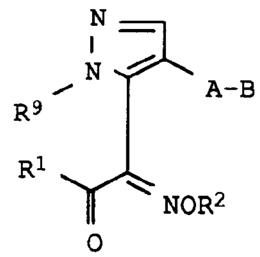

XXXI

in der $R^2$, A und B die in Anspruch 1 genannte Bedeutung besitzen und $R^9$ H, Alkyl, Alkenyl, Alkinyl oder Aryl bedeutet und $R^1$ $C_1$-$C_6$-Alkoxy ist, gekennzeichnet dadurch, daß man eine α,β-ungesättigte Carbonylverbindung der Formel XXIX gemäß Anspruch 40 mit Hydrazinen umsetzt.

**43.** Verfahren zur Herstellung einer heteroaromatischen Verbindung der Formel XXXII

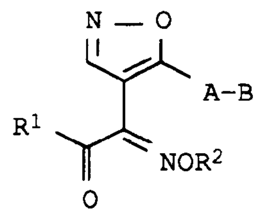

XXXII

in der $R^2$, A und B die in Anspruch 1 genannte Bedeutung besitzen und $R^1$ $C_1$-$C_6$-Alkoxy ist, gekennzeichnet dadurch, daß man eine α,β-ungesättigte Carbonylverbindung der Formel XXXIII

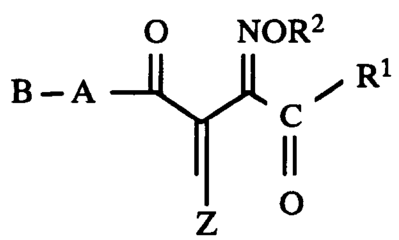

XXXIII

in der $R^2$, A und B die in Anspruch 1 genannte Bedeutung besitzen, und $R^1$ $C_1$-$C_6$-Alkoxy ist, Z Alkoxy oder Dialkylamino bedeutet, mit Hydroxylamin umsetzt.

**44.** Verfahren zur Herstellung einer α,β-ungesättigten Carbonylverbindung der Formel XXXIII gemäß Anspruch 43, gekennzeichnet dadurch, daß man eine Carbonylverbindung der Formel XXXIV

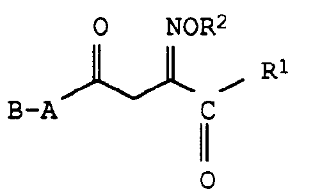

XXXIV

in der $R^2$, A und B die in Anspruch 1 genannte Bedeutung besitzen und $R^1$ $C_1$-$C_6$-Alkoxy ist, mit Trialkylorthoformiaten oder Dialkylformamiddialkylacetalen umsetzt.

**45.** Verfahren zur Herstellung einer heteroaromatischen Verbindung der Formel XXXV

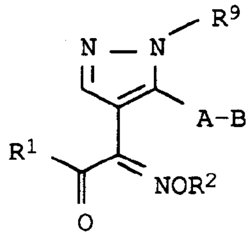

XXXV

in der $R^2$, A und B die in Anspruch 1 genannte Bedeutung besitzen, $R^9$ H, Alkyl, Alkenyl, Alkinyl bedeutet und $R^1$ $C_1$-$C_6$-Alkoxy ist, gekennzeichnet dadurch, daß man eine ($\alpha$,$\beta$-ungesättigte Carbonylverbindung der Formel XXXIII gemäß Anspruch 43 mit Hydrazinen umsetzt.

**46.** Verfahren zur Herstellung einer heteroaromatischen Verbindung der Formel Ib, in der $R^1$ $C_1$-$C_6$-Alkoxy bedeutet und $X_m$, A, B die in Anspruch 1 angegebene Bedeutung besitzen und U N-O-$R^1$, $CH_2$, CH-$R^2$, CH-Cl oder CH-O-$R^2$ bedeutet, gekennzeichnet dadurch, daß man einen $\alpha$-Ketoester der Formel IB, in der $R^1$ $C_1$-$C_6$-Alkoxy und U Sauerstoff bedeutet und $X_m$, A und B die in Anspruch 1 genannte Bedeutung besitzen, in einer Wittig-Reaktion mit dem entsprechenden Phosphoniumsalz, Phosphonat oder Phosphinoxid oder mit einem Alkoxyamin der Formel $H_2N$-O-$R^2$ umsetzt.

**47.** Verfahren zur Herstellung eines $\alpha$-Ketoesters der Formel IB gemäß Anspruch 46, gekennzeichnet dadurch, daß man eine heteroaromatische Verbindung der Formel IB, in der die Gruppe

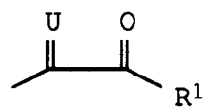

durch Wasserstoff besetzt ist und $X_m$, A und B die in Anspruch 1 genannte Bedeutung besitzen, mit Oxalsäuredialkylester oder Oxalsäurealkylesterchlorid unter saurer Katalyse oder unter basischer Katalyse umsetzt.

**48.** Verfahren zur Herstellung einer heteroaromatischen Verbindung der Formel IB, in der $R^1$ Mono-($C_1$-$C_6$-alkyl)-amin bedeutet und U, $X_m$, A und B die in Anspruch 1 genannte Bedeutung besitzen, gekennzeichnet dadurch, daß man einen Ester der Formel IB, in der $R^1$ $C_1$-$C_6$-Alkyl und U, $X_m$, A und B die in Anspruch 1 genannte Bedeutung besitzen, mit einem Mono-($C_1$-$C_6$-alkyl)amin umsetzt.

**49.** Verfahren zur Herstellung eines $\alpha$-Ketoesters der Formel IB gemäß Anspruch 46, gekennzeichnet dadurch, daß man einen $\alpha$-Hydroxyester der Formel IB, in der $R^1$ $C_1$-$C_6$-Alkoxy und C=U den Rest CHOH bedeutet und $X_m$, A und B die in Anspruch 1 genannte Bedeutung besitzen, mit NaOCl-Lösung umsetzt.

**50.** Verfahren zur Herstellung eines $\alpha$-Hydroxyesters der Formel IB gemäß Anspruch 49, gekennzeichnet dadurch, daß man ein Imidoester-hydrochlorid der Formel IB, in der die Gruppe

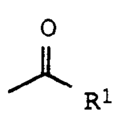

durch den Rest C(O-($C_1$-$C_6$-Alkyl)=$NH^+{}_2Cl^-$ ersetzt ist und C=U den Rest CHOH bedeutet und $X_m$, A und B die in Anspruch 1 genannte Bedeutung besitzen, mit Wasser umsetzt.

**51.** Verfahren zur Herstellung eines Imidoester-hydrochlorids der Formel IB gemäß Anspruch 50, gekennzeichnet dadurch, daß man ein Cyanhydrin der Formel IB, in der die Gruppe

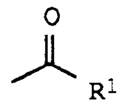

durch den Rest CN ersetzt ist und C=U den Rest CHOH bedeutet und $X_m$, A und B die in Anspruch 1 genannte Bedeutung besitzen, in Gegenwart von Salzsäure mit einem $C_1$-$C_6$-Alkylalkohol umsetzt.

**52.** Verfahren zur Herstellung eines Cyanhydrins der Formel IB gemäß Anspruch 51, gekennzeichnet dadurch, daß man einen Aldehyd der Formel IB, in der die Gruppe

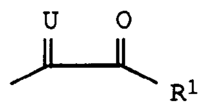

durch den Rest CHO ersetzt ist und $X_m$, A und B die in Anspruch 1 genannte Bedeutung besitzen, mit HCN umsetzt.

**53.** Verfahren zur Herstellung einer heteroaromatischen Verbindung der Formel IB, in der -A-B den Rest $-CH(R^4)_n-O-B$ bedeutet und $R^1$, $R^4$, U, $X_m$ und B die in Anspruch 1 genannte Bedeutung besitzen, gekennzeichnet dadurch, daß man eine heteroaromatische Verbindung der Formel IB, in der -A-B den Rest $-CH(R^4)_n-OH$ bedeutet und $R^1$, $R^4$, U und $X_m$ die in Anspruch 1 genannte Bedeutung besitzen, mit einem Alkohol B-O-H in Gegenwart von Triphenylphosphin und Diethylazodicarboxylat umsetzt.

**54.** Verfahren zur Herstellung einer heteroaromatischen Alkoholverbindung der Formel IB gemäß Anspruch 53, gekennzeichnet dadurch, daß man eine heteroaromatische Verbindung IB, in der -A-B den Rest

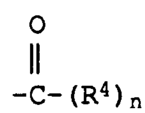

bedeutet und $R^1$, $R^4$, U und $X_m$ die in Anspruch 1 genannte Bedeutung besitzen, mit Reduktionsmitteln umsetzt.

**55.** Verfahren zur Herstellung einer heteroaromatischen Verbindung der Formel IB, in der $X_m$ den Rest

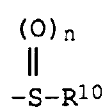

bedeutet und $R^1$, U, A und B die in Anspruch 1 genannte Bedeutung besitzen, $R^{10}$ Alkyl, Alkenyl, Alkinyl, Aryl oder Hetaryl bedeutet und n 1 oder 2 ist, gekennzeichnet dadurch, daß man eine heteroaromatische Verbindung der Formel IB, in der $X_m$ den Rest -S- $R^{10}$ bedeutet und $R^1$, U, A und B die in Anspruch 1 genannte Bedeutung besitzen und $R^{10}$ Alkyl, Alkenyl, Alkinyl, Aryl oder Hetaryl bedeutet, mit m-Chlorperbenzoesäure umsetzt.

**56.** Verfahren zur Herstellung einer heteroaromatischen Verbindung der Formel XXXIV

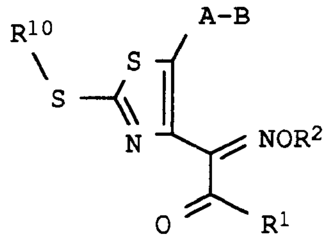

XXXIV

in der $R^2$, A und B die in Anspruch 1 genannte Bedeutung besitzen und $R^{10}$ Alkyl, Alkenyl, Alkinyl, Aryl oder Hetaryl bedeutet und $R^1$ $C_1$-$C_6$-Alkoxy ist, gekennzeichnet dadurch, daß man eine heteroaromatische Verbindung der Formel XXXV

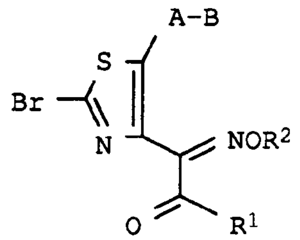

XXXV

in der $R^2$, A und B die in Anspruch 1 genannte Bedeutung besitzen und $R^1$ $C_1$-$C_6$-Alkoxy ist, unter alkalischen Bedingungen mit einem Mercaptan $R^{10}$-S-H umsetzt, wobei $R^{10}$ Alkyl, Alkenyl, Alkinyl, Aryl oder Hetaryl bedeutet.

**57.** Verfahren zur Herstellung einer heteroaromatischen Verbindung der Formel XXXV

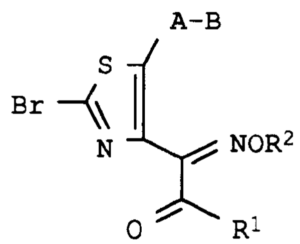

XXXV

in der $R^2$, A und B die in Anspruch 1 genannte Bedeutung besitzen und $R^1$ $C_1$-$C_6$-Alkoxy ist, gekennzeichnet dadurch, daß man eine heteroaromatische Verbindung der Formel XXXVI

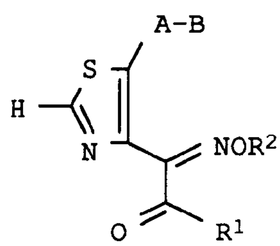

XXXVI

in der $R^2$, A und B die in Anspruch 1 genannte Bedeutung besitzen, und $R^1$ $C_2$-$C_6$-Alkoxy ist, mit einem Bromierungsmittel umsetzt.

**58.** Verfahren zur Herstellung einer heteroaromatischen Verbindung der Formel IB, in der U den Rest $NOR^2$ in cis-Konfiguration bedeutet, $R^2$, $X_m$, A und B die in Anspruch 1 genannte Bedeutung besitzen und $R^1$ $C_1$-$C_6$-Alkoxy bedeutet, gekennzeichnet dadurch, daß man eine heteroaromatische Verbindung der Formel IB, in der U den Rest $NOR^2$ in trans-Konfiguration bedeutet, $R^2$, $X_m$, A und B die in Anspruch 1 genannte Bedeutung besitzen und $R^1$ $C_1$-$C_6$-Alkoxy bedeutet, durch Bestrahlen mit UV-Licht oder in saurem Milieu katalysiert isomerisiert.

**59.** Verfahren zur Herstellung einer heteroaromatischen Verbindung der Formel XXXVII

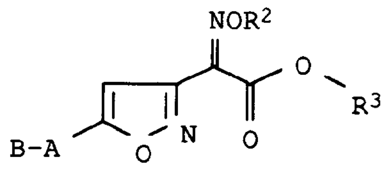

XXXVII

in der $R^3$ $C_1$-$C_6$-Alkyl bedeutet und $R^2$, A und B die in Anspruch 1 genannte Bedeutung besitzen, gekennzeichnet dadurch, daß man ein Oxim der Formel XXXVIII

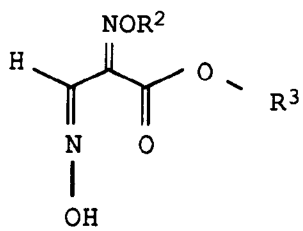

XXXVIII

in der $R^3$ $C_1$-$C_6$-Alkyl bedeutet und $R^2$ die in Anspruch 1 genannte Bedeutung besitzt, mit einem Acetylen der Formel B-A-C≡C-H in Gegenwart von Natriumhypochlorid umsetzt.

**60.** Verfahren zur Herstellung eines Oxims der Formel XXXVIII gemäß Anspruch 59, gekennzeichnet dadurch, daß man ein Bromid der Formel XXXIX

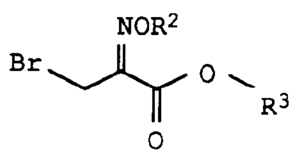

XXXIX

in der $R^3$ $C_1$-$C_6$-Alkyl bedeutet und $R^2$ die in Anspruch 1 genannte Bedeutung besitzt mit N-Methylmorpholin-N-oxid und anschließend in situ mit Hydroxylamin umsetzt.

**61.** Fungizid, enthaltend einen inerten Trägerstoff und eine fungizid wirksame Menge einer heteroaromatischen Verbindung der Formel IA und IB

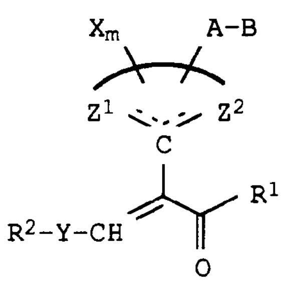

IA

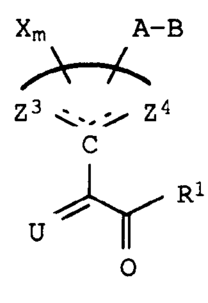

IB

in denen die gestrichelte Linie für eine Doppelbindung zwischen dem C-Atom und $Z^1$ oder $Z^3$ oder zwischen dem C-Atom und $Z^2$ oder $Z^4$ steht, und der Index und die Substituenten die folgende Bedeutung haben:
$R^1$
$C_1$-$C_6$-Alkyl, $C_1$-$C_6$-Alkoxy, $C_1$-$C_6$-Alkylamino oder Di-($C_1$-$C_6$-alkyl)-amino;

$R^2$
$C_1$-$C_6$-Alkyl;
A
eine direkte Bindung; ggf. subst. Alkylen, Alkenylen, Alkinylen;
$-(CHR^4)_n$-O-, $-(CHR^4)_n$-S-, $-(CHR^4)_n$-NH-, $-(CHR^4)_n$-$NR^5$, $-(CHR^4)_n$-S(=O)-, $-(CHR^4)_n$-$S(=O)_2$-, $-(CHR^4)_n$-O-S-(=O)-, $-(CHR^4)_n$-S(=O)-O-, $-(CHR^4)_n$-O-$S(=O)_2$-, $-(CHR^4)_n$-$S(=O)_2$-O-, $-(CHR^4(_n$-C(=O)-, $-(CHR^4)_n$-C(=O)O-, $-(CHR^4)_n$-O-C(=O)-, $-(CHR^4)_n$-$CR^5$=N-O-, $-(CHR^4)_n$-$CR^5$=N-N=$CR^6$-, $-(CHR^4)_n$-O-N=$CR^7$-,

| | |
|---|---|
| n | 0,1,2,3 oder 4 wobei die Reste $R^4$ verschieden sein können, wenn der Wert von n größer als 1 ist; |
| $R^4$, $R^5$ und $R^6$ | unabhängig voneinander Wasserstoff; ggf. subst. $C_1$-$C_6$-Alkyl, $C_2$-$C_6$-Alkenyl, $C_2$-$C_6$-Alkinyl oder Aryl; |
| $R^7$ | Wasserstoff, Cyano; ggf. subst. Alkyl, Alkenyl, Alkinyl, Cycloalkyl, Cycloalkenyl, Cycloalkinyl, Heterocyclyl, Aryl, Heteroaryl; ggf. subst. Alkoxy, Alkenyloxy, Alkinyloxy, Cycloalkoxy, Cycloalkenyloxy, Cycloalkinyloxy, Heterocyclyloxy, Aryloxy, Heteroaryloxy; oder $R^7$ und B gemeinsam mit dem C-Atom an das sie gebunden sind ein ggf. subst., gesättigtes oder teilweise ungesättigtes, alicyclisches oder heterocyclisches System; |

B
Wasserstoff, Halogen,
ggf. subst. Alkyl, Alkenyl, Alkinyl;
Cycloalkyl, Cycloalkenyl, Cycloalkinyl, Heterocyclyl, Aryl, Heteroaryl, $-(CHR^4)_p$-Cycloalkyl, $-(CHR^4)_p$-cycloalkenyl, $-(CHR^4)_p$-Cycloalkinyl, $-(CHR^4)_p$-Heterocyclyl, $-(CHR^4)_p$-Aryl, $-(CHR^4)_p$-Heteroaryl, $-(CHR^4)_p$-O-Cycloalkyl, $-(CHR^4)_p$-O-Cycloalkenyl, $-(CHR^4)_p$-O-Cycloalkinyl, $-(CHR^4)_p$-O-Heterocyclyl, $-(CHR^4)_p$-O-Aryl, $-(CHR^4)_p$-O-Heteroaryl, $-(CHR^4)_p$-S-Cycloalkyl, $-(CHR^4)_p$-S-Cycloalkenyl, $-(CHR^4)_p$-S-Cycloalkinyl, $-(CHR^4)_p$-S-Heterocyclyl, $-(CHR^4)_p$-S-Aryl, $-(CHR^4)_p$-S-Heteroaryl, $-(CHR^4)_p$-NH-Cycloalkyl, $-(CHR^4)_p$-NH-Cycloalkenyl, $-(CHR^4)_p$-NH-Cycloalkinyl, $-(CHR^4)_p$-NH-Heterocyclyl, $-(CHR^4)_p$-NH-Aryl, $-(CHR^4)_p$-NH-Heteroaryl, $-(CHR^4)_p$-$NR^5$-Cycloalkyl, $-(CHR^4)_p$-$NR^5$-cycloalkenyl, $-(CHR^4)_p$-$NR^5$-Cycloalkinyl, $-(CHR^4)_p$-$NR^5$-Heterocyclyl, $-(CHR^4)_p$-$NR^5$-Aryl, $-(CHR^4)_p$-$NR^5$-Heteroaryl, wobei die vorstehend genannten cyclischen Reste ihrerseits Substituenten tragen können;

| | |
|---|---|
| p | 1, 2, 3 oder 4, wobei die Reste $R^4$ verschieden sein können, wenn der Wert von n größer als 1 ist; |

U
eine Gruppe $CH_2$, CHCl, $CHR^2$ oder $NOR^2$;
X
Wasserstoff, Cyano, Nitro, Halogen, Haloalkyl, Haloalkoxy,
ggf. subst. Alkyl, Alkenyl, Alkinyl, Cycloalkyl, Cycloalkenyl, Heterocyclyl, Aryl, Heteroaryl;
ggf. subst. Alkoxy, Alkenyloxy, Alkinyloxy, Cycloalkoxy, Cycloalkenyloxy, Heterocyclyloxy, Aryloxy, Heteroaryloxy;
ggf. subst. Alkylthio, Alkenylthio, Alkinylthio, Cycloalkylthio, Cycloalkenylthio, Heterocyclylthio, Arylthio, Heteroarylthio;
Amino, welches ein oder zwei der folgenden Gruppen tragen kann: ggf. subst. Alkyl, Alkenyl, Alkinyl, Cycloalkyl, Cycloalkenyl, Heterocyclyl, Aryl und/oder Heteroaryl;
ggf. subst. Alkylcarbonyl, Alkenylcarbonyl, Alkinylcarbonyl, Cycloalkylcarbonyl, Cycloalkenylcarbonyl, Heterocyclylcarbonyl, Arylcarbonyl, Heteroarylcarbonyl;
ggf. subst. Alkylcarbonyloxy, Alkenylcarbonyloxy, Alkinylcarbonyloxy, Cycloalkylcarbonyloxy, Cycloalkenylcarbonyloxy, Heterocyclylcarbonyloxy, Arylcarbonyloxy, Heteroarylcarbonyloxy;
ggf. subst. Alkoxycarbonyl, Alkenyloxycarbonyl, Alkinyloxycarbonyl, Cycloalkoxycarbonyl, Cycloalkenyloxycarbonyl, Heterocyclyloxycarbonyl, Aryloxycarbonyl, Heteroaryloxycarbonyl;
ggf. subst. Alkylcarbonylamino, Alkenylcarbonylamino, Alkinylcarbonylamino, Cycloalkylcarbonylamino, Cycloalkenylcarbonylamino, Heterocyclylcarbonylamino, Arylcarbonylamino, Heteroarylcarbonylamino, wobei diese Reste an der Aminogruppe zusätzlich eine der folgenden Gruppen tragen können: ggf. subst. Alkyl, Alkenyl, Alkinyl, Cycloalkyl, Cycloalkenyl, Heterocyclyl, Aryl und/oder Heteroaryl;
Aminocarbonyl, welches an der Aminogruppe ein oder zwei der folgenden Gruppen tragen kann: ggf. subst. Alkyl, Alkenyl, Alkinyl, Cycloalkyl, Cycloalkenyl, Heterocyclyl, Aryl, und/oder Heteroaryl;
ggf. subst. Alkyl-S(=O)-, Alkenyl-S(=O)-, Alkinyl-S(=O)-, Cycloalkyl-S(=O)-, Cycloalkenyl-S(=O)-,

Heterocyclyl-S(=O)-, Aryl-S(=O)-, Heteroaryl-S(=O)-;
ggf. subst. Alkyl-S(=O)-O-, Alkenyl-S(=O)-O-, Alkinyl-S(=O)-O-, Cycloalkyl-S(=O)-O-, Cycloalkenyl-S(=O)-O-, Heterocyclyl-S(=O)-O-, Aryl-S(=O)-O-, Heteroaryl-S(=O)-O-,;
ggf. subst. Alkyl-O-S(=O)-, Alkenyl-O-S(=O)-, Alkinyl-O-S(=O)-, Cycloalkyl-O-S(=O)-, Cycloalkenyl-O-S(=O)-, Heterocyclyl-O-S(=O)-, Aryl-O-S(=O)-, Heteroaryl-O-S(=O)-;
ggf. subst. Alkyl-$S(=O)_2$-, Alkenyl-$S(=O)_2$-, Alkinyl-$S(=O)_2$-, Cycloalkyl-$S(=O)_2$-, Cycloalkenyl-$S(=O)_2$-, Heterocyclyl-$S(=O)_2$-, Aryl-$S(=O)_2$-, Heteroaryl-$S(=O)_2$-;
ggf. subst. Alkyl-$S(=O)_2$-O-, Alkenyl-$S(=O)_2$-O-, Alkinyl-$S(=O)_2$-O-, Cycloalkyl-$S(=O)_2$-O- Cycloalkenyl-$S(=O)_2$-O-, Heteroxyclyl-$S(=O)_2$-O-, Aryl-$S(=O)_2$-O-, Heteroaryl-$S(=O)_2$-O-;
ggf. subst. Alkyl-O-$S(=O)_2$-, Alkenyl-O-$S(=O)_2$-, Alkinyl-O-$S(=O)_2$-, Cycloalkyl-O-$S(=O)_2$-, Cycloalkenyl-O-$S(=O)_2$-, Heterocyclyl-O-$S(=O)_2$-, Aryl-O-$S(=O)_2$-, Heteroaryl-O-$S(=O)_2$-;
ggf. subst. Alkyl-ON=$CR^8$-, Alkenyl-ON=$CR^8$-, Alkinyl-ON=$CR^8$-, Cycloalkyl-ON=$CR^8$-, Cycloalkenyl-ON=$CR^8$-, Heterocyclyl-ON=$CR^8$-, Aryl-ON=$CR^8$-, Heteroaryl-ON=$CR^8$-;

$R^8$ Wasserstoff; Cyano;
ggf. subst. Alkyl, Alkenyl, Alkinyl, Cycloalkyl, Cycloalkenyl, Cycloalkinyl, Heterocyclyl, Aryl, Heteroaryl;
ggf. subst. Alkoxy, Alkenyloxy, Alkinyloxy, Cycloalkoxy, Cycloalkenyloxy, Cycloalkinyloxy, Heterocyclyloxy, Aryloxy, Heteroaryloxy;

m
1 oder 2, wobei die Reste X verschieden sein können, wenn m den Wert 2 hat;

Y
Sauerstoff oder Schwefel;

$Z^1$-$Z^2$
zusammen mit dem C-Atom an das sie gebunden sind entweder ein 6-gliedriger Heteroaromat enthaltend neben Kohlenstoffringgliedern zwei oder drei Stickstoffatome oder ein 5-gliedriger Heteroaromat enthaltend neben Kohlenstoffringgliedern zwei oder drei Stickstoffatome oder ein
oder zwei Stickstoffatome und ein Sauerstoff- oder ein Schwefelatom;

$Z^3$-$Z^4$
zusammen mit dem C-Atom an das sie gebunden sind entweder ein 6-gliedriger Heteroaromat enthaltend neben Kohlenstoffringgliedern ein, zwei oder drei Stickstoffatome oder ein 5-gliedriger Heteroaromat enthaltend neben Kohlenstoffringgliedern ein, zwei oder drei Stickstoffatome oder ein oder zwei Stickstoffatome und ein Sauerstoff- oder ein Schwefelatom oder ein Sauerstoff- oder ein Schwefelatom;
oder ihres pflanzenverträglichen Säureadditionsprodukte oder Basenadditionsproduktes,
ausgenommen solche Verbindungen, in denen
der Ring C-$Z^1$-$Z^2$ ein Pyrazolring ist, der Rest -Y-$R^2$ die Bedeutung -O-$CH_3$ hat und -A-B in 4-Stellung am Pyrazolring steht, -A- der Rest -O-$CH_2$- ist, B gegebenenfalls substituiertes Phenyl oder gegebenenfalls substituiertes Thiazolyl bedeutet oder
der Ring C-$Z^1$- $Z^2$ ein Pyrazolring ist, der Rest -Y-$R^2$ die Bedeutung -O-$CH_3$ hat und -A-B in 4-Stellung am Pyrazolring steht, -A- der Rest -O-CO- ist, B gegebenenfalls substituiertes Phenyl oder gegebenenfalls substituiertes Thiazolyl bedeutet oder
der Ring C-$Z^1$-$Z^2$ ein Thiazolring ist, Y Sauerstoff bedeutet und $R^1$ die Bedeutung $OCH_3$ hat, oder
der Ring C-$Z^3$-$Z^4$ ein Thiazolring ist, X an ein N-Atom des Thiazolrings gebunden ist und U den Rest $NOR^2$ bedeutet.

**62.** Verfahren zur Bekämpfung von Pilzen, dadurch gekennzeichnet, daß man die Pilze oder die vom Pilzbefall bedrohten Materialien, Pflanzen, Saatgut oder den Erdboden behandelt mit einer fungizid wirksamen Menge einer heteroaromatischen Verbindung der Formel IA, IB

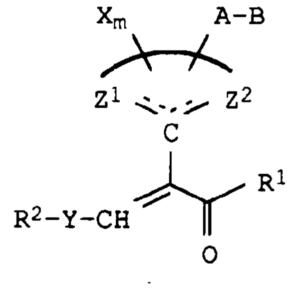

IA

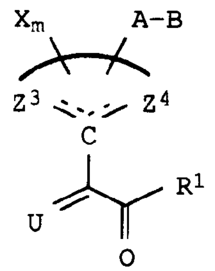

IB

in denen die gestrichelte Linie für eine Doppelbindung zwischen dem C-Atom und $Z^1$ oder $Z^3$ oder zwischen dem C-Atom und $Z^2$ oder $Z^4$ steht, und der Index und die Substituenten die folgende Bedeutung haben:

$R^1$

$C_1$-$C_6$-Alkyl, $C_1$-$C_6$-Alkoxy, $C_1$-$C_6$-Alkylamino oder Di- ($C_1$-$C_6$-alkyl)-amino;

$R^2$

$C_1$-$C_6$-Alkyl;

A

eine direkte Bindung; ggf. subst. Alkylen, Alkenylen, Alkinylen;

$-(CHR^4)_n-O-$, $-(CHR^4)_n-S-$, $-(CHR^4)_n-NH-$, $-(CHR^4)_n-NR^5-$, $-(CHR^4)_n-S(=O)-$, $-(CHR^4)_n-S(=O)_2-$, $-(CHR^4)_n-O-S(=O)-$, $-(CHR^4)_n-S(=O)-O-$, $-(CHR^4)_n-O-S(=O)_2-$, $-(CHR^4)_n-S(=O)_2-O-$, $-(CHR^4(_n-C(=O)-$, $-(CHR^4)_n-C(=O)O-$, $-(CHR^4)_n-O-C(=O)-$, $-(CHR^4)_n-CR^5=N-O-$, $-(CHR^4)_n-CR^5=N-N=CR^6-$, $-(CHR^4)_n-O-N=CR^7-$,

| | |
|---|---|
| n | 0, 1, 2, 3 oder 4, wobei die Reste $R^4$ verschieden sein können, wenn der Wert von n größer als 1 ist; |
| $R^4$, $R^5$ und $R^6$ | unabhängig voneinander Wasserstoff; ggf. subst. $C_1$-$C_6$-Alkyl, $C_2$-$C_6$-Alkenyl, $C_2$-$C_6$-Alkinyl oder Aryl; |
| $R^7$ | Wasserstoff, Cyano: ggf. subst. Alkyl, Alkenyl, Alkinyl, Cycloalkyl, Cycloalkenyl, Cycloalkinyl, Heterocyclyl, Aryl, Heteroaryl; ggf. subst. Alkoxy, Alkenyloxy, Alkinyloxy, Cycloalkoxy, Cycloalkenyloxy, Cycloalkinyloxy, Heterocyclyloxy, Aryloxy, Heteroaryloxy; oder $R^7$ und B gemeinsam mit dem C-Atom an das sie gebunden sind ein ggf. subst., gesättigtes oder teilweise ungesättigtes, alicyclisches oder heterocyclisches System; |

B

Wasserstoff, Halogen,

ggf. subst. Alkyl, Alkenyl, Alkinyl;

Cycloalkyl, Cycloalkenyl, Cycloalkinyl, Heterocyclyl, Aryl, Heteroaryl, $-(CHR^4)_p$-Cycloalkyl $-(CHR^4)_p$-Cycloalkenyl, $-(CHR^4)_p$-Cycloalkinyl, $-(CHR^4)_p$-Heterocyclyl, $-(CHR^4)_p$-Aryl, $-(CHR^4)_p$-Heteroaryl, $-(CHR^4)_p$-O-Cycloalkyl, $-(CHR^4)_p$-O-Cycloalkenyl, $-(CHR^4)_p$-O-Cycloalkinyl, $-(CHR^4)_p$-O-Heterocyclyl $-(CHR^4)_p$-O-Aryl, $-(CHR^4)_p$-O-Heteroaryl, $-(CHR^4)_p$-S-Cycloalkyl $-(CHR^4)_p$-S-Cycloalkenyl, $-(CHR^4)_p$-S-Cycloalkinyl, $-(CHR^4)_p$-S-Heterocyclyl, $-(CHR^4)_p$-S-Aryl, $-(CHR^4)_p$-S-Heteroary $-(CHR^4)_p$-NH-Cycloalkyl $-(CHR^4)_p$-NH-Cycloalkenyl, $-(CHR^4)_p$-NH-Cycloalkinyl, $-(CHR^4)_p$-NH-Heterocyclyl, $-(CHR^4)_p$-NH-Aryl, $-(CHR^4)_p$-NH-Heteroaryl, $-(CHR^4)_p-NR^5$-Cycloalkyl, $-(CHR^4)_p-NR^5$-Cycloalkenyl $-(CHR^4)_p-NR^5$-Cycloalkinyl $-(CHR^4)_p-NR^5$-Heterocyclyl, $-(CHR^4)_p-NR^5$-Aryl, $-(CHR^4)_p-NR^5$-Heteroaryl, wobei die vorstehend genannten cyclischen Reste ihrerseits Substituenten tragen können;

| | |
|---|---|
| p | 1, 2, 3 oder 4, wobei die Reste $R^4$ verschieden sein können, wenn der Wert von n größer als 1 ist; |

U

eine Gruppe $CH_2$, CHCl, $CHR^2$ oder $NOR^2$;

X

Wasserstoff, Cyano, Nitro, Halogen, Haloalkyl, Haloalkoxy,

ggf. subst. Alkyl, Alkenyl, Alkinyl, Cycloalkyl, Cycloalkenyl, Heterocyclyl, Aryl, Heteroaryl;

ggf. subst. Alkoxy, Alkenyloxy, Alkinyloxy, Cycloalkoxy, Cycloalkenyloxy, Heterocyclyloxy, Aryloxy,

Heteroaryloxy;
ggf. subst. Alkylthio, Alkenylthio, Alkinylthio, Cycloalkylthio, Cycloalkenylthio, Heterocyclylthio, Arylthio, Heteroarylthio;
Amino, welches ein oder zwei der folgenden Gruppen tragen kann: ggf. subst. Alkyl, Alkenyl, Alkinyl, Cycloalkyl, Cycloalkenyl, Heterocyclyl, Aryl und/oder Heteroaryl;
ggf. subst. Alkylcarbonyl, Alkenylcarbonyl, Alkinylcarbonyl, Cycloalkylcarbonyl, Cycloalkenylcarbonyl, Heterocyclylcarbonyl, Arylcarbonyl, Heteroarylcarbonyl;
ggf. subst. Alkylcarbonyloxy, Alkenylcarbonyloxy, Alkinylcarbonyloxy, Cycloalkylcarbonyloxy, Cycloalkenylcarbonyloxy, Heterocyclylcarbonyloxy, Arylcarbonyloxy, Heteroarylcarbonyloxy;
ggf. subst. Alkoxycarbonyl, Alkenyloxycarbonyl, Alkinyloxycarbonyl, Cycloalkoxycarbonyl, Cycloalkenyloxycarbonyl, Heterocyclyloxycarbonyl, Aryloxycarbonyl, Heteroaryloxycarbonyl;
ggf. subst. Alkylcarbonylamino, Alkenylcarbonylamino, Alkinylcarbonylamino, Cycloalkylcarbonylamino, Cycloalkenylcarbonylamino, Heterocyclylcarbonylamino, Arylcarbonylamino, Heteroarylcarbonylamino, wobei diese Reste an der Aminogruppe zusätzlich eine der folgenden Gruppen tragen können: ggf. subst. Alkyl, Alkenyl, Alkinyl, Cycloalkyl, Cycloalkenyl, Heterocyclyl, Aryl und/oder Heteroaryl;
Aminocarbonyl, welches an der Aminogruppe ein oder zwei der folgenden Gruppen tragen kann: ggf. subst. Alkyl, Alkenyl, Alkinyl, Cycloalkyl, Cycloalkenyl, Heterocyclyl, Aryl, und/oder Heteroaryl;
ggf. subst. Alkyl-S(=O)-, Alkenyl-S(=O)-, Alkinyl-S(=O)-, Cycloalkyl-S(=O)-, Cycloalkenyl-S(=O)-, Heterocyclyl-S(=O)-, Aryl-S(=O)-, Heteroaryl-S(=O)-;
ggf. subst. Alkyl-S(=O)-O-, Alkenyl-S(=O)-O-, Alkinyl-S(=O)-O-, Cycloalkyl-S(=O)-O-, Cycloalkenyl-S(=O)-O-, Heterocyclyl-S(=O)-O-, Aryl-S(=O)-O-, Heteroaryl-S(=O)-O-,;
ggf. subst. Alkyl-O-S(=O)-, Alkenyl-O-S(-O)-, Alkinyl-O-S(=O)-, Cycloalkyl-O-S(=O)-, Cycloalkenyl-O-S(=O)-, Heterocyclyl-O-S(=O)-, Aryl-O-S(=O)-, Heteroaryl-O-S(=O)-;
ggf. subst. Alkyl-$S(=O)_2$-, Alkenyl-$S(=O)_2$-, Alkinyl-$S(=O)_2$-, Cycloalkyl-$S(=O)_2$-,Cycloalkenyl-$S(=O)_2$-, Heterocyclyl-$S(=O)_2$-, Aryl-$S(=O)_2$-, Heteroaryl-$S(=O)_2$-;
ggf. subst. Alkyl-$S(=O)_2$-O-, Alkenyl-$S(=O)_2$-O-, Alkinyl-$S(=O)_2$-O-, Cycloalkyl-$S(=O)_2$-O-, Cycloalkenyl-$S(=O)_2$-O-, Heteroxyclyl-$S(=O)_2$-O-, Aryl-$S(=O)_2$-O-, Heteroaryl-$S(=O)_2$-O-;
ggf. subst. Alkyl-O-$S(=O)_2$-, Alkenyl-O-$S(=O)_2$-, Alkinyl-O-$S(=O)_2$-, Cycloalkyl-O-$S(=O)_2$-, Cycloalkenyl-O-$S(=O)_2$-, Heterocyclyl-O-$S(=O)_2$-, Aryl-O-$S(=O)_2$-, Heteroaryl-O-$S(=O)_2$-;
ggf. subst. Alkyl-ON=$CR^8$-, Alkenyl-ON=$CR^8$-, Alkinyl-ON=$CR^8$-, Cycloalkyl-ON=$CR^8$-, Cycloalkenyl-ON=$CR^8$-, Heterocyclyl-ON=$CR^8$-, Aryl-ON=$CR^8$-, Heteroaryl-ON=$CR^8$-;

$R^8$ Wasserstoff; Cyano;
ggf. subst. Alkyl, Alkenyl, Alkinyl, Cycloalkyl, Cycloalkenyl, Cycloalkinyl, Heterocyclyl, Aryl, Heteroaryl;
ggf. subst. Alkoxy, Alkenyloxy, Alkinyloxy, Cycloalkoxy, Cycloalkenyloxy, Cycloalkinyloxy, Heterocyclyloxy, Aryloxy, Heteroaryloxy;

m
1 oder 2, wobei die Reste X verschieden sein können, wenn m den Wert 2 hat;
Y
Sauerstoff oder Schwefel;
$Z^1$-$Z^2$
zusammen mit dem C-Atom an das sie gebunden sind entweder ein 6-gliedriger Heteroaromat enthaltend neben Kohlenstoffringgliedern zwei oder drei Stickstoffatome oder ein 5-gliedriger Heteroaromat enthaltend neben Kohlenstoffringgliedern zwei oder drei Stickstoffatome oder ein
oder zwei Stickstoffatome und ein Sauerstoff- oder ein Schwefelatom;
$Z^3$-$Z^4$
zusammen mit dem C-Atom an das sie gebunden sind entweder ein 6-gliedriger Heteroaromat enthaltend neben Kohlenstoffringgliedern ein, zwei oder drei Stickstoffatome oder ein 5-gliedriger Heteroaromat enthaltend neben Kohlenstoffringgliedern ein, zwei oder drei Stickstoffatome oder ein oder zwei Stickstoffatome und ein Sauerstoff- oder ein Schwefelatom oder ein Sauerstoff- oder ein Schwefelatom;
oder ihres pflanzenverträglichen Säureadditionsproduktes oder Basenadditionsproduktes,
ausgenommen solche Verbindungen, in denen
der Ring C-$Z^1$-$Z^2$ ein Pyrazolring ist, der Rest -Y-$R^2$ die Bedeutung -O-$CH_3$ hat und -A-B in 4-Stellung am Pyrazolring steht, -A- der Rest -O-$CH_2$- ist, B gegebenenfalls substituiertes Phenyl oder gegebenenfalls substituiertes Thiazolyl bedeutet oder
der Ring C-$Z^1$-$Z^2$ ein Pyrazolring ist, der Rest -Y-$R^2$ die Bedeutung -O-$CH_3$ hat und -A-B in 4-Stellung am Pyrazolring steht, -A- der Rest -O-CO- ist, B gegebenenfalls substituiertes Phenyl oder gegebenenfalls substituiertes Thiazolyl bedeutet oder

der Ring C-$Z^1$-$Z^2$ ein Thiazolring ist, Y Sauerstoff bedeutet und $R^1$ die Bedeutung $OCH_3$ hat, oder der Ring C-$Z^3$-$Z^4$ ein Thiazolring ist, X an ein N-Atom des Thiazolrings gebunden ist und U den Rest $NOR^2$ bedeutet.